# EUROPEAN PATENT APPLICATION

(11) **EP 4 810 526 A1**
(43) Date of publication of application: **23.09.2026**
(21) Application number: 24896665.7
(22) Date of filing: 28.11.2024
(51) Int. Cl.: C07K 19/00, C07C 229/16, C07D 211/62, C07C 237/10, C07C 323/25, A61K 9/127, A61K 47/18, A61K 47/22, A61K 31/7105, A61K 31/7088, A61P 35/00, A61P 37/06, A61P 31/00

(54) **MOLECULE HAVING TARGETING FUNCTION AND APPLICATION**

(30) Priority: 30.11.2023 CN 202311627230; 10.07.2024 CN 202410918685
(71) Applicant: Shenzhen MagicRNA Biotechnology Co., Ltd., Shenzhen, Guangdong 518107 (CN)
(72) Inventor: FANG, Wanyin, Shenzhen, Guangdong 518107 (CN); XIAO, Zexiu, Shenzhen, Guangdong 518107 (CN)
(74) Representative: karo IP
(86) International application number: PCT/CN2024/135350
(87) International publication number: WO 2025/113580

(57) **Abstract**

The present invention relates to the technical field of medicine, and relates in particular to a molecule having a targeting function. The molecule having a targeting function can specifically deliver a drug to a specific cell, which facilitates precise programming of a specific cell in the body, a therapeutic effect of the drug is produced, and consequently drug dosage and side effects are greatly reduced, and the effect of precise treatment to cells in the body is achieved.

## Description

### Cross-reference to Related Applications

The present application claims the priority of Chinese Patent Application No. 2023116272306, filed on November 30, 2023, with the title "LIPID NANOPARTICLES AND NANOPARTICLE DRUGS", and Chinese Patent Application No. 2024109186851, filed on July 10, 2024, with the title "MOLECULE HAVING TARGETING FUNCTION AND APPLICATION". The above applications are hereby incorporated by reference in their entirety.

### Technical Field

The present invention relates to the field of medical technology, particularly to a molecule having targeting function and applications.

### Background

A lipid nanoparticle (LNP) is composed of four parts: an ionizable lipid compound, cholesterol, a phospholipid, and a PEGylated lipid. LNP represents a relatively mature delivery system technology platform that can be used to deliver an RNA drug, a vaccine, or a gene editing tool. Current research on the composition of LNP is quite in-depth.

The composition of LNP significantly affects its drug delivery performance. The ionizable lipid compound is the core component of LNP, determining mRNA delivery and transfection efficiency. In practice, nucleic acid drugs are prone to non-specific cellular uptake during delivery, leading to higher dosages and noticeable side effects.

In view of this, the present invention is proposed.

### Disclosure of the Invention

To address the aforementioned technical problem, the present invention provides a molecule having targeting function and applications thereof, which can specifically deliver pharmacologically active molecules to specific cells in the body, reducing non-specific cellular uptake of drugs, thereby decreasing drug dosage and minimizing side effects and achieving more effective and precise in vivo therapeutic effects for diseases.

Specifically, the technical solutions provided by the present invention are as follows:
In a first aspect, the present invention provides a molecule having targeting function, comprising:
(I) one or more hydrophobic chains;
(II) one or more linkers;
(III) one or more nanobodies.

Preferably, the number of the nanobody is 20 or less, and preferably 1-7.

Preferably, one amino acid containing a reactive site is interposed between the nanobody and the linker, and the amino acid comprises, but not limited to, cysteine and lysine.

Preferably, the nanobody is connected via a connecting sequence, which is located between the nanobody and the amino acid, and the connecting sequence comprises (GS)n and/or (GGGS)m, where n and m are the same or different values, and the values of n or m are selected from 0, 1, 2, 3, or 4.

Preferably, the hydrophobic chain comprises one or more substituted or unsubstituted C12-C18 alkyl.

Preferably, the linker comprises one or more structural units selected from -C(=O)O-, -O-C(=O)O-, -C(=O)NH-, -C(=O)S-, -S-, -S-S-, triazolyl, hydrazone bond, SMCC, Sulfo-SMCC, SATA, polypeptides as structural units, PEG, or PEG derivatives;

Preferably the PEG derivatives can be selected as maleimide-functionalized polyethylene glycol, such as PEG-MAL.

Preferably, the relative molecular mass of PEG or PEG derivatives is 500-5,000, and preferably 1,000, 2,000, 3,000, 4,000, or 5,000.

Preferably, the nanobody comprises an antigen-binding domain specific to an antigen, and the antigen is a membrane protein molecule.

Preferably, the membrane protein molecule is an immune cell-related cell membrane protein molecule or an immune-related cell membrane protein molecule, including but not limited to, one or more of the group consisting of CD3, CD4, CD5, CD7, CD8, CD25, CD38, CD61, CD42a, CD105, CD90, CD15, CD127, CD56, CD68, CD19, CD11c, CD138, F4/80, CD62P, CD49f, CD31, RANK, ALPL, PDPN, CD34, FcεR1α, CD203c, CD63, CD193, CD66b, CD41, CD117, and ASGPR.

Preferably, the membrane protein molecule is a central nervous system cell membrane protein molecule, including but not limited to, one or more of the group consisting of GD2, GD3, MOG, and TMEM119.

Preferably, the membrane protein molecule is a tumor-associated antigen, including but not limited to, one or more of the group consisting of CD133, PSMA, CLDN18.2, DLL3, TROP2, EGFRVIII, CA125, MUC1, MUC16, MSLN, CA9, HER2, HER3, TGM4, PSCA, CLDN6, STEAP2, GPC3, IL-13Ra2, EGFR, STEAP1, BCMA, FRa, VEGFR2, PDGFR-β, CEA, NCAM, FAP, SLC2A2, SEZ6L2 and LRP11.

Preferably, the membrane protein molecule is an immune regulatory molecule, including but not limited to, one or more of the group consisting of B7-H1, B7-H3, and B7-H4.

Preferably, the membrane protein molecule comprises, but is not limited to, one or more of the following targets: CD3, CD4, CD5, CD7, CD8, CD25, CD38, CD61, CD42a, CD105, CD90, CD15, CD127, CD56, CD68, CD19, CD11c, CD138, F4/80, CD62P, CD49f, CD31, RANK, ALPL, PDPN, CD34, FcεR1α, CD203c, CD63, CD193, CD66b, CD41, CD117, ASGPR, GD2, GD3, MOG, TMEM119, CD133, PSMA, CLDN18.2, DLL3, TROP2, EGFRVIII, CA125, MUC1, MUC16, MSLN, CA9, HER2, HER3, TGM4, PSCA, CLDN6, STEAP2, GPC3, IL-13Ra2, EGFR, STEAP1, BCMA, FRa, VEGFR2, PDGFR-β, CEA, NCAM, FAP, SLC2A2, SEZ6L2, LRP11, B7-H1, B7-H3 and B7-H4.

Preferably, the nanobody is a nanobody targeting CD8, with the amino acid sequence of the nanobody represented by any one of SEQ ID NOs:1-3;
and/or, the nanobody is a nanobody targeting CD19, with the amino acid sequence of the nanobody represented by any one of SEQ ID NOs:79-81;
and/or, the nanobody is a nanobody targeting CD56;
and/or, the nanobody is a nanobody targeting CD5, with the amino acid sequence of the nanobody represented by any one of SEQ ID NOs:4-78;
and/or, the nanobody is a nanobody targeting PSMA, with the amino acid sequence of the nanobody represented by any one of SEQ ID NOs:82-83;
and/or, the nanobody is a nanobody targeting CD133, with the amino acid sequence of the nanobody represented by any one of SEQ ID NOs:84-85.

The sequences in the present application are as follows:
Anti-human CD8 Nb seq(corresponding to SEQ ID NOs:1-3)
SEQ ID NO:1: 
QVQLQESGGGSVQPGGSLRLSCAASGFTFDDYAMSWVRQVPGKGLEWVSTI
NWNGGSAEYAEPVKGRFTISRDNAKNTVYLQMNSLKLEDTAVYYCAKDADLVW
YNLSTGQGTQVTVSSAAAYPYDVPDYGS
SEQ ID NO:2: 
QVQLQESGGGLVQPGGSLRLSCAASGFTFDDYAMSWVRQVPGKGLEWVSTI
NWNGGSAEYAEPVKGRFTISRDNAKNTVYLQMNSLKLEDTAVYYCAKDADLVW
YNLRTGQGTQVTVSSAAAYPYDVPDYGS
SEQ ID NO:3: 
QVQLQESGGGLVQAGGSLRLSCAASGFTFDDYAIGWFRQAPGKEREGVSCIR
VSDGSTYYADPVKGRFTISSDNAKNTVYLQMNSLKPEDAAVYYCAAGSLYTCVQS
IVVPARPYYDMDYWGKGTQVTVSSAAAYPYDVPDYGS
Anti-human CD5 Nb seq(corresponding to SEQ ID NOs:4-78)
SEQ ID NO:4: 
DVQLQESGGGLVQPGDSLRLSCAASGRTFSSYDMGWFRQAPGKEREFVAAIS
RSGRDTLHADSVKGRFTISRDNAKNTVYLQMNSLKPEDTAVYICAGTGFEYAGSTP
 YKYPYWGQGTQVTVSS
SEQ ID NO:5: 
DVQLQESGGGLVQAGGSLRLSCTASGSIVSIISMGWYRQAPGKQRELIAKITTD
GTTNYADSVRGRFTISRDIPKKIMYLQMSSLTPEDTAVYFCNRDRLLYGLVKSDWG
QGTQVTVSS
SEQ ID NO:6: 
DVQLQESGGGFVRPGGSLRLSCAASGPTVSSYTVGWFRQAPGKEREYVATIS
WSDASTYYADFVKGRFTISRDYAKQVVYLQMNSLKPEDTAVYHCAAVFRRVPISAT
LYDYWGQGTQVTVSS
SEQ ID NO:7: 
DVQLQESGGGLVQPGGSLRLSCAASGSAFSRIGVGWYRQAPGKEREFVAVITA
GGNTNYADSVKGRFTISRDNAKNTVYLQMNSLKPEDTGVYYCNYPGRPWGQGTQ
VTVSS
SEQ ID NO:8: 
DVQLQESGGGLVQPGGSLRLSCAASGSAFSRIGVGWYRQAPGKEREFVAVITA
GGNTNYADSVKGRFTISRDNAKNTVYLQMNSLKPGDTGVYYCNYPGRPWGQGT
QVTVSS
SEQ ID NO:9: 
DVQLQESGGGLVQPGGSLRLACAASGNIFEIHAMYWYRQAPGKQREWVAAI
TTRGSTNVADSVKGRFTISRDNAKNTVYLQMNSLKPEDTAVYLCNADVFSALTGA
GTEYWGQGTQVTVSS
SEQ ID NO:10: 
DVQLQESGGGLVQPGGSLRLSCVASGSAFSRIGVGWYRQAPGKEREFVAEITA
GGNTDYADSVKGRFTISRDNAKNMVYLQMNSLKPEDTGVYYCNYPGRPWGQGT
QVTVSS
SEQ ID NO:11: DVQLQESGGGLVQAGGSLRLSCAASGHTLSIYAMTWFRQDSGKDREFVAAIT 
WPDYSTYYADSVKGRFTISGDRPKNTVYLEMNSVKPEDTAVYYCAADRYVNSVPT
ATPQEWRYWGQGTQVTVSS
SEQ ID NO:12: 
DVQLQESGGGLVQAGGSLRLSCAVSGRTISGMSVGWFRQAPGKEREFVAADN
WAGAKTSYADSVKGRFTISRDNAKAMVYLQMNGLKPEDTAVYYCAGRTSDGLST
YARIEDYYGWGQGTQVTVSS
SEQ ID NO:13: 
DVQLQESGGGLAQAGGSLRLSCAASGGIFSIDAAAWYRQAPGKERELVAHIFH
GGSTTYEDSVKGRFTISRDNAKNTVYLQMNSLKPEDTAVYYCYGGPPGMAIWGK
GTQVTVSS
SEQ ID NO:14: 
DVQLQESGGGLVQAGASLSLSCAASGRTVNYVFGMGWFRQALGKEREFVAA
ITRSGGIPLYADSVKGRFTISRDNAKNTVYLQMNSLKPEDTAVYYCAIDRQYYSGR
NYPQSDPAEYDYWGQGTQVTVSS
SEQ ID NO:15: 
DVQLQESGGGLVQAGGSLRLSCAASVRTFSKYDIGWFRQAPGKEREFVATISS
SGRSTYYADFAKGRFGISRDYAKNTVYLEMNSLKPEDTAVYACAVRLGYSGSETDP
RQYAYWGQGTQVTVSS
SEQ ID NO:16: 
DVQLQESGGGFVQPGGSLRLSCAASGPTVSSYTVGWFRQAPGKEREYVATIS
WSDASTYYADFVKGRFTISRDYAKQVVYLQMNSLKPEDTAVYHCAAVFRRVPISAT
LYDYWGQGTQVTVSS
SEQ ID NO:17: 
DVQLQESGGGLVQAGDSLRLSCAPSGGIFSINAAAWYRQAPGKERELVAHINH
GGSTTYVGSVKGRFTISRDNAKNTVYLQMNSLKPEDTAVYYCYGGPPGMAIWGK
GTQVTVSS
SEQ ID NO:18: 
DVQLQESGGGLVQAGGSLRLSCTASGTIFNVMGWYRQAPGKQRELVATITFGL
TTNYADSVKGRFAISRDNSEKTVYLQMNSLKPEDTAVYYCNAGSRYWGQGTQVT
VSS
SEQ ID NO:19: 
DVQLQESGGGLVQAGGSLRLSCAASGRTFSSYAMGWFRQAPGKEREFVAAIS
WSGGSTTEADSVKGRFTISRDNAKNTVYLQMYSLKPEDTAVYYCAAARDLVSTVV
AETSEYDYWGQGTQVTVSS
SEQ ID NO:20: 
DVQLQESGGGLVQAGGSLRLSCAVSGRTINDMSVGWFRQAPGKEREFVAADI
WPGAKTSYADPVKGRFTITRDNAKAMVYLQMNGLKPEDTAVYYCAGRTSDGLRT
YTRIEDYYGWGQGTQVTVSS
SEQ ID NO:21: 
DVQLQESGGGLVQPGGSLRLACAASGSGFSINAMGWYRQAPGKQREWVAAI
NSVGMTNYRDSVKGRFTISRDDAKNTVYLQMNSLKPEDTAVYYCNVKPLGTADY
WGQGTQVTVSS
SEQ ID NO:22: 
DVQLQESGGGLVQAGGSLRLSCAVSGRTISGMSVGWFRQAPGKEREFVAADS
WAGAKTSYADSVKGRFTISRDNAKAMVYLQMKGLKPEDTAVYYCAGRTSDGLFN
YARIEDYYGWGQGTQVTVSS
SEQ ID NO:23: 
DVQLQESGGGLVQAGDSLRLSCAVSGRTISGMSVGWFRQAPGKEREFVAADS
SAGAKTSYADSVKGRFTISRDNAKAMVYLQMNGLKPEDTAVYYCAGRTSYGLST
YARMEDYYGWGQGTQVTVSS
SEQ ID NO:24: 
DVQLQESGGGLVQPGGSLRLSCAASGSIFSSNVMGWYRQAPGKQREWVSTII
SDGRTNYAGSVKGRFTISRDNAKNTVYLQMNNLKPEDTALYYCKLSTLNRDYWG
QGTLVTVGQGTQVTVSS
SEQ ID NO:25: 
DVQLQESGGGLVQAGGSLRLSCAASGRTSTIYTMGWFRQAPGKEREFVAATS
WSGGNTNYAASMKGRFTISRNNAKNTVYLQMNSLKPEDTAVYYCAANRGSYYLG
PSPYEYDWWGQGTQVTVSS
SEQ ID NO:26: 
DVQLQESGGGLVQPGGSLRLSCAASGSIFSINAMGWYRQAPGKQRDVVAYIM
SDGNTHYADSVKGRFTISRDENTSYLQMNSLKPDDTAVYYCNADIQPPMPEATIYV
YWGQGTQVTVSS
SEQ ID NO:27: 
DVQLQESGGGLVQTGGSLRVSCAASGRTFSSAYMGWFRQAPGKERVLVAEIW
LSSGQTYYADSVKGRFTISRENAKNTVYLQMNSLKPEDTAVYYCAAQNRATGRYD
YWGQGTQVTVSS
SEQ ID NO:28: 
DVQLQESGGGLVQPGGSLRLSCAASGSIFSSNVMGWYRQAPGKQREWVSTII
SDGRTNYAGSVKGRFTISRDNAKNTVYLQMNNLKPEDTALYYCKLSTLNRDYWG
QGTQVTVGQGTQVTVSS
SEQ ID NO:29: 
DVQLQESGGGLVQPGGSLRLSCAASGGIFSINAAAWYRQAPGKERELVAHIYH
GGSTTYVGSVKGRFTISRDNAKNTVYLQMNSLKPEDTAVYYCYGGPPGMAIWGK
GTQVTVSS
SEQ ID NO:30: 
DVQLQESGGGFVQPGGSLRLSCAASGPTVSSYTVGWFRQAPGKEREYVATIS
WSDASTYYADFVKGRFTISRDYAKKVVYLQMNSLKPEDTAVYHCAAVFRRVPISAT
LYDYWGQGTQVTVSS
SEQ ID NO:31: 
DVQLQESGGGLVQAGGSLRLSCAASGGIFSINAAAWYRQAPGKERELVAHITH
GGSTTYEGSVKGRFTISKDNAKNTVYLQMNSLKPEDTAVYYCYGGPPGMAIWGK
 GTQVTVSS
SEQ ID NO:32: 
DVQLQESGGGLVQAGGSLRLSCAASGSIVSIISVGWYRQAPGKQRELVAKITT
DGTTNYADSVRGRFTISRDIAKKMLYLQMSSLTPEDTAVYFCNRDRLVYGLVKAD
WGQGTQVTVSS
SEQ ID NO:33: 
DVQLQESGGGLVQAGGSLRLSCAASGRTSTIYTMGWFRQAPGREREFVAATS
WSGGNTNYAGSVKGRFTISRNDAKNTVYLQMNSLKPEDTAVYYCAANRGSYYLG
PSFYEYDYWGQGTQVTVSS
SEQ ID NO:34: 
DVQLQESGGGLVQAGGSLRLSCTASGSIVSIISMGWYRQAPGKQRELIAKITTD
GTTNYADSVRGRFTTSRDIPKKIMYLQMSSLTPEDTAVYFCNRDRLLYGLVKSDWG
QGTQVTVSS
SEQ ID NO:35: 
DVQLQESGGGLVQTGGSLRVSCAASGRTFSSSYMGWFRQAPGQERELVAEIW
LSSGQTYYADSVKGRFTISRENAKNTAYLQMNSLTPEDTAVYYCAAQNRATGRYD
YWGQGTQVTVSS
SEQ ID NO:36: 
DVQLQESGGGLVQAGGSLRLSCTASGSIVSIISMGWYRQAPGKQRELIAKITTD
GTTSYADSVRGRFTISRDIPKKIMYLQMSSLTPEDTAVYFCNRDRLLYGLVKSDWG
QGTQVTVSS
SEQ ID NO:37: 
DVQLQESGGGLVQAGGSLRLSCAASGGIFSINAAAWYRQAPGKERELVAHIY
HGGSTTYVGSVKGRFTISRDNAKNTVYLQMNSLKPEDTAVYYCYGGPPGMAIWG
KGTQVTVSS
SEQ ID NO:38: DVQLQESGGGLVQAGGSLRLSCAASGRTFSSTGMGWFRQAPGKEREFVAAIN 
WSGEDTYYADSVKGRFTISGDNAKNVVYLQMNSLKPEDTAVYYCAASPYYNPYG
PRLRTPSWYDYWGQGTQVTVSS
SEQ ID NO:39: 
DVQLQESGGGLVQAGGSLRLSCAASGGTFSSSGMGWYRQAPGKEREFVAAIN
WSGEDTLYADSVKGRFTISGDNAKNTVHLQMNSLKPEDTAVYYCAASPYYNPYGP
RLRTPSWYDYWGQGTQVTVSS
SEQ ID NO:40: 
DVQLQESGGGLVQAGGSLRLSCTASGTIFNVMGWYRQAPGKQRELVATITFD
LSTNYADSVKGRFAISRDVTEKTVYLQMDSLKPEDTAVYYCNAGSRYWGQGTQV
TVSS
SEQ ID NO:41: 
DVQLQESGGGLVQAGGSLRLSCAASGSTSGYSFRFWTMNWYRQGSGKQREL
VATITSSGITTYADSVKGRFTVSRDNTKNTLYLQMNSLKPEDTATYYCNFPGNSWG
QGTQVTVSS
SEQ ID NO:42: 
DVQLQESGGGLVQAGDSLRLSCAVSGRTISDMSVGWFRQAPGKEREFVAADS
WAGAKTSYADSVKGRFTISRNNAKAMVYLQMNGLKPEDTAVYYCAGRTSDGLST
YEWIEDYYGWGQGTQVTVSS
SEQ ID NO:43: 
DVQLQESGGGLVQAGDSLRLSCVASGGIFSINAAAWYRQAPGKERELVAHIYH
GGSTTYVGSVKGRFTISRDNAKNTVYLQMNSLKPEDTAVYYCYGGPPGMAIWGK
GTQVTVSS
SEQ ID NO:44: 
DVQLQESGGGLVQPGGSLTLSCAASGSIFSRDVMGWYRQAPGKQRELVAAITI
GGYADYADSVKGRFTISRDNAKNTVYLRMNSLKPEDTAVYFCKLGYGSGSWGQG
TQVTVSS
SEQ ID NO:45: 
DVQLQESGGGLVQPGGSLRLSCAASRSTFSIDIMAWYRQAPEKKRELVALITR
RGTTNYADSVKGRFTISRDNAKNTMYLQMNNLKPEDTAVYYCNSDDTETRWGLR
QYWGQGTQVTVSS
SEQ ID NO:46: 
DVQLQESGGGLVQAGGSLRLSCAASGHTLSIYAMTWFRQDSGKDREFVAAIT
WPDYSTYYADSVKGRFTISGDRPKNTVHLEMNSVKPEDTAVYYCAADRYVNSVPT
ATPQEWRYWGQGTQVTVSS
SEQ ID NO:47: 
DVQLQESGGGLVQPGASLSLSCAASGRTVNYVFGMGWFRQALGKEREFVAAI
TRSGGIPLYADSVKGRFTISRDNAKNTVYLQMNSLKPEDTAVYYCAIDRQYYSGRN
YPQSDPAEYDYWGQGTQVTVSS
SEQ ID NO:48: 
DVQLQESGGGLVQAGGSLRLSCSASGSIVSIISMGWYRQAPGKQRELIAKITT
DGTTNYADSVRGRFTISRDIPKKIMYLQMSSLTPEDTAVYFCNRDRLLYGLVKSDW
GQGTQVTVSS
SEQ ID NO:49: 
DVQLQESGGGLVQAGGSLRLSCAASGGIFSINAAAWYRQAPGKERELVAHIFH
GGSTTYEDSVKGRFTISRDNAKNTVYLQMNSLKPEDTAVYYCYGGPPGMAIWGK
GTQVTVSS
SEQ ID NO:50: 
DVQLQESGGGLVQPGGSLRLSCAASGSIFSINAMGWYRQAPGKQRDVVAYIM
SDGNTHYADSVKGRFTISRDKNTSYLQMNSLKPDDTAVYYCNADIQPPMPEATIYV
YWGQGTQVTVSS
SEQ ID NO:51: 
DVQLQESGGGLVQAGGSLRLSCTASGTIFNVMGWYRQAPGKQRELVATITFG
LATNYADSVKGRFAISRDDSEKTVYLQMNSLKPEDTAVYYCNAGSRYWGQGTQV
TVSS
SEQ ID NO:52: 
DVQLQESGGGLVQAGGSLRLSCAASGSTSGYSFGFWTMNWYRQGSGKQREL
VATITSSGITTYADSVKGRFTVSRDNTKNTLYLQMNSLKPEDTATYYCNFPGNSWG
QGTQVTVSS
SEQ ID NO:53: 
DVQLQESGGGLVQAGGSLRLSCTASGSIVSIISMGWYRQAPGKQRELIAKVTT
DGTTNYADSVRGRFTISRDIPKKIMYLQMSSLTPEDTAVYFCNRDRLLYGLVKSDW
GQGTQVTVSS
SEQ ID NO:54: 
DVQLQESGGGLVQPGGSLRLSCLVSGSIYSIPAMYWYRQAPGKQREFVADITN
RGSTNYADSVKGRFTISRDNAKNTVNLQMNSLKPEDTAVYYCNVLGTLGPRGSLQ
DYWGQGTQVTVSS
SEQ ID NO:55: 
DVQLQESGGGLVQPGGSLRLSCTASGSIVSIISMGWYRQAPGKQRELIAKITTD
GTTNYADSVRGRFTISRDIPKKIMYLQMSSLTPEDTAVYFCNRDRLLYGLVKSDWG
QGTQVTVSS
SEQ ID NO:56: 
DVQLQESGGGLVQAGGSLRLSCAASVRTFSKYDIGWFRQAPGKEREFVATISS
SGRSTYYADFVKGRFGISRDYAKNTVYLEMNSLKPEDTAVYACAVRLGYSGSETDP
RQYAYWGQGTQVTVSS
SEQ ID NO:57: 
DVQLQESGGGLVQAGGSLRLSCTASGSIVSIISMGWYRQAPGKQRELIAKITTD
GTTNYADSVRGRFTISRGIPKKIMYLQMSSLTPEDTAVYFCNRDRLLYGLVKSDWG
QGTQVTVSS
SEQ ID NO:58: 
DVQLQESGGGLVQTGGSLRLSCAASGRTFEHYTMGMGWFRQAPGKEREFVA
AISWSDGSTYYEDSVKGRFTISRGNAKTTVYLQMNSLEPEDTGVYICAAKRGIGSD
 YPATLLYEYDYWGQGTQVTVSS
SEQ ID NO:59: 
DVQLQESGGGLVQAGGSLRLSCAVSGRTISDMSVGWFRQAPGKEREFVAADT
WAGAKTSYADSVKGRFTISRDTAKAMVYLQMNGLKPEDTAVYYCAGRTSYGLST
YARIEDYYGWGQGTQVTVSS
SEQ ID NO:60: 
DVQLQESGGGLVQAGGSLRLSCAASGRTFNTNAMGWFRQAPGKEREFVAAI
DWSGDSTFYADSVKGRFTISRDNAKNTVYLQMNSLKPEDTAVYYCAADGLLYYS
GSYYPDYWGQGTQVTVSS
SEQ ID NO:61: 
DVQLQESGGGLVQAGGSLRLSCAVSGRTISDMSVGWFRQAPGKEREFVAADS
WAGAKASYADSVKGRFTISRDNTKAMVHLQMNGLKPEDTAVYYCAGRTSDGLST
YARIEDYYGWGQGTQVTVSS
SEQ ID NO:62: 
DVQLQESGGGLVQAGGSLRLSCTASGTIFNVMGWYRQAPGKQRELVATITFGL
TTNYADSVKGRFAISRDSSEKTVYLQMNSLKPEDTAVYYCNAGSRYWGQGTQVTV
SS
SEQ ID NO:63: 
DVQLQESGGGLVQAGGSLRLSCAVSGRTISDMSVGWFRQAPGKEREFVAADT
WAGAKTSYADSVKGRFTISRDNAKAMVYLQMNGLKPEDTAVYYCAGRTSYGLST
YARIEDYYGWGQGTQVTVSS
SEQ ID NO:64: 
DVQLQESGGGLVQPGGSLRLSCAASGSAFSRIGVGWYRQAPGKEREFVAVITA
GGNTNCADSVKGRFTISRDNAKNTVYLQMNSLKPEDTGVYYCNYPGRPWGQGT
QVTVSS
SEQ ID NO:65: DVQLQESGGGLGQAGGSLRLSCAVSEIIFRFTTLNWYRQAPGKQRELVASISSG 
GSTNYDDSVKGRFTISRDNAKKLVYLQMNSLKPEDTAVYYCNVPGNPWGQGTQV
TVSS
SEQ ID NO:66: 
DVQLQESGGGLVQPGGSLKLSCAVSGRTISGMSVGWFRQAPGKEREFVAADS
WAGAKTSYADSVKGRFTISRDNAKAMVYLQMKGLKPEDTAVYYCAGRTSDGLFN
YARIEDYYGWGQGTQVTVSS
SEQ ID NO:67: 
DVQLQESGGGFVQAGDSLRLSCAASGRAVISYTLGWFRQAPGKEREFVATIS
WTDGSTYYSDSVKGRFTMSRDDAKKTVYLQMNSLKPEDTAVYYCAAVFRRVPTS
ATLYDYWGQGTQVTVSS
SEQ ID NO:68: 
DVQLQESGGGLVQAGDSLRLSCAVSGRTISGMAVGWFRQAPGKEREFVAADS
RAGAKTSYADSVKGRFTLSRDNAKAMVYLQMNGLKPEDTAVYYCAGRTSDGLST
YARIQDYYGWGQGTQVTVSS
SEQ ID NO:69: 
DVQLQESGGGLVQPGGSLRLSCAASGSAFSRIGVGWYRQAPGKEHEFVAEIN
AGGNTNYADSVKGRFTISRDNAKNTVYLQMNSLKPEDTGVYYCNYPGRPWGQGT
QVTVSS
SEQ ID NO:70: 
DVQLQESGGGLVQPGGSLRLSCAASGRFFSISAMGWYRQAPGKQRELVAAIIN
GGGTHYADSVKGRFTISRDNAKNTVYLQMNSLKPEDTAVYYCNADAQMESGTLY
FIVFWGQGTQVTVSS
SEQ ID NO:71: 
DVQLQESGGGLVQAGDSLRLSCAVSGRTISDMSVGWFRQAPGKEREFVAADS
WAGAKTSYADSVKGRFTISRDNAKAMVYLQMNGLKPEDTAVYYCAGRTSDGLST
YARIEDYYGWGQGTQVTVSS
SEQ ID NO:72: 
DVQLQESGGGLVQPGDSLRLSCAASGRTFSSYDMGWFRQAPGKEREFVAAIS
RSGRDTLHADSVKGRFTISRDNAKNTVYLQMYSLKPEDTAVYICAGTGFEYAGSTP
YKYPYWGQGTQVTVSS
SEQ ID NO:73: 
DVQLQESGGGLVQPGGSLRLSCAASGSAFSRIGVGWYRQAPGKEREFVAVIA
AGGNTNYADSVKGRFTISRDNAKNTVYLQMNSLKPEDTGVYYCNYPGRPWGQGT
QVTVSS
SEQ ID NO:74: 
DVQLQESGGGLVQAGGSLRLSCAASGGIFSRDVMGWYRQAPGEQRELVAAIT
IGGSASYADSVKGRFTISRDNAKNTVYLRMNSLTPEDTAVYFCKLGYGSGSWGQG
TQVTVSS
SEQ ID NO:75: 
DVQLQESGGGLVQAGGSLRLSCVVSGRTLSGMSVGWFRQAPGKEREFVAAD
SWAGAKASYADSVKGRFTISRDNAEAIVYLQMNGLKPEDTAVYYCAGRTSYGLST
YARIEDYYGWGQGTQVTVSS
SEQ ID NO:76: 
DVQLQESGGGLVQAGGSLRLSCAVSGRTISGMSVGWFRQAPGKEREFVAADT
WAGAKTSYADSVKGRFTISRDNTKAMVYLQMNGLKPEDTAVYYCAGRTSDGLST
YARIEDYYGWGQGTQVTVSS
SEQ ID NO:77: 
DVQLQESGGGLVQPGGSLRLSCAASGSIFSINAMGWYRQAPGKQRELVAHITN
IGTTNYADSVKGRFTISRDNGKNTVYLQMNSLKPEDTAVYICNADLPSMVETDYYL
VYWGQGTQVTVSS
SEQ ID NO:78: 
DVQLQESGGGLVQAGDSLRLSCAVSGRTISDMSVGWFRQAPGKEREFVAADS
WAGAKTSYADSVKGRFTISRDNAKAMVYLQMSGLKPEDTAVYYCAGRTSSGLSTY
ARIEDYYGWGQGTQVTVSS
SEQ ID NO:79: 
QVQLQESGGGSVQAGGSLRLSCAAVSGITFRRNCMGWFRQVPGKEREGVATL
DGASLTRYADSVKGRFIISQDNAKNTLYLQMNALKPEDTAMYYCAASPTRYGCGG
ADFDSWGQGTQVTVSS
SEQ ID NO:80: 
QVQLVESGGGLVQAGDSVTLSCTASGRTFSSYALAWFRQRPGKEREFVAGLR
WGGPTNYADSVKDRFTISGDSAKHTMYLQMNSLKPEDTAVYYCGYNPGGWAVPS
QYEYDSWGQGTQVTVSSAHHSEDPS
SEQ ID NO:81: 
QLQLVESGGGAAQTGGSLRLSCAASGGDSNINIMGWNRQVPGKQRELVAAIT
SDGNSNYGGSAKGRFTISRDNAKNTVYLQMNSLKPEDTAVYYCTVWTYSGKYEG
QGTQVTVSSEPKTPKPQD
SEQ ID NO:82: 
ESGGGSVQTGGSLRLSCTASEYTYMNNCMGWFRQTPGKEREGIAAISSDGRRI
EYDDSVKGRFVLSRDNDRNALILEMNALKSEDSGRYYCAAVRWGYPYSKSSYLGP
GDPGHRLL
SEQ ID NO:83: 
ATGGCCCAGGTGCAGCTCGTGGAGTCAGGCGGAGGATTGGTGCAGGCTGG
GGCTCTCTGAGACTCTCCTGTGTAGCCTCTGGACGCGCCTTTCGTAGATATACC
ATGGGCTGGTTCCGCCAGGCTCCAGGGAAGGAGCGTGAGTTTGTAGCAACAAT
TAACTGGAGTGGTCGTAATACAGCGTATGCCGACTCCGTGAAGGGCCGATTCAC
CATCTCCAGAGACAGCCGCAAAAACACCGCGTATCTCCAAATGAACAGCCTGA
AACCTGAAGATACGGCCGTTTATTATTGTGCACAATCGCGAGCGATTACAGGTG
GCACAGTTCCCGCCGGTTATAACATCTGGGGCCAGGGGACCCAGGTCACCGTCT
CCTCAGAACCCAAGACACCAAAACCACAAGCGGCCGC
SEQ ID NO:84: QVQLVESGGGLVHSGGSLRLSCAASGFTFSSEWMYWVRRDPLQWVSSINSPG 
SKTYYADSVRGRFTISRDNDKNMVYLQMNSLKPEDTGIYYCVRGVAQSVTGGEW
YKVDLGDLEEWGQGTQVTVSS
SEQ ID NO:85:

EVQLVESGGDQVQPGGSLRLSCVGSGFSFGTYTMMWFRHSPGKRLEWVSTIN
SDGSNPRYLNSVEDRFIISRDNAKNTLYLQMNNLKSEDTGLYYCTNGGTRGRGTQ
VTVSS

In a second aspect, the present invention provides a nanobody having targeting function, wherein the amino acid sequence of the nanobody is represented by any one of SEQ ID NOs:4-78; or the amino acid sequence of the nanobody has a homology of 80% or greater, preferably 90% or greater, more preferably 95% or greater, with the amino acid sequence represented by any one of SEQ ID NOs:4-78.

In a third aspect, the present invention provides a lipid nanoparticle, wherein its components comprise the molecule having targeting function, and the content of the molecule having targeting function is 0.0001-1.0 mol% of the total lipids of the lipid nanoparticle, preferably 0.01-0.5 mol%.

Preferably, the components of the lipid nanoparticle comprise a polymer-conjugated lipid and an ionizable amino lipid; the polymer-conjugated lipid is selected from lipids modified with PEG or PEG derivatives; and
the relative molecular mass of the lipids modified with PEG or PEG derivatives is 2,000-5,000, preferably 2,000, 3,000, 4,000, or 5,000.

Preferably, the content of the polymer-conjugated lipid is 0.2-5 mol% of the total lipids of the lipid nanoparticle, preferably, 0.5-2 mol%.

Preferably, the PEG derivatives comprise maleimide-functionalized polyethylene glycol and non-maleimide-functionalized polyethylene glycol; wherein the polyethylene glycol can be selected from DMG-PEG2000; the maleimide-functionalized polyethylene glycol can be selected from PEG-MAL; and the content of the maleimide-functionalized polyethylene glycol is 0.01-1.0 mol% of the total lipids of the lipid nanoparticle, preferably 0.01-0.5 mol%.

Preferably, the content of the ionizable amino lipid is 30-70 mol% of the total lipids of the lipid nanoparticle, preferably 40-60 mol%.

More preferably, the ionizable amino lipid has the structure represented by general formula (I), or is an isomer, a pharmaceutically acceptable salt, a prodrug, or a solvate of the structure represented by general formula (I); 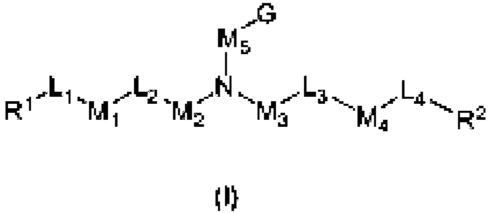
wherein, G is selected from H, OR, CN, -C(=O)OR', -OC(=O)R', -C(=O)NR'R", -NR'C(=O)R", NR'R", or a cycloalkyl structure containing at least one heteroatom, wherein the heteroatom is O or N;
wherein R, R', and R" are the same or different from each other, and each is independently selected from H, C₁-C₁₀ alkyl, C₃-C₁₀ cycloalkyl, C₃-C₁₀ alkenyl, or C₃-C₁₀ cycloalkenyl, C₁-C₁₀ alkyl with a terminal tertiary amino, C₃-C₁₀ cycloalkyl with a terminal tertiary amino, C₃-C₁₀ alkenyl with a terminal tertiary amino, or a cycloalkyl structure containing at least one heteroatom;
wherein the carbon atom or heteroatom that is substitutable in the cycloalkyl structure is unsubstituted or substituted with one or more hydroxyl, C₁-C₄ alkyl, C₂-C₄ alkenyl, C₃-C₈ cycloalkyl , or C₃-C₈ cycloalkenyl;
preferably, the cycloalkyl structure is unsubstituted or substituted with one or more C₁-C₄ alkyl, C₃-C₈ cycloalkyl, or hydroxyl;
more preferably, R', and R" are the same or different from each other, and each is independently selected from H, C₁-C₄ alkyl, C₁-C₁₀ alkyl with a terminal tertiary amino, C₃-C₁₀ cycloalkyl with a terminal tertiary amino, or a cycloalkyl structure containing at least one heteroatom; the cycloalkyl structure is unsubstituted or substituted with one or more C₁-C₄ alkyl or C₃-C₈ cycloalkyl,
and/or, M₁, M₂, M₃, and M₄ are the same or different from each other, and each is independently selected from C₁-C₂₄ alkylene, C₃-C₂₄ cycloalkylene, C₂-C₂₄ alkenylene, or C₃-C₂₄ cycloalkenylene; preferably, M₁, M₂, M₃, and M₄ are the same or different from each other, and each is independently selected from branched C₄-C₂₂ alkylene, branched C₄-C₂₂ cycloalkylene, branched C4-C₂₂ alkenylene, or C₄-C₂₂ cycloalkenylene; preferably, M₂ and M₃ are the same, being C₄-C₂₂ alkylene; M₁ and M₄ are the same, being branched C₄-C₂₂ alkylene;
and/or, R¹ and R² are the same or different, and each is independently selected from H, C₁-C₂₄ alkyl, C₃-C₂₄ cycloalkyl, C₂-C₂₄ alkenyl, or C₃-C₂₄ cycloalkenyl; preferably, R¹ and R² are the same or different, and each is independently selected from C₄-C₂₂ alkyl, or C₄-C₂₂ alkenyl;
and/or, L₁, L₂, L₃, and L₄ are the same or different from each other, and each is independently selected from -C(=O)O-, -OC(=O)-, -C(=O)S-, -SC(=O)-, -C(=O)NR-, -NRC(=O)-, -S(=O)-, -OS(=O)₂-, -S(=O)₂O-, -O-, -S-, or -S-S-; preferably, L₁, L₂, L₃, and L₄ are the same or different from each other, and each is independently selected from -C(=O)O-, -OC(=O)-, -C(=O)NR-, -NRC(=O)-, or-S-S-; when each of L₁, L₂, L₃, and L₄ is independently selected from -C(=O)NR- or -NRC(=O)-, preferably R is independently selected from H or C₁-C₁₀ alkyl; preferably, L₁ and L₄ are the same, and are -C(=O)O- or -OC(=O)-;
and/or, M₅ is selected from a single bond, C₁-C₁₆ alkylene, C₂-C₁₆ alkenylene, C₃-C₈ cycloalkylene, or C₃-C₈ cycloalkenylene. Preferably, M₅ is selected from a single bond, C₂-C₁₆ alkylene, C₂-C₁₆ alkenylene, C₄-C₈ cycloalkylene, or C₃-C₈ cycloalkenylene; preferably, M₅ is selected from a single bond, C₂-C₁₆ alkylene or C₄-C₆ cycloalkylene.

More preferably, M₅ and G are connected to form a structure 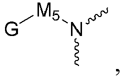 the structure can be one specifically selected from the following structures:

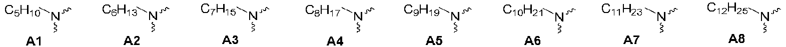

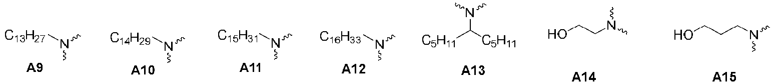

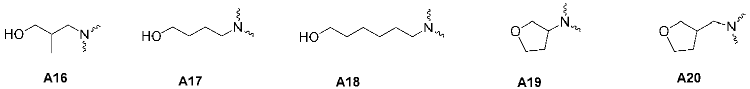

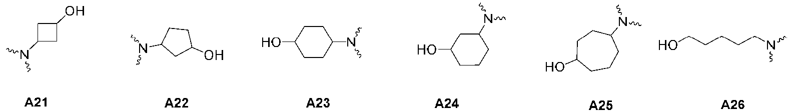

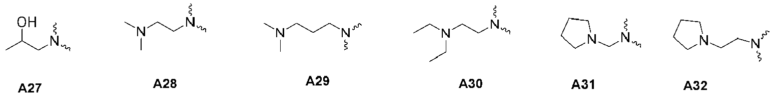

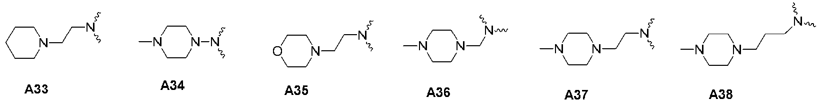

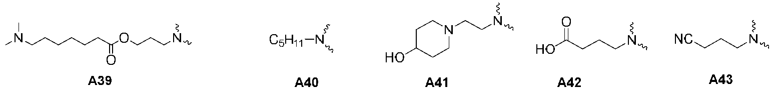

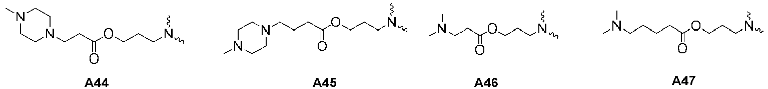

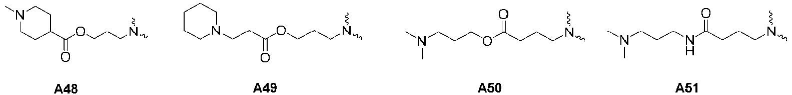

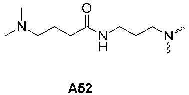

Wherein, the 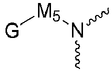 is preferably one selected from A1-A18, A22-A24, andA28-A52, and more preferably, one selected from A15, A23, A29, A33, and A39-A52;
and/or, fragment R¹-L₁-M₁-L₂-M₂- is R¹-C(=O)O-M₁-OC(=O)-M₂-, R¹-C(=O)NR-M₁-OC(=O)-M₂-, or R¹-C(=O)O-M₁-S-S-M₂-; fragment R²-L₄-M₄-L₃-M₃- is R²-C(=O)O-M₄-OC(=O)-M₃-, R²-C(=O)NR-M₄-OC(=O)-M₃-, or R²-C(=O)O-M₄-S-S-M₃-;
more preferably, general formula (I) structure is one of the following structures:

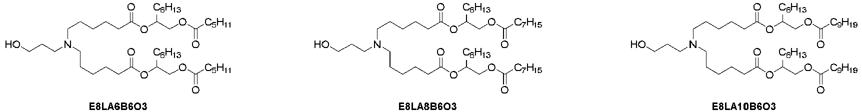

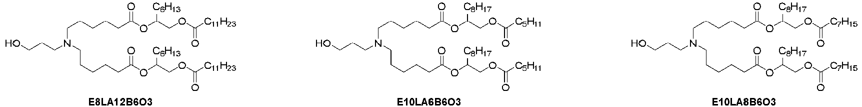

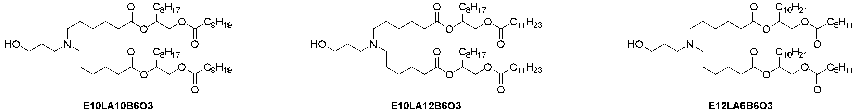

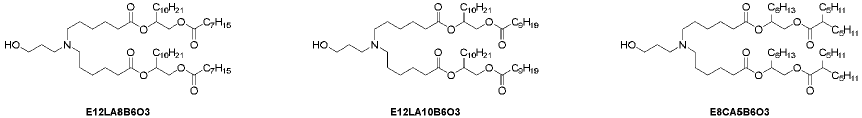

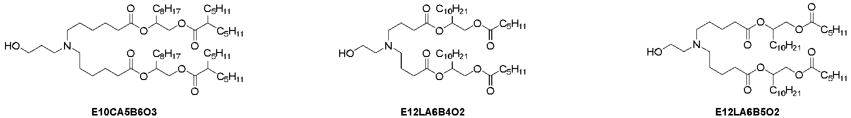

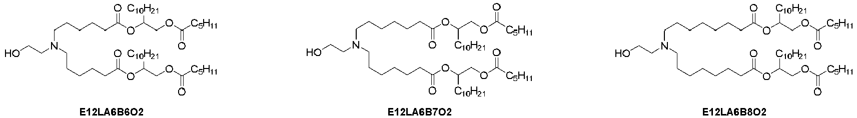

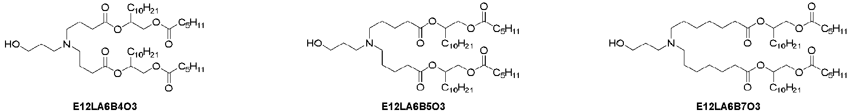

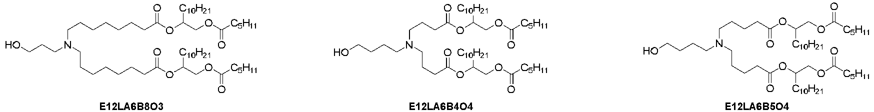

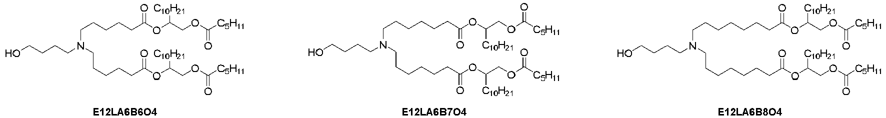

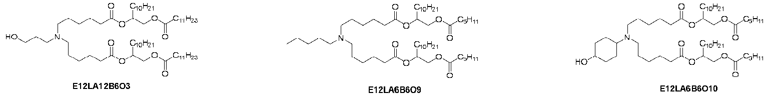

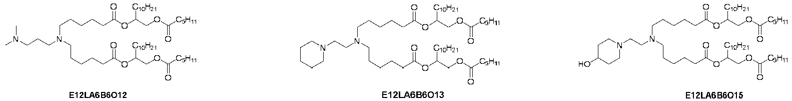

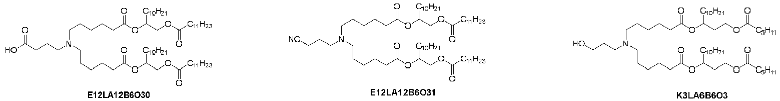

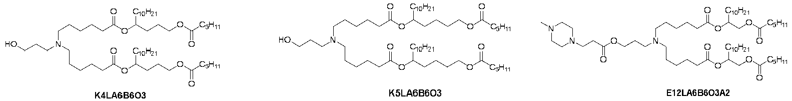

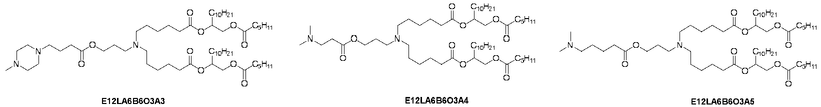

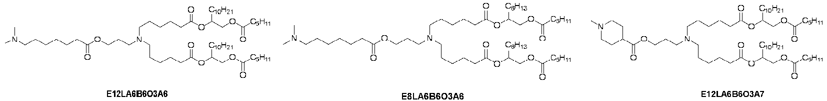

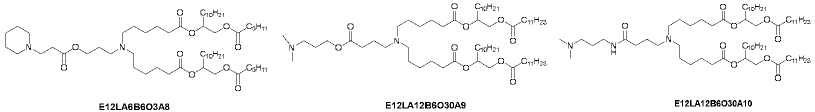

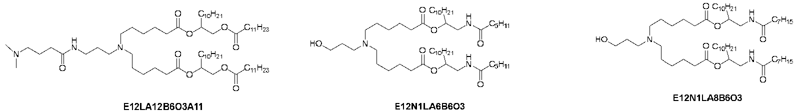

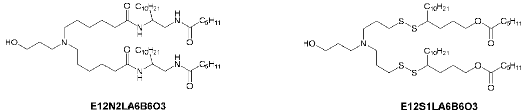

Preferably, the components of the lipid nanoparticle further comprise at least one of a steroid and a neutral lipid;
preferably, the molar ratio of the ionizable amino lipid, steroid, neutral lipid, and polymer-conjugated lipid is (30-70) : (0-65) : (0-30) : (0.2-5);
preferably, the steroid is one or more of the group consisting of cholesterol and derivatives thereof, cholesterol ester, steroid hormone, steroid vitamin, and phytosterol; preferably cholesterol; and/or the neutral lipid is a phospholipid; and/or the polymer-conjugated lipid is a PEGylated lipid, preferably DMG-PEG2000;
more preferably, the molar ratio of the ionizable amino lipid, cholesterol, phospholipid, and PEGylated lipid is (40-50) :(40-45) :(0-15) : (0.5-2).

Preferably, the lipid nanoparticles are utilized for targeted delivery of nucleic acids to target cells of a subject, including immune cells, central nervous system cells, tumor cells, etc. When the target cells come into contact with the lipid nanoparticles, the lipid nanoparticles provide at least one of the following benefits:
(i) increased specificity of targeted delivery to the target cells compared to reference LNP;
(ii) increased half-life of the nucleic acid or the polypeptide encoded by the nucleic acid in the target cells compared to reference LNP;
(iii) increased transfection efficiency compared to reference LNP; and
(iv) low levels of dye-accessible mRNA (<15%) and high RNA encapsulation efficiency, wherein at least 80% of the mRNA is recovered in the final formulation relative to the total RNA used in the bulk preparation of LNPs.

In a fourth aspect, the present invention provides a method for preparing the lipid nanoparticle, comprising the following steps: mixing a polymer-conjugated lipid, an ionizable amino lipid, a steroid, and an auxiliary phospholipid to obtain a lipid nanoparticle without targeting function, and then connecting the molecule having targeting function of to the lipid nanoparticle without targeting function through membrane fusion or chemical bond to obtain a lipid nanoparticle having targeting function.

In a fifth aspect, the present invention provides a method for preparing the lipid nanoparticle comprising the following steps: mixing a polymer-conjugated lipid, an ionizable amino lipid, a steroid, and an auxiliary phospholipid to obtain a lipid nanoparticle without targeting function, and then connecting a nanobody to the lipid nanoparticle without targeting function through a chemical bond ,linkage to obtain a lipid nanoparticle having targeting function;
preferably, the polymer-conjugated lipid comprises maleimide-functionalized polyethylene glycol, and the nanobody is connected to the maleimide-functionalized polyethylene glycol.

In a sixth aspect, the present invention provides another method for preparing the lipid nanoparticle, comprising the following steps: mixing a polymer-conjugated lipid, an ionizable amino lipid, a steroid, an auxiliary phospholipid, and the molecule having targeting function to obtain the lipid nanoparticle having targeting function.

In a seventh aspect, the present invention provides a method for targeted delivery of pharmacologically active molecules to specific cells of a subject, comprising contacting the specific cells with the lipid nanoparticle; preferably, the specific cells comprise, but are not limited to, immune cells, immune-related cells, central nervous cells, tumor cells, somatic cells, and cells infected by viruses, bacteria, or fungi; preferably, the target cells are B cells, T cells, or NK cells.

In an eighth aspect, the present invention provides a method for expressing a target protein or polypeptide in target cells of a subject, comprising contacting the target cells with the lipid nanoparticle; preferably, the target cells comprise, but are not limited to, immune cells, immune-related cells, central nervous cells, tumor cells, somatic cells, and cells infected by viruses, bacteria, or fungi; preferably, the target cells are B cells, T cells, or NK cells.

In a ninth aspect, the present invention provides a method for treating, ameliorating, or preventing symptom(s) of a disease in a subject in need thereof, the method comprises: administering the lipid nanoparticle to the subject to deliver a pharmacologically active molecule to target cells of the subject; preferably, the target cells comprise, but are not limited to, immune cells, immune-related cells, central nervous system cells, tumor cells, somatic cells, and cells infected by viruses, bacteria, or fungi; preferably, the disease is an immune disease, inflammatory disease, central nervous system disease, degenerative disease, bone-related disease, pathogen infection, metabolic disease, endocrine disease, aging, or cancer; preferably, the pharmacologically active molecule comprise antigens used in therapeutic or preventive vaccines for the treatment or prevention of immune diseases, inflammatory diseases, central nervous system diseases, degenerative diseases, bone-related diseases, pathogen infections, metabolic diseases, endocrine diseases, aging, or cancer; preferably, the target cells are B cells, T cells, or NK cells.

In a tenth aspect, the present invention provides use of the lipid nanoparticle as a drug delivery carrier.

In a eleventh aspect, the present invention provides a pharmaceutical composition,comprising the lipid nanoparticle, and the lipid nanoparticle comprises a pharmacologically active molecule;
preferably, the pharmacologically active molecule comprises one or more selected from the group consisting of mRNA, DNA, siRNA, saRNA, shRNA, miRNA, circle RNA, lnc RNA, gRNA, polypeptide, or protein;
preferably, the pharmacologically active molecule comprises mRNA, and the mRNA is used to encode a CAR molecule, a TCR molecule, an immune regulatory molecule, a functional protein molecule, or a gene editing tool such as a base editor or Cas9 protein.

Preferably, the CAR molecule comprises at least one sequence of the following sequences: a sequence capable of specifically recognizing a tumor-specific antigen, a transmembrane connecting sequence, and/or a sequence that assists the immune function of T cells.

Preferably, a sequence encoding a TCR molecule comprises a sequence capable of recognizing at least one molecule selected from the group consisting of NY-ESO-1, AFP, HBsAg, MAGEA1, Mesothelin, MART-1, CD19, CD28, PD-1, CD8, CT83, E6, E7, E8, GPC3, H3.3-K27M, HIV Gag polyprotein, HLA-A/AFP, KRASG12(V/D), KRASG12D, KRASG12V, LMP1, LMP2, EBNA1, MAGEA10, MAGEA3, MAGEA4, MC2R, mHag HA-1, MYO1G, PRAME, WT1, gp100, CEA, p53, HLA-A2, EGFR, DR5, RAS, LAGE-1, CMV, HCV, MCPyv, and HA-1H.

Preferably, a sequence encoding an immune regulatory molecule comprises a sequence of at least one molecular selected from the group consisting of CD28, PD-1, CTLA-4, RGMB, ICOS, CD28H, NKp30, HVEM, OX40, Fas, 4-1BB, CD27, CD30, APO-2, APO-3, BCMA, BAFFR, GITR, IL-2, IL-12A, IL-12B, IL-15, IL-23, IL-27, FLT3L, IL-36A, IL-36B, IL-36C, GM-CSF, CCL20, CXCL9, CXCL10, CXCL11, CXCL12, CCR7, CXCR4, CCR5, CCL4, CCL5, CCL19, CXCR3, CCR6, B7-H1, B7-DC, B7-H3, B7-H4, PD-1H, IL-10, TGF-β, HGF, B7-1, B7-2, B7-H2, B7-H3, CD40, FasL, CD70, CD30L, 4-1BBL, OX40L, TRAIL, RANKL, TWEAK, APRIL, BAFF, LIGHT, GITRL, CD73, STAT1, IRF4, CCAR2, BCL6, iNOS and CD103.

Preferably, a nucleic acid encoding a CAR molecule comprises a sequence capable of recognizing at least one molecule selected from the group consisting of CD19, BMCA, CD22, CD20, CD123, GD2, CD30, GPC3, CLDN18.2, Mesothelin, CD33, CD38, EGFRvlll, CD138, CEA, HER2, PSMA, CLL1, CD56, EGFR, MUC-1, EpCAM, CD7, NKG2D, PD-L1, LewisY, FAP, c-MET, ROR1, IL13Rα2, AFP, CD133, CD4, BTK, ROBO1, CD5, CD70, LILRB4, FLT3, Sigle-6, CD229 and SLAMF7.

In a twelfth aspect, the present invention provides a pharmaceutical formulation, comprising the pharmaceutical composition, and a pharmaceutically acceptable carrier;
preferably, the pharmaceutical formulation is an aqueous injection, a freeze-dried powder, or a spray.

Beneficial effects:
The present invention provides a molecule having targeting function, which can specifically deliver drugs to specific cells such as T cells, B cells, NK cells, NKT cells, γδT cells, regulatory T cells, eosinophils, basophils, mast cells, neutrophils, platelets, mesenchymal stem cells, macrophages, plasma cells, dendritic cells, microglia, neuronal cells, astrocytes, endothelial cells, osteoclasts, osteoblasts, synovial cells, tumor cells, etc. For example, by delivering a nucleic acid sequence for a CAR molecule specifically to T cells, cytotoxic T cells targeting specific tumors can be directly generated in vivo to kill tumors; for example, by delivering a nucleic acid sequence for a TCR molecule specifically to T cells, TCR-T cells targeting specific antigen sequences can be directly generated in vivo to kill virus-infected cells or tumors; for example, by delivering an immune-functional molecule or a molecule that blocks cellular dysfunction specifically to immune cells, the functions of the immune cells can be altered and immune responses can be precisely regulated; for example, by delivering a functional regulatory molecule specifically to other types of cells, target cells can be precisely programmed in vivo to enable the functional expression of a therapeutic molecule. Specific cellular delivery can significantly enhance drug efficacy and reduce side effects caused by drugs acting on non-target cells, achieving the following effects: first, the pharmacologically active molecule can directly enter target cells, even those traditionally inaccessible to conventional drugs, to exert its effects; second, maximum therapeutic effects can be achieved with extremely low administration dosages.

### Brief Description of the Drawings

To provide a clearer illustration of the technical solutions in the present invention or the prior art, the following is an explanation of the Figures required for describing the Examples or the prior art.
Figure 1A and Figure 1B show the quality control information for LNP containing a conjugation site in Example 20 of the present invention.
Figure 2A, Figure 2B, Figure 2C, and Figure 2D show the *in vitro* transfection efficiency of LNPs with different targeting molecular structures in Example 20 of the present invention.
Figure 3 shows the in vivo transfection efficiency of TLNPs with different targeting molecular structures in Example 20 of the present invention.
Figure 4A and Figure 4B show specific characterization data of Pre-TLNPs in Example 21 of the present invention.
Figure 5A and Figure 5B show specific characterization data of TLNPs in Example 21 of the present invention.
Figure 6 shows the in vivo delivery efficiency of TLNPs with varying ratios of targeting molecules in Example 21 of the present invention.
Figure 7A, Figure 7B, Figure 7C, Figure 7D, and Figure 7E show flow cytometry analysis of EGFP-mRNA expression efficiency in hPBMCs for cLNP-1 and TLNP-2 in Example 24 of the present invention.
Figure 8 shows the in vivo transfection efficiency of TLNP-2 at different dosages in Example 24 of the present invention.
Figure 9A, Figure 9B, Figure 9C, Figure 9D, and Figure 9E are statistical graphs showing the in vivo transfection efficiency of TLNP-2 with different formulations in Example 25 of the present invention.
Figure 10A, Figure 10B, Figure 10C, Figure 10D, Figure 10E, and Figure 10F show a long-term stability study on conventional LNPs and targeted TLNPs in Example 27 of the present invention.
Figure 11A, Figure 11B, Figure 11C, and Figure 11D show the biodistribution results in Balb/c mice using Cy5 fluorescence, Luc imaging, and qPCR methods in Example 29 of the present invention.
Figure 12A and Figure 12B show the biodistribution results in NGC mice using Luc imaging and qPCR methods in Example 29 of the present invention.
Figure 13 shows dosage safety study data of TLNP-2 at different working concentrations in hPBMCs in Example 30 of the present invention.
Figure 14 shows liver toxicity indicator detection results from acute toxicity experiments of conventional cLNP and targeted TLNP at different dosages in mice in Example 31 of the present invention.
Figure 15 shows in vivo EGFP expression in NHP for TLNP-2 in Example 32 of the present invention.
Figure 16 shows the EGFP targeted and off-target results of TLNP-2 in NHP in Example 32 of the present invention.
Figure 17 shows the dynamic changes in EGFP expression of TLNP-2 in NHP in Example 32 of the present invention.
Figure 18 shows the body weight changes in NHP after administration of TLNP-2 in Example 33 of the present invention.
Figure 19 shows the body temperature changes in NHP after administration of TLNP-2 in Example 33 of the present invention.
Figure 20 shows the Complete Blood Count (CBC) changes in NHP after administration of TLNP-2 in Example 33 of the present invention.
Figure 21 shows the cytokine changes in the serum of NHP after administration of TLNP-2 in Example 33 of the present invention.
Figure 22A, Figure 22B, Figure 22C, Figure 22D, Figure 22E, Figure 22F, Figure 22G, Figure 22H, Figure 22I, and Figure 22J show the blood biochemistry results in NHP after administration of TLNP-2 in Example 33 of the present invention.
Figure 23A, Figure 23B, Figure 23C, and Figure 23D show the *in vitro* transfection results of CD5-TLNPs containing different targeting molecules in Example 34 of the present invention.
Figure 24A and Figure 24B show the in *vitro* transfection results of CD5-TLNPs with different ratios of targeting molecules in Example 35 of the present invention.
Figure 25A and Figure 25B show the in vivo transfection efficiency of L2-TLNPs with different formulations in Example 36 of the present invention.
Figure 26A and Figure 26B show the biodistribution experimental results in hCD5 humanized mice using Luc imaging and qPCR methods in Example 37 of the present invention.

### Specific Mode for Carrying out the Present Invention

This present invention provides certain molecules having targeting functions and their applications. Among the more specific embodiments, the present invention offers lipid nanoparticles (LNPs) with targeting function. These LNPs having targeting function comprise ionizable amino lipids, polymer-conjugated lipids, and nanobodies; optionally, these LNPs having targeting functions may also comprise steroids and/or phospholipids.

To better illustrate the methods for obtaining the a LNP having targeting function described in the present invention, explanations are provided below through Examples.

### (I) Preparation Method for Five-Component Cell-Targeting Nanoparticle (Comprising an Ionizable Amino Lipids, a Polymer-Conjugated Lipid, a Steroid, a Phospholipid, and a Nanobody)

### Method 1: Post-Insertion Chemical Conjugation Method

The ionizable amino lipid E12LA6B6O3, phospholipid, cholesterol, DMG-PEG2000, and a PEG lipid derivative containing a chemical conjugation site were dissolved in ethanol at a specific molar ratio. The mRNA was dissolved in an acidic buffer solution to ensure a concentration of 0.33 mg/mL in the aqueous phase. The molar ratio of the ionizable amino lipid to mRNA (N/P) was set at 6.5.

The ethanol phase and the aqueous phase were mixed using a microfluidic device, with the flow rate controlled such that the ratio of the ethanol phase to the aqueous phase is 1 : 3. 25 mM Tris buffer is added to the crude LNP product to terminate the reaction. Subsequently, ultrafiltration purification was performed once at 4°C and 1,200 rcf, followed by the addition of 25 mM Tris buffer and two further rounds of ultrafiltration purification. The LNPs containing a chemical conjugation site (Pre-TLNPs) were then collected. After adding a volume of 400 mg/mL sucrose solution equivalent to 0.278 times the volume of the LNPs, sterile filtration was performed using a PES filter with a pore size of 0.22 µm in a laminar flow hood.

A certain amount of the aforementioned Pre-TLNP was mixed with a corresponding nanobody at a specific molar ratio and incubated overnight at 4°C. The LNP mixture after overnight incubation was diluted with an sucrose solution of 87 mg/mL and purified using a 100 KD ultrafiltration tube. After sterile filtration using a PES filter with a pore size of 0.22 µm in a laminar flow hood, the final nanobody-modified cell-targeting nanoparticles (TLNPs) were obtained.

### Method 2: Post-Insertion Incubation Method for Targeting Functional Molecule

The PEG lipid derivative containing a chemical conjugation site was mixed with a nanobody at a specific molar ratio in PBS buffer solution and incubated overnight at 4°C. The reaction solution was then purified by column chromatography to obtain the targeting functional PEG lipid-nanobody conjugate (PEG-Nb).

The ionizable amino lipid, phospholipid, cholesterol, and DMG-PEG2000 were dissolved in ethanol at a specific molar ratio. The mRNA was dissolved in an acidic buffer solution to ensure a concentration of 0.33 mg/mL in the aqueous phase. The molar ratio of the ionizable amino lipid to mRNA (N/P) was set at 6.5. The LNP preparation process was carried out with reference to the description in Method 1.

A certain amount of the LNPs prepared above was mixed with the targeting functional molecule (PEG-Nb) at a specific molar ratio and incubated overnight at 4°C. Subsequent LNP purification processes and quality control methods were carried out as described in Method 1, ultimately yielding nanobody-modified cell-targeting nanoparticles (TLNPs).

### Method 3: One-Step Insertion Method for Targeting Functional Molecule

The ionizable amino lipid, phospholipid, cholesterol, and DMG-PEG2000 were dissolved in ethanol at a specific molar ratio. The targeting functional molecule (PEG lipid-nanobody conjugate) and mRNA were dissolved in an acidic buffer solution to ensure a concentration of 0.33 mg/mL of mRNA in the aqueous phase. The molar ratio of the ionizable amino lipid to mRNA (N/P) was set at 6.5. Subsequent LNP preparation, purification, and quality control processes were carried out with reference to the description in Method 1, resulting in nanobody-modified cell-targeting nanoparticles (TLNPs).

### (II) Preparation Method for Four-Component Cell-Targeting Nanoparticle (Comprising an Ionizable Amino Lipids, a Polymer-Conjugated Lipid, a Steroid, and a Nanobody)

The ionizable amino lipid, cholesterol, DMG-PEG2000, and DSPE-PEG2000-Mal lipids were dissolved in ethanol at a molar ratio of 40:58.5:1.45:0.05. The mRNA was dissolved in an acidic buffer solution to ensure a concentration of 0.33 mg/mL in the aqueous phase. The molar ratio of the ionizable amino lipid to mRNA (N/P) was set at 6.5.

Subsequent LNP preparation processes were carried out with reference to the description in Method 1 of (I), resulting in the final four-component cell-targeting nanoparticles (TLNPs).

### (III) Preparation Method for Four-Component Cell-Targeting Nanoparticle (Comprising an Ionizable Amino Lipids, a Polymer-Conjugated Lipid, a Phospholipid, and a Nanobody)

The ionizable amino lipid, phospholipid DSPC, DMG-PEG2000, and DSPE-PEG2000-Mal lipids were dissolved in ethanol at a molar ratio of 47.5 : 51.0 : 1.45 : 0.05. The mRNA was dissolved in an acidic buffer solution to ensure a concentration of 0.33 mg/mL in the aqueous phase. The molar ratio of the ionizable amino lipid to mRNA (N/P) was set at 6.5.

Subsequent LNP preparation processes were carried out with reference to the description in Method 1 of (I), resulting in the final four-component cell-targeting nanoparticles (TLNPs).

The technical solution provided by the present invention will be elaborated in detail below in conjunction with the Examples, but these Examples should not be construed as limiting the scope of protection of the present invention. Unless otherwise specified, the experimental methods used in the Examples are all conventional methods; the materials, reagents, etc., used can be obtained through commercial channels.

### Example 1:

In this Example, the post-insertion incubation method for targeting functional molecule was employed to prepare five-component cell-targeting nanoparticles (containing an ionizable amino lipid, a polymer-conjugated lipid, a steroid, a phospholipid, and a nanobody).

The DSPE-PEG2000-Mal lipid containing a chemical conjugation site was mixed with CD8 nanobody (with or without a GS-cysteine at the terminal end) at a molar ratio of 1 : 1 in PBS as a reaction medium and incubated overnight at 4°C. After purification using a hydrophobic interaction chromatography column, the conjugate of CD8 nanobody and DSPE-PEG2000-Mal lipid, namely the CD8 targeting functional molecule, was obtained.

The ionizable amino lipid E12LA6B6O3, phospholipid, cholesterol (CHO), and DMG-PEG2000 were dissolved in ethanol according to the molar ratio specified in Table 1. The mRNA was dissolved in an acidic buffer solution to ensure a concentration of 0.33 mg/mL in the aqueous phase. The molar ratio of the ionizable amino lipid to mRNA (N/P) was set at 6.5.

The ethanol phase and the aqueous phase were mixed using a microfluidic device, with the flow rate controlled such that the volume ratio of the ethanol phase to the aqueous phase was 1 : 3. The total flow rate of the two phases was 12 mL/min, with a flow rate ratio of the ethanol phase to the aqueous phase of 1 : 3, resulting in the preparation of crude LNPs. Immediately after LNPs preparation, 10 volumes of 25 mM Tris buffer (prepared with Tris/Tris-HCl, pH = 7.4-7.5) were added to terminate the reaction.

The crude LNPs with the termination buffer were subjected to ultrafiltration purification once at 4°C and 1200 rcf; subsequently, 25 mM Tris buffer ultrafiltrate was added, and the mixture was further ultrafiltrated and concentrated twice at 4°C and 1200 rcf. After collecting the finished LNPs, a volume of 400 mg/mL sucrose solution (prepared by volumetric method with 25 mM Tris buffer, pH = 7.4-7.5) equivalent to 0.278 times the volume of the LNPs was added, and sterile filtration was performed using a PES filter with a pore size of 0.22 µm in a laminar flow hood. 30 µL of the resultant sample was taken for the detection of particle size, polydispersity index, and encapsulation efficiency, and the remaining sample was stored in a refrigerator at 4°C.

A certain amount of the aforementioned LNPs was mixed with the CD8 targeting functional molecule according to the formulation specified in Table 1 and incubated overnight at 4°C. The LNP mixture after overnight incubation was diluted with an 87 mg/mL sucrose solution (prepared with 25 mM Tris buffer, pH = 7.4-7.5) and purified using a 100 KD ultrafiltration tube. After sterile filtration using a PES filter with a pore size of 0.22 µm in a laminar flow hood, CD8 nanobody-modified cell-targeting nanoparticles (CD8-TLNPs) were obtained.

**Table 1: Formulation table for the molar ratios of lipids and targeting molecule in the preparation of CD8-TLNPs using the post-insertion method.**

| Group | Molar Ratios of Lipids and Targeting Molecule (E12LA6B6O3 : DSPC : CHO : DMG-PEG2000 : CD8 targeting functional molecule) |
|---|---|
| CD8-TLNP1 | 47.5 : 10 : 41 : 1.5 : 0.01 |
| CD8-TLNP2 | 50 : 10 : 38.5 : 1.5 : 0.01 |
| CD8-TLNP3 | 45 : 10 : 43.5 : 1.5 : 0.01 |
| CD8-TLNP4 | 40 : 20 : 38.5 : 1.5 : 0.01 |
| CD8-TLNP5 | 55 : 15 : 28.5 : 1.5 : 0.01 |
| CD8-TLNP6 | 47.5 : 15 : 36 : 1.0 : 0.01 |

### Example 2:

In this Example, the one-step insertion method for Targeting Functional Molecule was employed to prepare five-component cell-targeting nanoparticles (containing an ionizable amino lipid, a polymer-conjugated lipid, a steroid, a phospholipid, and a targeting functional molecule).

The CD8 targeting functional molecule was prepared according to the method described in Example 1.

The ionizable amino lipid E12LA6B6O3, phospholipid, cholesterol, and DMG-PEG2000 were dissolved in ethanol according to the molar ratio specified in Table 2. The CD8 targeting functional molecule and mRNA were dissolved in an acidic buffer solution to ensure a concentration of 0.33 mg/mL of mRNA in the aqueous phase. The molar ratio of the ionizable amino lipid to mRNA (N/P) was set at 6.5.

The ethanol phase and the aqueous phase were mixed using a microfluidic device, with the flow rate controlled such that the volume ratio of the ethanol phase to the aqueous phase was 1 : 3. The total flow rate of the two phases was 12 mL/min, with a flow rate ratio of the ethanol phase to the aqueous phase of 1 : 3, resulting in the preparation of crude LNPs. Immediately after LNP preparation, 10 volumes of 25 mM Tris buffer (prepared with Tris/Tris-HCl, pH = 7.4-7.5) were added to terminate the reaction.

The crude LNPs with the termination buffer were subjected to ultrafiltration purification once at 4°C and 1200 rcf; subsequently, 25 mM Tris buffer ultrafiltrate was added, and the mixture was further ultrafiltrated and concentrated twice at 4°C and 1200 rcf. After collecting the finished LNPs, a volume of 400 mg/mL sucrose solution (prepared by volumetric method with 25 mM Tris buffer, pH = 7.4-7.5) equivalent to 0.278 times the volume of the LNPs was added, and sterile filtration was performed using a PES filter with a pore size of 0.22 µm in a laminar flow hood, yielding CD8 nanobody-modified cell-targeting nanoparticles (CD8-TLNPs).

**Table 2: Formulation table for the molar ratios of lipids and targeting molecule in the preparation of CD8-TLNPs using one-step insertion method**

| Group | Molar Ratios of Lipids and Targeting Molecule (E12LA6B6O3 : DSPC : CHO : DMG-PEG2000 : CD8 targeting functional molecule) |
|---|---|
| CD8-TLNP1 | 47.5:10: 41:1.5:0.01 |
| CD8-TLNP2 | 50:10:38.5:1.5:0.01 |
| CD8-TLNP3 | 45:10:43.5:1.5:0.01 |
| CD8-TLNP4 | 40:20:38.5:1.5:0.01 |
| CD8-TLNP5 | 55:15:28.5:1.5:0.01 |
| CD8-TLNP6 | 47.5:15: 36:1.0:0.01 |

### Example 3

In this Example, the post-insertion chemical conjugation method was employed to prepare five-component cell-targeting nanoparticles (containing an ionizable amino lipid, a polymer-conjugated lipid, a steroid, a phospholipid, and a nanobody). The specific steps are as follows:
The ionizable amino lipid E12LA6B6O3, phospholipid, cholesterol, DMG-PEG2000, and DSPE-PEG2000-Mal were dissolved in ethanol according to the molar ratio specified in Table 3. The mRNA was dissolved in an acidic buffer solution to ensure a concentration of 0.33 mg/mL of mRNA in the aqueous phase. The molar ratio of the ionizable amino lipid to mRNA (N/P) was set at 6.5.

The ethanol phase and the aqueous phase were mixed using a microfluidic device, with the flow rate controlled such that the volume ratio of the ethanol phase to the aqueous phase was 1 : 3. The total flow rate of the two phases was 12 mL/min, with a flow rate ratio of the ethanol phase to the aqueous phase of 1 : 3, resulting in the preparation of crude LNPs. Immediately after LNP preparation, 10 volumes of 25 mM Tris buffer (prepared with Tris/Tris-HCl, pH = 7.4-7.5) were added to terminate the reaction.

The crude LNPs with the termination buffer were subjected to ultrafiltration purification once at 4 °C and 1200 rcf; subsequently, 25 mM Tris buffer ultrafiltrate was added, and the mixture was further ultrafiltrated and concentrated twice at 4 °C and 1200 rcf. After collecting the LNPs with a chemical conjugation site (Pre-TLNP), a volume of 400 mg/mL sucrose solution (prepared by volumetric method with 25 mM Tris buffer, pH = 7.4-7.5) equivalent to 0.278 times the volume of the LNPs was added, and sterile filtration was performed using a PES filter with a pore size of 0.22 µm in a laminar flow hood. 30 µL of the sample was taken for the detection of particle size, polydispersity index, and encapsulation efficiency, and the remaining sample was stored in a refrigerator at 4 °C.

A certain amount of the aforementioned Pre-TLNP was taken, and DSPE-PEG2000-Mal was mixed with a corresponding CD8 nanobody (with or without a terminal GS-cysteine) at a molar ratio of 1 : 1 and incubated overnight at 4 °C. The LNP mixture after overnight incubation was diluted with an 87 mg/mL sucrose solution (prepared with 25 mM Tris buffer, pH = 7.4-7.5) and purified using a 100 KD ultrafiltration tube. After sterile filtration using a PES filter with a pore size of 0.22 µm in a laminar flow hood, the final nanobody-modified cell-targeting nanoparticles (TLNPs) were obtained. 30 µL of the sample was taken for the detection of particle size, polydispersity index, and encapsulation efficiency, and the remaining sample was stored in a refrigerator at 4 °C. After the test for encapsulation efficiency, the sample was diluted to the desired concentration with an 87 mg/mL sucrose solution (prepared with 25 mM Tris buffer, pH = 7.4-7.5) and stored for future use.

**Table 3: Formulation table for the ratios of lipids in the preparation of CD8-TLNPs using post-insertion method**

| Group | Molar Ratios of five Lipids (E12LA6B6O3 : DSPC : CHO : DMG-PEG2000 : DSPE-PEG2000-Mal) |
|---|---|
| CD8-TLNP1 | 47.5 : 10 : 41 : 1.5 : 0.01 |
| CD8-TLNP2 | 50 : 10 : 38.5 : 1.5 : 0.01 |
| CD8-TLNP3 | 45 : 10 : 43.5 : 1.5 : 0.01 |
| CD8-TLNP4 | 40 : 20 : 38.5 : 1.5 : 0.01 |
| CD8-TLNP5 | 55 : 15 : 28.5 : 1.5 : 0.01 |
| CD8-TLNP6 | 47.5 : 15 : 36 : 1.0 : 0.01 |

### Example 4

### (I) Preparation of Nanoparticles Conjugated with anti-CD19 Nanobody

Refer to the preparation method in Example 3. The ionizable amino lipid, DSPC, CHO, DMG-PEG2000, and DSPE-PEG2000-Mal were dissolved in ethanol at a molar ratio of 47.5 : 10 : 41 : 1.49 : 0.01. The mRNA was dissolved in an acidic buffer solution containing 75 mM citric acid at pH 4 to ensure a concentration of 0.33 mg/mL of mRNA in the aqueous phase. The molar ratio of the ionizable amino lipid to mRNA (N/P) was set at 6.5.

The ethanol phase and the aqueous phase were mixed using a microfluidic device, with the flow rate controlled such that the volume ratio of the ethanol phase to the aqueous phase was 1 : 3. The total flow rate of the two phases was 12 mL/min, with a flow rate ratio of the ethanol phase to the aqueous phase of 1 : 3, resulting in the preparation of crude LNPs. Immediately after LNPs preparation, 10 volumes of 25 mM Tris buffer (prepared with Tris/Tris-HCl, pH = 7.4-7.5) were added to terminate the reaction.

The crude LNPs were subjected to ultrafiltration purification three times at 4°C and 1200 rcf, and LNPs with a chemical conjugation site (Pre-TLNPs) were collected. Then, Pre-TLNPs were mixed with anti-CD19 nanobody with a terminal cysteine residue at a molar ratio of 1 : 1 and incubated overnight at 4°C. Subsequently, after ultrafiltration washing with an 87 mg/mL sucrose solution and sterile filtration, the final nanoparticles conjugated with anti-CD19 nanobody (CD19-TLNPs) were obtained.

### (II) Evaluation of In Vitro Delivery Efficiency of Nanoparticles Targeting Human CD19 Cells

Anti-CD3 Coating: One day in advance, 200 µL of anti-Human-CD3 antibody at a concentration of 1 µg/mL was added to each well of a 24-well plate. The plate was then stored at 4°C and allowed to be coated overnight.

Preparation before Cell Plating: The liquid in the 24-well plate was aspirated out. Subsequently, the wells were washed twice with 1640 complete medium (containing 10% New Zealand serum and 1% PS (penicillin-streptomycin) dual antibiotic).

Cell Plating: Human PBMCs were diluted to a concentration of 1 million/mL using 1640 complete medium (containing 10% New Zealand serum and 1% PS dual antibiotic) supplemented with anti-CD28 antibody at a concentration of 1 µg/mL and IL-2 at a working concentration of 400 U/mL. One million cells were added to each well of the 24-well plate. After cell plating, the cells were stabilized for 4 h under conditions of 37 °C and 5% CO₂ before LNPs were added.

CD19-TLNPs encapsulating eGFP-mRNA were added so that the working concentration of LNPs in the well plate reached 0.1 µg/mL. In addition, a negative control group was established by adding phosphate-buffered saline (PBS). After 24 h, the expression ratio of eGFP-mRNA in the CD19+ cell subset of PBMCs was detected by flow cytometry. .

**Table 4: In Vitro Transfection Results of CD19-TLNP**

| LNP No. | Positive Rate of CD19+ Cells (%) |
|---|---|
| PBS | 0.1 |
| CD19-TLNP | 70 |

The experimental results shows that CD19-TLNPs exhibited a significant targeting effect on CD19+ cells and had a relatively good transfection efficiency.

### Example 5:

### Preparation of Nanoparticles Conjugated with anti-CD133 Nanobody

Refer to the preparation method in Example 3. The ionizable amino lipid, DSPE, CHO, DMG-PEG4000, andDSPE-PEG4000-Mal were dissolved in ethanol at a molar ratio of 50.5 : 5 : 43 : 1.47 : 0.03. The mRNA was dissolved in an acidic buffer solution containing 75 mM citric acid at pH 4 to ensure a concentration of 0.33 mg/mL of mRNA in the aqueous phase. The molar ratio of the ionizable amino lipid to mRNA (N/P) was set at 6.5.

The ethanol phase and the aqueous phase were mixed using a microfluidic device, with the flow rate controlled such that the volume ratio of the ethanol phase to the aqueous phase was 1 : 3. The total flow rate of the two phases was 12 mL/min, with a flow rate ratio of the ethanol phase to the aqueous phase of 1 : 3, resulting in the preparation of crude LNPs. Immediately after LNPs preparation, 10 volumes of 25 mM Tris buffer (prepared with Tris/Tris-HCl, pH = 7.4-7.5) were added to terminate the reaction.

The crude LNPs were subjected to ultrafiltration purification three times at 4°C and 1200 rcf, and LNPs with a chemical conjugation site (Pre-TLNPs) were collected. Then, Pre-TLNPs were mixed with anti-CD133 nanobody with a terminal cysteine residue at a molar ratio of 1:1.2 and incubated overnight at 4°C. Subsequently, after ultrafiltration washing with an 87 mg/mL sucrose solution and sterile filtration, the final nanoparticles conjugated with anti-CD133 nanobody (CD133-TLNPs) were obtained.

### Example 6:

### (I) Preparation of Nanoparticles Conjugated with anti-PSMA Nanobody

Refer to the preparation method in Example 3. The ionizable amino lipid, DOPC, CHO, DMG-PEG2000, and DSPE-PEG3000-Mal were dissolved in ethanol at a molar ratio of 43.5 : 12 : 43 : 1.2 : 0.3. The mRNA was dissolved in an acidic buffer solution containing 75 mM citric acid at pH 4 to ensure a concentration of 0.33 mg/mL of mRNA in the aqueous phase. The molar ratio of the ionizable amino lipid to mRNA (N/P) was set at 6.5.

The ethanol phase and the aqueous phase were mixed using a microfluidic device, with the flow rate controlled such that the volume ratio of the ethanol phase to the aqueous phase was 1 : 3. The total flow rate of the two phases was 12 mL/min, with a flow rate ratio of the ethanol phase to the aqueous phase of 1 : 3, resulting in the preparation of crude LNPs. Immediately after LNPs preparation, 10 volumes of 25 mM Tris buffer (prepared with Tris/Tris-HCl, pH = 7.4-7.5) were added to terminate the reaction.

The crude LNPs were subjected to ultrafiltration purification three times at 4°C and 1200 rcf, and LNPs with a chemical conjugation site (Pre-TLNPs) were collected. Then, Pre-TLNPs were mixed with anti-PSMA nanobody with a terminal cysteine residue at a molar ratio of 1.2:1 and incubated overnight at 4°C. Subsequently, after ultrafiltration washing with an 87 mg/mL sucrose solution and sterile filtration, the final nanoparticles conjugated with anti-PSMA nanobody (PSMA-TLNPs) were obtained.

### (II) Evaluation of In Vitro Delivery Efficiency of Nanoparticles Targeting Human PSMA Cells

LNCaP cells were thawed and cultured. PSMA-TLNPs encapsulating eGFP-mRNA were added such that the working concentration of LNPs in the well plate reached 0.1 µg/mL. In addition, a negative control group was established by adding phosphate-buffered saline (PBS). After 24 h, the expression ratio of eGFP-mRNA in the PSMA+ cells of LNCaP cells was detected by flow cytometry. .

**Table 5: In Vitro Transfection Results of PSMA-TLNPs**

| LNP No. | Positive Rate of PSMA+ Cells (%) |
|---|---|
| PBS | 0.13 |
| PSMA-TLNP | 76.5 |

The experimental results shows that PSMA-TLNPs exhibited a significant targeting effect on PSMA+ cells and had relatively good transfection efficiency.

### Example 7:

### (I) Preparation of Nanoparticles Conjugated with anti-TMEM119 Nanobody

Refer to the preparation method in Example 3. The ionizable amino lipid, DOPE, cholesterol ester, DMG-PEG2000, and DSPE-PEG2000-Mal were dissolved in ethanol at a molar ratio of 55.5: 0: 43: 1.495: 0.005. The mRNA was dissolved in an acidic buffer solution containing 75 mM citric acid at pH 4 to ensure a concentration of 0.33 mg/mL of mRNA in the aqueous phase. The molar ratio of the ionizable amino lipid to mRNA (N/P) was set at 6.5.

The ethanol phase and the aqueous phase were mixed using a microfluidic device, with the flow rate controlled such that the volume ratio of the ethanol phase to the aqueous phase was 1 : 3. The total flow rate of the two phases was 12 mL/min, with a flow rate ratio of the ethanol phase to the aqueous phase of 1 : 3, resulting in the preparation of crude LNPs. Immediately after LNPs preparation, 10 volumes of 25 mM Tris buffer (prepared with Tris/Tris-HCl, pH = 7.4-7.5) were added to terminate the reaction.

The crude LNPs were subjected to ultrafiltration purification three times at 4°C and 1200 rcf, and LNPs with a chemical conjugation site (Pre-TLNPs) were collected. Then, Pre-TLNPs were mixed with anti-TMEM119 nanobody with a terminal cysteine residue at a molar ratio of 1 : 1 and incubated overnight at 4°C. Subsequently, after ultrafiltration washing with an 87 mg/mL sucrose solution and sterile filtration, the final nanoparticles conjugated with anti-TMEM119 nanobody (TMEM119-TLNPs) were obtained.

### (II) Evaluation of In Vitro Delivery Efficiency of Nanoparticles Targeting Human TMEM119 Cells

SH-SY5Y cells were thawed and cultured. TMEM119-TLNPs encapsulating eGFP-mRNA were added such that the working concentration of LNPs in the well plate reached 0.1 µg/mL. In addition, a negative control group was established by adding phosphate-buffered saline (PBS). After 24 h, the expression ratio of eGFP-mRNA in the TMEM119+ cells of SH-SY5Y cells was detected by flow cytometry.

**Table 6: In Vitro Transfection Results of TMEM119-TLNPs**

| LNP No. | Positive Rate of TMEM119+ Cells (%) |
|---|---|
| PBS | 0.11 |
| TMEM119-TLNP | 78.4 |

The experimental results shows that TMEM119-TLNPs exhibited a significant targeting effect on TMEM119+ cells and had relatively good transfection efficiency.

### Example 8:

### Preparation of Nanoparticles Conjugated with anti-B7-H1 Nanobody

Refer to the preparation method in Example 3. The ionizable amino lipid, DSPC, CHO, DMG-PEG2000, and DSPE-PEG2000-Mal were dissolved in ethanol at a molar ratio of 62.5 : 5 : 31 : 1.49 : 0.01. The mRNA was dissolved in an acidic buffer solution containing 75 mM citric acid at pH 4 to ensure a concentration of 0.33 mg/mL of mRNA in the aqueous phase. The molar ratio of the ionizable amino lipid to mRNA (N/P) was set at 6.5.

The ethanol phase and the aqueous phase were mixed using a microfluidic device, with the flow rate controlled such that the volume ratio of the ethanol phase to the aqueous phase was 1 : 3. The total flow rate of the two phases was 12 mL/min, with a flow rate ratio of the ethanol phase to the aqueous phase of 1 : 3, resulting in the preparation of crude LNPs. Immediately after LNPs preparation, 10 volumes of 25 mM Tris buffer (prepared with Tris/Tris-HCl, pH = 7.4-7.5) were added to terminate the reaction.

The crude LNPs were subjected to ultrafiltration purification three times at 4°C and 1200 rcf, and LNPs with a chemical conjugation site (Pre-TLNPs) were collected. Then, Pre-TLNPs were mixed with anti-B7-H1 nanobody with a terminal cysteine residue at a molar ratio of 1 : 1 and incubated overnight at 4°C. Subsequently, after ultrafiltration washing with an 87 mg/mL sucrose solution and sterile filtration, the final nanoparticles conjugated with anti-B7-H1 nanobody (B7-H1-TLNPs) were obtained.

### Example 9:

### (I) Preparation of Nanoparticles Conjugated with anti-HER2 Nanobody

Refer to the preparation method in Example 3. The ionizable amino lipid, DOPC, CHO, DMG-PEG2000, and DSPE-PEG2000-Mal were dissolved in ethanol at a molar ratio of 58.5 : 10 : 30 : 1.49 : 0.01. The mRNA was dissolved in an acidic buffer solution containing 75 mM citric acid at pH 4 to ensure a concentration of 0.33 mg/mL of mRNA in the aqueous phase. The molar ratio of the ionizable amino lipid to mRNA (N/P) was set at 6.5.

The ethanol phase and the aqueous phase were mixed using a microfluidic device, with the flow rate controlled such that the volume ratio of the ethanol phase to the aqueous phase was 1 : 3. The total flow rate of the two phases was 12 mL/min, with a flow rate ratio of the ethanol phase to the aqueous phase of 1 : 3, resulting in the preparation of crude LNPs. Immediately after LNPs preparation, 10 volumes of 25 mM Tris buffer (prepared with Tris/Tris-HCl, pH = 7.4-7.5) were added to terminate the reaction.

The crude LNPs were subjected to ultrafiltration purification three times at 4°C and 1200 rcf, and LNPs with a chemical conjugation site (Pre-TLNPs) were collected. Then, Pre-TLNPs were mixed with anti-HER2 nanobody with a terminal cysteine residue at a molar ratio of 1 : 1 and incubated overnight at 4°C. Subsequently, after ultrafiltration washing with an 87 mg/mL sucrose solution and sterile filtration, the final nanoparticles conjugated with anti-HER2 nanobody (HER2-TLNPs) were obtained.

### (II) Evaluation of In Vitro Delivery Efficiency of Nanoparticles Targeting Human HER2 Cells

Human breast cancer SK-BR-3 cells were thawed and cultured. HER2-TLNPs encapsulating eGFP-mRNA were added such that the working concentration of LNPs in the well plate reached 0.1 µg/mL. In addition, a negative control group was established by adding phosphate-buffered saline (PBS). After 24 h, the expression ratio of eGFP-mRNA in the HER2+ cells of PBMCs was detected by flow cytometry.

**Table 7: In Vitro Transfection Results of HER2-TLNPs**

| LNP No. | Positive Rate of HER2+ Cells (%) |
|---|---|
| PBS | 0.13 |
| HER2-TLNP | 77.3 |

The experimental results shows that HER2-TLNPs exhibited a significant targeting effect on HER2+ cells and had relatively good transfection efficiency.

### Example 10:

### Preparation of Nanoparticles Conjugated with anti-EGFR Nanobody

Refer to the preparation method in Example 3. The ionizable amino lipid, DSPC, CHO, DMG-PEG2000, and DSPE-PEG2000-Mal were dissolved in ethanol at a molar ratio of 45.5 : 15 : 38 : 1.49 : 0.01. The mRNA was dissolved in an acidic buffer solution containing 75 mM citric acid at pH 4 to ensure a concentration of 0.33 mg/mL of mRNA in the aqueous phase. The molar ratio of the ionizable amino lipid to mRNA (N/P) was set at 6.5.

The ethanol phase and the aqueous phase were mixed using a microfluidic device, with the flow rate controlled such that the volume ratio of the ethanol phase to the aqueous phase was 1 : 3. The total flow rate of the two phases was 12 mL/min, with a flow rate ratio of the ethanol phase to the aqueous phase of 1 : 3, resulting in the preparation of crude LNPs. Immediately after LNPs preparation, 10 volumes of 25 mM Tris buffer (prepared with Tris/Tris-HCl, pH = 7.4-7.5) were added to terminate the reaction.

The crude LNPs were subjected to ultrafiltration purification three times at 4°C and 1200 rcf, and LNPs with a chemical conjugation site (Pre-TLNPs) were collected. Then, Pre-TLNPs were mixed with anti-EGFR nanobody with a terminal cysteine residue at a molar ratio of 1 : 1 and incubated overnight at 4°C. Subsequently, after ultrafiltration washing with an 87 mg/mL sucrose solution and sterile filtration, the final nanoparticles conjugated with anti-EGFR nanobody (EGFR-TLNPs) were obtained.

### Example 11:

### (I) Preparation of Nanoparticles Targeting human CD7 Cells

Refer to the preparation method in Example 3. Pre-TLNPs were mixed with anti-CD7 nanobody with a terminal cysteine residue at a molar ratio of 1 : 1 and incubated overnight at 4°C. Subsequently, after ultrafiltration washing with an 87 mg/mL sucrose solution and sterile filtration, the final nanoparticles conjugated with anti-CD7 nanobody (CD7-TLNPs) were obtained.

### (II) Evaluation of In Vitro Delivery Efficiency of Nanoparticles Targeting Human CD7 Cells

Anti-CD3 Coating: One day in advance, 200 µL of anti-Human-CD3 antibody at a concentration of 1 µg/mL was added to each well of a 24-well plate. The plate was then stored at 4°C and allowed to be coated overnight.

Preparation before Cell Plating: The liquid in the 24-well plate was aspirated out. Subsequently, the wells were washed twice with 1640 complete medium (containing 10% New Zealand serum and 1% PS (penicillin-streptomycin) dual antibiotic).

Cell Plating: Human PBMCs were diluted to a concentration of 1 million/mL using 1640 complete medium (containing 10% New Zealand serum and 1% PS dual antibiotic) supplemented with anti-CD28 antibody at a concentration of 1 µg/mL and IL-2 at a working concentration of 400 U/mL. One million cells were added to each well of the 24-well plate. After cell plating, the cells were stabilized for 4 h under conditions of 37 °C and 5% CO₂ before LNPs were added.

CD7-TLNPs encapsulating eGFP-mRNA were added so that the working concentration of LNPs in the well plate reached 0.1 µg/mL. In addition, a negative control group was established by adding phosphate-buffered saline (PBS). After 24 h, the expression ratio of eGFP-mRNA in the CD7+ cell subset of PBMCs was detected by flow cytometry. .

**Table 8: In Vitro Transfection Results of CD7-TLNP**

| LNP No. | Positive Rate of CD7+ Cells (%) |
|---|---|
| PBS | 0.2 |
| CD7-TLNP | 75 |

The experimental results shows that CD7-TLNPs exhibited a significant targeting effect on CD7+ cells and had a relatively good transfection efficiency.

### Example 12:

### (I) Preparation of Nanoparticles Targeting human CD4 Cells

Refer to the preparation method in Example 3. Pre-TLNPs were mixed with anti-CD4 nanobody with a terminal cysteine residue at a molar ratio of 1 : 1 and incubated overnight at 4°C. Subsequently, after ultrafiltration washing with an 87 mg/mL sucrose solution and sterile filtration, the final nanoparticles conjugated with anti-CD4 nanobody (CD4-TLNPs) were obtained.

### (II) Evaluation of In Vitro Delivery Efficiency of Nanoparticles Targeting Human CD4 Cells

Anti-CD3 Coating: One day in advance, 200 µL of anti-Human-CD3 antibody at a concentration of 1 µg/mL was added to each well of a 24-well plate. The plate was then stored at 4°C and allowed to be coated overnight.

Preparation before Cell Plating: The liquid in the 24-well plate was aspirated out. Subsequently, the wells were washed twice with 1640 complete medium (containing 10% New Zealand serum and 1% PS (penicillin-streptomycin) dual antibiotic).

Cell Plating: Human PBMCs were diluted to a concentration of 1 million/mL using 1640 complete medium (containing 10% New Zealand serum and 1% PS dual antibiotic) supplemented with anti-CD28 antibody at a concentration of 1 µg/mL and IL-2 at a working concentration of 400 U/mL. One million cells were added to each well of the 24-well plate. After cell plating, the cells were stabilized for 4 h under conditions of 37 °C and 5% CO₂ before LNPs were added.

CD4-TLNPs encapsulating eGFP-mRNA were added so that the working concentration of LNPs in the well plate reached 0.1 µg/mL. In addition, a negative control group was established by adding phosphate-buffered saline (PBS). After 24 h, the expression ratio of eGFP-mRNA in the CD4+ cell subset of PBMCs was detected by flow cytometry. .

**Table 9: In Vitro Transfection Results of CD4-TLNPs**

| LNP No. | Positive Rate of CD4+ Cells (%) |
|---|---|
| PBS | 0.11 |
| CD7-TLNPs | 74.3 |

The experimental results shows that CD4-TLNPs exhibited a significant targeting effect on CD4+ cells and had a relatively good transfection efficiency.

### Example 13:

### (I) Preparation of Nanoparticles Targeting human CD38 Cells

Refer to the preparation method in Example 3. Pre-TLNPs were mixed with anti-CD38 nanobody at a molar ratio of 1 : 1 and incubated overnight at 4°C. Subsequently, after ultrafiltration washing with an 87 mg/mL sucrose solution and sterile filtration, the final nanoparticles conjugated with anti-CD38 nanobody (CD38-TLNPs) were obtained.

### (II) Evaluation of In Vitro Delivery Efficiency of Nanoparticles Targeting Human CD38 Cells

Anti-CD3 Coating: One day in advance, 200 µL of anti-Human-CD3 antibody at a concentration of 1 µg/mL was added to each well of a 24-well plate. The plate was then stored at 4°C and allowed to be coated overnight.

Preparation before Cell Plating: The liquid in the 24-well plate was aspirated out. Subsequently, the wells were washed twice with 1640 complete medium (containing 10% New Zealand serum and 1% PS (penicillin-streptomycin) dual antibiotic).

Cell Plating: Human PBMCs were diluted to a concentration of 1 million/mL using 1640 complete medium (containing 10% New Zealand serum and 1% PS dual antibiotic) supplemented with anti-CD28 antibody at a concentration of 1 µg/mL and IL-2 at a working concentration of 400 U/mL. One million cells were added to each well of the 24-well plate. After cell plating, the cells were stabilized for 4 h under conditions of 37 °C and 5% CO₂ before LNPs were added.

CD38-TLNPs encapsulating eGFP-mRNA were added so that the working concentration of LNPs in the well plate reached 0.1 µg/mL. In addition, a negative control group was established by adding phosphate-buffered saline (PBS). After 24 h, the expression ratio of eGFP-mRNA in the CD38+ cell subset of PBMCs was detected by flow cytometry. .

**Table 10: In Vitro Transfection Results of CD4-TLNPs**

| LNP No. | Positive Rate of CD38+ Cells (%) |
|---|---|
| PBS | 0.14 |
| CD38-TLNPs | 78.2 |

The experimental results shows that CD38-TLNPs exhibited a significant targeting effect on CD38+ cells and had a relatively good transfection efficiency.

### Example 14:

### (I) Preparation of Nanoparticles Targeting human CD68 Cells

Refer to the preparation method in Example 3. Pre-TLNPs were mixed with anti-CD68 nanobody at a molar ratio of 1 : 1 and incubated overnight at 4°C. Subsequently, after ultrafiltration washing with an 87 mg/mL sucrose solution and sterile filtration, the final nanoparticles conjugated with anti-CD68 nanobody (CD68-TLNPs) were obtained.

### (II) Evaluation of In Vitro Delivery Efficiency of Nanoparticles Targeting Human CD68 Cells

Anti-CD3 Coating: One day in advance, 200 µL of anti-Human-CD3 antibody at a concentration of 1 µg/mL was added to each well of a 24-well plate. The plate was then stored at 4°C and allowed to be coated overnight.

Preparation before Cell Plating: The liquid in the 24-well plate was aspirated out. Subsequently, the wells were washed twice with 1640 complete medium (containing 10% New Zealand serum and 1% PS (penicillin-streptomycin) dual antibiotic).

Cell Plating: Human PBMCs were diluted to a concentration of 1 million/mL using 1640 complete medium (containing 10% New Zealand serum and 1% PS dual antibiotic) supplemented with anti-CD28 antibody at a concentration of 1 µg/mL and IL-2 at a working concentration of 400 U/mL. One million cells were added to each well of the 24-well plate. After cell plating, the cells were stabilized for 4 h under conditions of 37 °C and 5% CO₂ before LNPs were added.

CD68-TLNPs encapsulating eGFP-mRNA were added so that the working concentration of LNPs in the well plate reached 0.1 µg/mL. In addition, a negative control group was established by adding phosphate-buffered saline (PBS). After 24 h, the expression ratio of eGFP-mRNA in the CD68+ cell subset of PBMCs was detected by flow cytometry. .

**Table 11: In Vitro Transfection Results of CD68-TLNPs**

| LNP No. | Positive Rate of CD68+ Cells (%) |
|---|---|
| PBS | 0.1 |
| CD68-TLNPs | 78.8 |

The experimental results shows that CD68-TLNPs exhibited a significant targeting effect on CD68+ cells and had a relatively good transfection efficiency.

### Example 15:

### (I) Preparation of Nanoparticles Targeting human CD117 Cells

Refer to the preparation method in Example 3. Pre-TLNPs were mixed with anti-CD117 nanobody at a molar ratio of 1 : 1 and incubated overnight at 4°C. Subsequently, after ultrafiltration washing with an 87 mg/mL sucrose solution and sterile filtration, the final nanoparticles conjugated with anti-CD117 nanobody (CD117-TLNPs) were obtained.

### (II) Evaluation of In Vitro Delivery Efficiency of Nanoparticles Targeting Human CD117 Cells

HLF cells were thawed and cultured. CD117-TLNPs encapsulating eGFP-mRNA were added such that the working concentration of LNPs in the well plate reached 0.1 µg/mL. In addition, a negative control group was established by adding phosphate-buffered saline (PBS). After 24 h, the expression ratio of eGFP-mRNA in the CD117+ cells of HLF cells was detected by flow cytometry.

**Table 12: In Vitro Transfection Results of CD117-TLNPs**

| LNP No. | Positive Rate of CD117+ Cells (%) |
|---|---|
| PBS | 0.1 |
| CD117-TLNPs | 75.9 |

The experimental results shows that CD117-TLNPs exhibited a significant targeting effect on CD117+ cells and had relatively good transfection efficiency.

### Example 16:

### (I) Preparation of Nanoparticles Targeting human B7H3 Cells

Refer to the preparation method in Example 3. Pre-TLNPs were mixed with anti-B7H3 nanobody at a molar ratio of 1 : 1 and incubated overnight at 4°C. Subsequently, after ultrafiltration washing with an 87 mg/mL sucrose solution and sterile filtration, the final nanoparticles conjugated with anti-B7H3 nanobody (B7H3-TLNPs) were obtained.

### (II) Evaluation of In Vitro Delivery Efficiency of Nanoparticles Targeting Human B7H3 Cells

A2780 cells were thawed and cultured. B7H3-TLNPs encapsulating eGFP-mRNA were added such that the working concentration of LNPs in the well plate reached 0.1 µg/mL. In addition, a negative control group was established by adding phosphate-buffered saline (PBS). After 24 h, the expression ratio of eGFP-mRNA in the B7H3+ cells of A2780 cells was detected by flow cytometry.

**Table 13: In Vitro Transfection Results of B7H3-TLNPs**

| LNP No. | Positive Rate of B7H3+ Cells (%) |
|---|---|
| PBS | 0.12 |
| B7H3-TLNPs | 76.5 |

The experimental results shows that B7H3-TLNPs exhibited a significant targeting effect on B7H3+ cells and had relatively good transfection efficiency.

### Example 17:

### (I) Preparation of Nanoparticles Targeting human GPC3 Cells

Refer to the preparation method in Example 3. Pre-TLNPs were mixed with anti-GPC3 nanobody with a terminal cysteine residue at a molar ratio of 1 : 1 and incubated overnight at 4°C. Subsequently, after ultrafiltration washing with an 87 mg/mL sucrose solution and sterile filtration, the final nanoparticles conjugated with anti-GPC3 nanobody (GPC3-TLNPs) were obtained.

### (II) Evaluation of In Vitro Delivery Efficiency of Nanoparticles Targeting Human GPC3 Cells

HepG2 cells were thawed and cultured. GPC3-TLNPs encapsulating eGFP-mRNA were added such that the working concentration of LNPs in the well plate reached 0.1 µg/mL. In addition, a negative control group was established by adding phosphate-buffered saline (PBS). After 24 h, the expression ratio of eGFP-mRNA in the GPC3+ cells of HepG2 cells was detected by flow cytometry.

**Table 14: In Vitro Transfection Results of GPC3-TLNPs**

| LNP No. | Positive Rate of GPC3+ Cells (%) |
|---|---|
| PBS | 0.12 |
| GPC3-TLNPs | 75.1 |

The experimental results shows that GPC3-TLNPs exhibited a significant targeting effect on GPC3+ cells and had relatively good transfection efficiency.

### Example 18:

Preparation of Nanoparticles Targeting Human CD8+ T Cells: These Nanoparticles Are Assembled from a Targeting Functional Molecule Created by Conjugating Multiple Nanobodies with DSPE-PEG2000-Lys-Mal2 Lipid

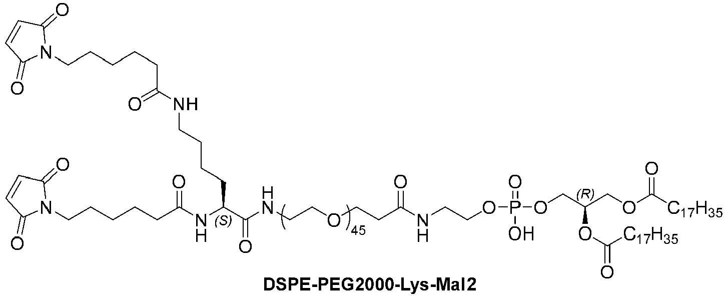

Refer to the preparation method in Example 3. The ionizable amino lipid, DSPC, CHO, DMG-PEG2000, and DSPE-PEG2000-Lys-Mal2 were dissolved in ethanol at a molar ratio of 47.5 : 10 : 41 : 1.49 : 0.01. The mRNA was dissolved in an acidic buffer solution containing 75 mM citric acid at pH 4 to ensure a concentration of 0.33 mg/mL of mRNA in the aqueous phase. The molar ratio of the ionizable amino lipid to mRNA (N/P) was set at 6.5.

The ethanol phase and the aqueous phase were mixed using a microfluidic device, with the flow rate controlled such that the volume ratio of the ethanol phase to the aqueous phase was 1 : 3. The total flow rate of the two phases was 12 mL/min, with a flow rate ratio of the ethanol phase to the aqueous phase of 1 : 3, resulting in the preparation of crude LNPs. Immediately after LNPs preparation, 10 volumes of 25 mM Tris buffer (prepared with Tris/Tris-HCl, pH = 7.4-7.5) were added to terminate the reaction.

Afther the crude LNPs were subjected to ultrafiltration purification three times at 4°C and 1200 rcf, a volume of 400 mg/mL sucrose solution equivalent to 0.278 times the volume of the LNPs was added, and LNPs with a chemical conjugation site (Pre-TLNPs) were collected. Then, Pre-TLNPs were mixed with anti-CD8 nanobody at a molar ratio of 1 : 1 and incubated overnight at 4°C. Subsequently, after ultrafiltration washing with an 87 mg/mL sucrose solution and sterile filtration, the final nanoparticles conjugated with anti-CD8 nanobody (CD8-TLNPs) were obtained.

### Example 19:

Preparation of Nanoparticles Targeting Human CD8+ T Cells: These Nanoparticles Are Assembled from a Targeting Functional Molecule Created by Conjugating Multiple Nanobodies with Mal-PEG2000-Mal Lipid

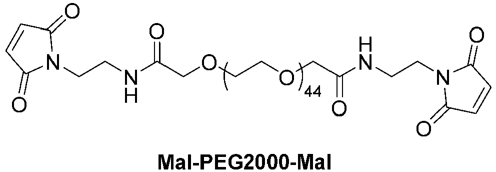

Refer to the preparation method in Example 3. The ionizable amino lipid, DSPC, CHO, DMG-PEG2000, and Mal-PEG2000-Mal were dissolved in ethanol at a molar ratio of 47.5 : 10 : 41 : 1.49 : 0.01. The mRNA was dissolved in an acidic buffer solution containing 75 mM citric acid at pH 4 to ensure a concentration of 0.33 mg/mL of mRNA in the aqueous phase. The molar ratio of the ionizable amino lipid to mRNA (N/P) was set at 6.5.

The ethanol phase and the aqueous phase were mixed using a microfluidic device, with the flow rate controlled such that the volume ratio of the ethanol phase to the aqueous phase was 1 : 3. The total flow rate of the two phases was 12 mL/min, with a flow rate ratio of the ethanol phase to the aqueous phase of 1 : 3, resulting in the preparation of crude LNPs. Immediately after LNPs preparation, 10 volumes of 25 mM Tris buffer (prepared with Tris/Tris-HCl, pH = 7.4-7.5) were added to terminate the reaction.

Afther the crude LNPs were subjected to ultrafiltration purification three times at 4°C and 1200 rcf, a volume of 400 mg/mL sucrose solution equivalent to 0.278 times the volume of the LNPs was added, and LNPs with a chemical conjugation site (Pre-TLNPs) were collected. Then, Pre-TLNPs were mixed with anti-CD8 nanobody at a molar ratio of 1 : 1 and incubated overnight at 4°C. Subsequently, after ultrafiltration washing with an 87 mg/mL sucrose solution and sterile filtration, the final nanoparticles conjugated with anti-CD8 nanobody (CD8-TLNPs) were obtained.

### Example 20:

This Example is for the structural screening of the targeting functional molecule in nanoparticles targeting human CD8+ T cells.

### (I) Preparation of Nanoparticles Targeting Human CD8 T Cells

**Table 15: Preparation Table of LNPs with Different Targeting Molecular Structures (Lkx)**

| **LNP No.** | **(E12LA6B6O3: DSPC : CHO : DMG-PEG2000: LKx) 47.5 : 10 : 41: 1.49 : 0.01** |
|---|---|
| **cLNP-1** | / |
| **TLNP-1** | Lk1 (DSPE-PEG2000-Mal) |
| **TLNP-2** | Lk2 (DSPE-Mal) |
| **TLNP-3** | Lk3 (Chol-PEG2000-Mal) |
| **TLNP-4** | Lk4 (Chol-Mal) |
| **TLNP-5** | Lk5 (DOPE-Ala-Val-Mal) |

### 1) Preparation of LNPs Comprising a Conjugation Site

The five lipid components, namely ionizable amino lipid, DSPC, CHO, DMG-PEG2000, and Lkx (where Lk1 and Lk3 contain a PEG fragment, while Lk2, Lk4, and Lk5 do not), were dissolved in ethanol at a molar ratio of 47.5 : 10 : 41 : 1.49 : 0.01. eGFP-φ-mRNA was dissolved in an acidic buffer solution containing 75 mM citric acid at pH 4 to ensure a concentration of 0.33 mg/mL of mRNA in the aqueous phase. The molar ratio of the ionizable amino lipid to mRNA (N/P) was set at 6.5.

The ethanol phase and the aqueous phase were mixed using a microfluidic device, with the flow rate controlled such that the volume ratio of the ethanol phase to the aqueous phase was 1 : 3. The total flow rate of the two phases was 12 mL/min, with a flow rate ratio of the ethanol phase to the aqueous phase of 1 : 3, resulting in the preparation of crude LNPs. Immediately after LNPs preparation, 10 volumes of 25 mM Tris buffer (prepared with Tris/Tris-HCl, pH = 7.4-7.5) were added to terminate the reaction.

The LNPs with the termination buffer were subjected to ultrafiltration purification once at 4 °C and 1200 rcf; subsequently, 25 mM Tris buffer ultrafiltrate was added, and the mixture was further ultrafiltrated and concentrated twice at 4 °C and 1200 rcf. After collecting the prepared eGFP-φ-LNPs, a volume of 400 mg/mL sucrose solution (prepared by volumetric method with 25 mM Tris buffer, pH = 7.4-7.5) equivalent to 0.278 times the volume of the LNPs was added, and sterile filtration was performed using a PES filter with a pore size of 0.22 µm in a laminar flow hood. 30 µL of the sample was taken for the detection of particle size, polydispersity index, and encapsulation efficiency, and the remaining sample was stored in a refrigerator at 4 °C. After the test for encapsulation efficiency, the sample was diluted to the desired concentration with an 87 mg/mL sucrose solution (prepared with 25 mM Tris buffer, pH = 7.4-7.5).

### 2) Preparation of TLNPs Modified with Nanobody

In addition to cLNP-1, a certain amount of the prepared eGFP-φ-Pre TLNPs comprising a conjugation site from other groups was taken. Based on the LNP formulation, the molar amount of DSPE-PEG2000-Mal in the lipid nanoparticles corresponding to each group of eGFP-φ-Pre TLNPs was calculated. Then, Pre-TLNPs were mixed with anti-human-CD8-nanobody with a terminal GS-cysteine (concentration: 2.3 mg/mL, stored in a mixed buffer composed of 20 mM Tris at pH = 8.0, 150 mM NaCl, and 5% glycerol) at a molar ratio of 1:1 of Lkx and the nanobody. The mixture was incubated overnight at 4 °C.

The TLNPs after overnight incubation was diluted with an 87 mg/mL sucrose solution (prepared with 25 mM Tris buffer, pH = 7.4-7.5) and purified using a 100 KD ultrafiltration tube. After sterile filtration using a PES filter with a pore size of 0.22 µm in a laminar flow hood, 30 µL of the resultant sample was taken for the detection of particle size, polydispersity index, and encapsulation efficiency, and the remaining sample was stored in a refrigerator at 4 °C. After the test for encapsulation efficiency, the sample was diluted to the desired concentration with an 87 mg/mL sucrose solution (prepared with 25 mM Tris buffer, pH = 7.4-7.5).

**Table 16: Quality Control Information for TLNPs Modified with Nanobody and Comprising Different Targeting Molecular Structures**

| LNP No. | Particle Size(nm) | PDI | Encapsulation Efficiency(%) |
|---|---|---|---|
| cLNP-1 | 97.64 | 0.093 | 96.97 |
| Lk1-TLNP-1 | 87.27 | 0.063 | 96.38 |
| Lk2-TLNP-2 | 88.02 | 0.098 | 95.91 |
| Lk3-TLNP-3 | 87.69 | 0.091 | 95.47 |
| Lk4-TLNP-4 | 87.49 | 0.067 | 96.09 |
| Lk5-TLNP-5 | 89.35 | 0.016 | 96.02 |

The experimental results show that the lipid nanoparticles targeting CD8+ T cells prepared with targeting molecules of different structures all exhibited good characterization data, which are within the recognized numerical range in the LNP field. This indicates that the preparation process of the targeting LNPs in the present application is stable and universally applicable.

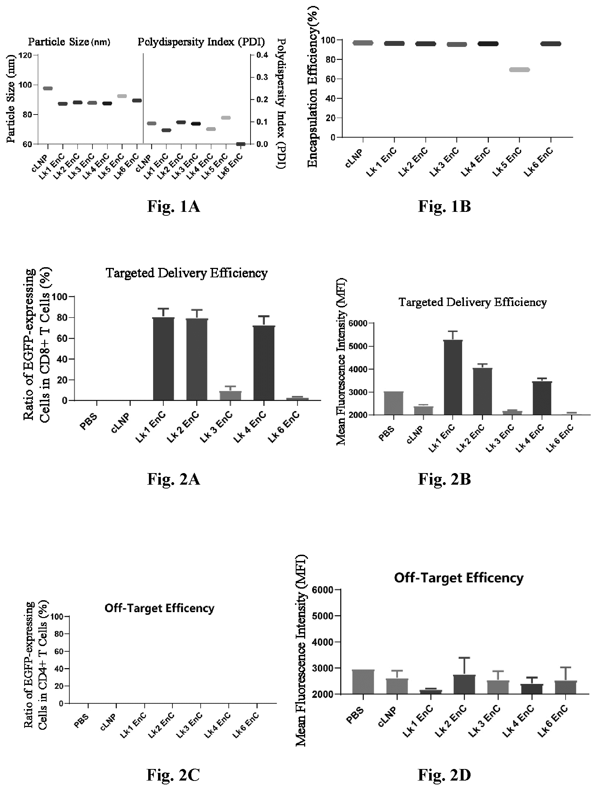

Detailed statistical charts of the specific characterization data of LNPs are shown in Figure 1A and Figure 1B.

### (II) Evaluation of the In Vitro Delivery Efficiency of Nanoparticles Targeting Human CD8+ T Cells

Anti-CD3 Coating: One day in advance, 200 µL of anti-Human-CD3 antibody at a concentration of 1 µg/mL was added to each well of a 24-well plate. The plate was then stored at 4 °C overnight for coating.

Preparation before Cell Plating: The liquid in the 24-well plate was aspirated out. Subsequently, the wells were washed twice with 1640 complete medium (containing 10% New Zealand serum and 1% PS (penicillin-streptomycin) dual antibiotic).

Cell Plating: Human PBMCs were diluted to a concentration of 1 million/mL using 1640 complete medium (containing 10% New Zealand serum and 1% PS dual antibiotic) supplemented with anti-CD28 antibody at a concentration of 1 µg/mL and IL-2 at a working concentration of 400 U/mL. One million cells were added to each well of the 24-well plate. After cell plating, the cells were stabilized for 4 h under conditions of 37 °C and 5% CO₂ before LNPs were added.

Drug Administration: Each well was added with one of the six types of LNPs prepared in Table 16, ensuring a working concentration of 0.1 µg/mL of LNPs in the well plate. In addition, a negative control group was established by adding phosphate-buffered saline (PBS).

Culture: After adding LNPs, PBMCs were transfected for 24 h at 37 °C and 5% CO₂.

Detection of eGFP-mRNA Expression: Cells from each well were collected, stained with a commercially available antibody for flow cytometry, and then the expression ratio and mean fluorescence intensity (MFI) of eGFP-mRNA in CD4+ T and CD8+ T cell subsets in PBMCs were detected by flow cytometry.

**Table 17: In Vitro Transfection Results of TLNPs Containing Different Targeting Molecular Structures**

| LNP No. | Positive Rate(%)and MFI of CD4 T cells | | 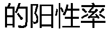 (%) and MFI of CD8 T cells | |
|---|---|---|---|---|
| PBS | 0.12 | 2977 | 0.14 | 3067 |
| cLNP-1 | 0.17 | 2630 | 0.18 | 2397 |
| Lk1-TLNP-1 | 0.24 | 2190 | 81.1 | 5304 |
| Lk2-TLNP-2 | 0.18 | 2776 | 79.8 | 4084 |
| Lk3-TLNP-3 | 0.13 | 2558 | 9.98 | 2197 |
| Lk4-TLNP-4 | 0.15 | 2423 | 73.15 | 3501 |
| Lk5-TLNP-5 | 0.15 | 2544 | 2.92 | 2069 |

The experimental results show that TLNPs prepared with Lk1, Lk2, and Lk4 exhibited significant targeting effects on CD8+ T cells and had good transfection efficiency at extremely low dosages.

Detailed statistical charts of the in vitro transfection of LNPs with different targeting molecular structures are shown in Figure 2A, Figure 2B, Figure 2C, and Figure 2D.

### (III) Evaluation of the In Vivo Delivery Efficiency of Nanoparticles Targeting Human CD8+ T Cells

Twelve 6-8-week-old NCG female mice that passed quarantine and met the weight requirements of the SPF (specific pathogen-free) level were selected and divided into 4 groups. Each NCG mouse was injected with 20 million (20M) hPBMC cells in a volume of 200 µL via the tail vein. About 30 min later, after the venous wound healed, the TLNPs prepared in Table 16 (Lk1, Lk2, and Lk4) were also injected via the tail vein. Each NCG mouse was administered a dosage of 20 µg- mRNA-LNP in a volume of 200 µL. After 24 h of expression, peripheral blood was collected, and the spleen was harvested, and they weree subjected to red blood cell lysis. Then, flow cytometry was performed to detect the expression ratio of eGFP-mRNA in various cell subsets.

**Table 18: In Vivo Experimental Grouping for TLNPs with Different Targeting Molecular Structures**

| **Experimental Group No.** | **Name of Administered LNP** |
|---|---|
| **G1** | PBS |
| **G2** | Lk1-TLNP-1 |
| **G3** | Lk2-TLNP-2 |
| **G4** | Lk4-TLNP-4 |

The experimental results show that TLNPs prepared with Lk1 exhibit the best CD8+ T cell targeting effect and the lowest off-target rate in NCG mice. Subsequent experiments will be carried out based on Lk1-TLNP.

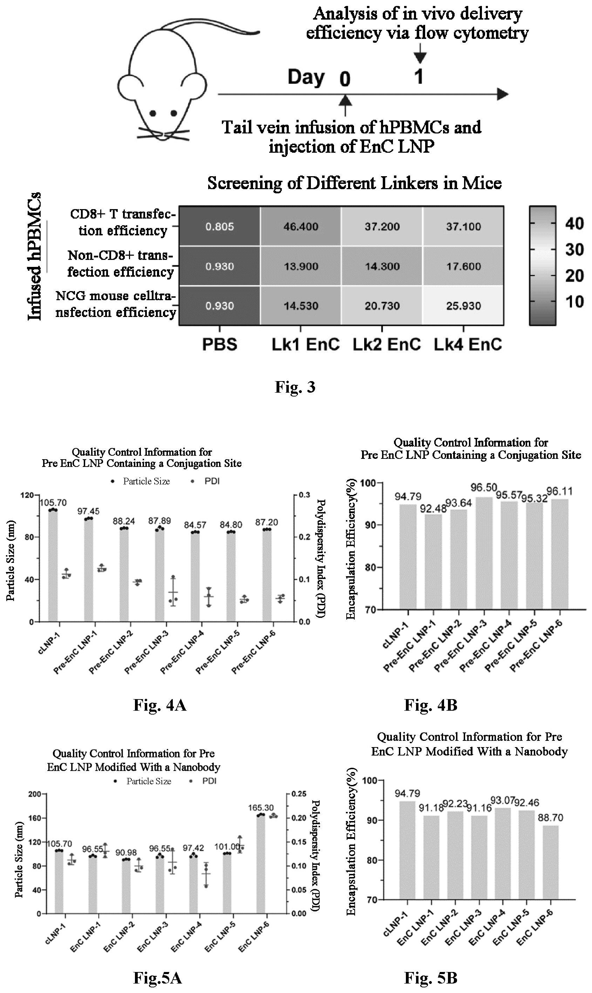

Detailed statistical charts of the in vivo transfection of TLNPs with different targeting molecular structures are shown in Figure 3.

### Example 21:

This Example is for a screening experiment for the ratio of the targeting functional molecule in nanoparticles targeting human CD8+ T cells.

### (I) Preparation of Nanoparticles Targeting Human CD8 T Cells

**Table 19: Preparation Table of Pre-LNPs with Different Ratios of Targeting Molecules**

| **LNP No.** | **Ratio of Five Components of LNPs (Ionizable amino lipid : DSPC : CHO : DMG-PEG2000: DSPE-PEG2000-Mal)** |
|---|---|
| cLNP-1 | 47.5 : 10 : 41 : 1.5 |
| Pre-TLNP-1 | 47.5 : 10 : 41 : 1.5 : 0.0001 |
| Pre-TLNP-2 | 47.5 : 10 : 41 : 1.5 : 0.01 |
| Pre-TLNP-3 | 47.5 : 10 : 41 : 1.45 : : 0.05 |
| Pre-TLNP-4 | 47.5 : 10 : 41 : 1.40 : : 0.10 |
| Pre-TLNP-5 | 47.5 : 10 : 41 : 1.25 : : 0.25 |
| Pre-TLNP-6 | 47.5 : 10 : 41 : 1.0 : 0.5 |

### 1) Preparation of LNPs Comprising a Conjugation Site

The ionizable amino lipid, DSPC, CHO, DMG-PEG2000, DSPE-PEG2000-Mal were dissolved in ethanol according to the molar ratio shown in Table 19, and eGFP-φ-mRNA was dissolved in an acidic buffer solution containing 75 mM citric acid at pH 4 to ensure a concentration of 0.33 mg/mL of mRNA in the aqueous phase. The molar ratio of the ionizable amino lipid to mRNA (N/P) was set at 6.5.

The ethanol phase and the aqueous phase were mixed using a microfluidic device, with the flow rate controlled such that the volume ratio of the ethanol phase to the aqueous phase was 1 : 3. The total flow rate of the two phases was 12 mL/min, with a flow rate ratio of the ethanol phase to the aqueous phase of 1 : 3, resulting in the preparation of crude LNPs. Immediately after LNPs preparation, 10 volumes of 25 mM Tris buffer (prepared with Tris/Tris-HCl, pH = 7.4-7.5) were added to terminate the reaction.

The LNPs with the termination buffer were subjected to ultrafiltration purification once at 4 °C and 1200 rcf; subsequently, 25 mM Tris buffer ultrafiltrate was added, and the mixture was further ultrafiltrated and concentrated twice at 4 °C and 1200 rcf. After collecting the prepared eGFP-φ-LNPs, a volume of 400 mg/mL sucrose solution (prepared by volumetric method with 25 mM Tris buffer, pH = 7.4-7.5) equivalent to 0.278 times the volume of the LNPs was added, and sterile filtration was performed using a PES filter with a pore size of 0.22 µm in a laminar flow hood. 30 µL of the sample was taken for the detection of particle size, polydispersity index, and encapsulation efficiency, and the remaining sample was stored in a refrigerator at 4 °C. After the test for encapsulation efficiency, the sample was diluted to the desired concentration with an 87 mg/mL sucrose solution (prepared with 25 mM Tris buffer, pH = 7.4-7.5).

The experimental results show that: through the quality control data analysis of the seven prepared groups of lipid nanoparticles (LNPs), when 0.0001 mol%-0.50 mol% of DSPE-PEG2000-Mal lipid was introduced into the LNP formulation, compared with the original four-component LNP, the particle size of the corresponding LNP will decrease.

**Table 20: Quality Control Information for Pre TLNP Containing a Conjugation Site**

| LNP No. | Particle Size (nm) | PDI | encapsulation efficiency (%) |
|---|---|---|---|
| cLNP-1 | 105.7 | 0.11 | 94.79 |
| Pre-TLNP-1 | 97.45 | 0.13 | 92.48 |
| Pre-TLNP-2 | 88.24 | 0.09 | 93.64 |
| Pre-TLNP-3 | 87.89 | 0.07 | 96.50 |
| Pre-TLNP-4 | 84.57 | 0.06 | 95.57 |
| Pre-TLNP-5 | 84.80 | 0.05 | 95.32 |
| Pre-TLNP-6 | 86.87 | 0.06 | 93.04 |

Statistical charts of the specific characterization data of Pre-TLNPs are shown in Figure 4A and Figure 4B.

### 2) Preparation of TLNPs Modified with Nanobody

In addition to cLNP-1, a certain amount of the prepared eGFP-φ-Pre TLNPs comprising a conjugation site from other groups was taken. Based on the LNP formulation, the molar amount of DSPE-PEG2000-Mal in the lipid nanoparticles corresponding to each group of eGFP-φ-Pre TLNPs was calculated. Then, Pre-TLNPs were mixed with anti-human-CD8-nanobody with a terminal cysteine (concentration: 2.3 mg/mL, stored in a mixed buffer composed of 20 mM Tris at pH = 8.0, 150 mM NaCl, and 5% glycerol) at a molar ratio of 1:1 of the DSPE-PEG2000-Mal lipid and the nanobody. The mixture was incubated overnight at 4 °C.

The TLNPs after overnight incubation was diluted with an 87 mg/mL sucrose solution (prepared with 25 mM Tris buffer, pH = 7.4-7.5) and purified using a 100 KD ultrafiltration tube. After sterile filtration using a PES filter with a pore size of 0.22 µm in a laminar flow hood, 30 µL of the resultant sample was taken for the detection of particle size, polydispersity index, and encapsulation efficiency, and the remaining sample was stored in a refrigerator at 4 °C. After the test for encapsulation efficiency, the sample was diluted to the desired concentration with an 87 mg/mL sucrose solution (prepared with 25 mM Tris buffer, pH = 7.4-7.5).

**Table 21: Quality Control Information for TLNPs with Different Ratios of Targeting Molecules**

| LNP No. | Particle Size (nm) | PDI | Encapsulation Efficiency (%) |
|---|---|---|---|
| cLNP-1 | 105.7 | 0.11 | 94.79 |
| TLNP-1 | 96.55 | 0.13 | 91.18 |
| TLNP-2 | 90.98 | 0.10 | 92.23 |
| TLNP-3 | 96.55 | 0.11 | 91.16 |
| TLNP-4 | 97.42 | 0.08 | 93.07 |
| TLNP-5 | 101.00 | 0.14 | 92.46 |
| TLNP-6 | 104.57 | 0.15 | 90.25 |

The experimental results show that when the nanobody was conjugated to the Pre TLNPs containing a conjugation site, the corresponding TLNPs all exhibited favorable characterization data, which fall within the generally recognized numerical ranges in the LNP field. This indicates that the preparation process of the targeting LNPs in the present patent is stable and feasible.

Statistical charts of the specific characterization data of TLNPs are shown in Figure 5A and Figure 5B.

### (II) Evaluation of the In Vitro Delivery Efficiency of Nanoparticles Targeting Human CD8+ T Cells

Anti-CD3 Coating: One day in advance, 200 µL of anti-Human-CD3 antibody at a concentration of 1 µg/mL was added to each well of a 24-well plate. The plate was then stored at 4 °C overnight for coating.

Preparation before Cell Plating: The liquid in the 24-well plate was aspirated out. Subsequently, the wells were washed twice with 1640 complete medium (containing 10% New Zealand serum and 1% PS (penicillin-streptomycin) dual antibiotic).

Cell Plating: Human PBMCs were diluted to a concentration of 1 million/mL using 1640 complete medium (containing 10% New Zealand serum and 1% PS dual antibiotic) supplemented with anti-CD28 antibody at a concentration of 1 µg/mL and IL-2 at a working concentration of 400 U/mL. One million cells were added to each well of the 24-well plate. After cell plating, the cells were stabilized for 4 h under conditions of 37 °C and 5% CO₂ before LNPs were added.

Drug Administration: Each well was added with one of the LNP groups in Table 21, ensuring a working concentration of 1.0 µg/mL of LNPs in the well plate. In addition, a negative control group was established by adding phosphate-buffered saline (PBS).

Culture: After adding LNPs, PBMCs were transfected for 24 h at 37 °C and 5% CO₂.

Detection of eGFP-mRNA Expression: Cells from each well were collected, stained with a commercially available antibody for flow cytometry, and then the expression ratios of eGFP-mRNA in all cells of PBMCs, as well as in CD4+ T and CD8+ T cell subsets of PBMCs were detected by flow cytometry.

**Table 22: In Vitro Transfection Results of TLNPs Containing Different Ratios of Targeting Molecule**

| LNP No. | Positive Rate of All Cells in PBMCs (%) | Positive Rate of CD4+ T Cells (%) | Positive Rate of CD8+ T cells (%) |
|---|---|---|---|
| PBS | 0.16 | 0.30 | 0.33 |
| cLNP-1 | 24.1 | 51.4 | 24.9 |
| TLNP-1 | 45.1 | 52.3 | 94.4 |
| TLNP-2 | 47.2 | 53.7 | 93.4 |
| TLNP-3 | 53.3 | 56.7 | 96.0 |
| TLNP-4 | 47.4 | 47.1 | 94.4 |
| TLNP-5 | 39.2 | 27.6 | 94.5 |
| TLNP-6 | 36.6 | 25.8 | 90.5 |

### (III) Evaluation of the In Vivo Delivery Efficiency of Nanoparticles Targeting Human CD8+ T Cells

Nine 6-8-week-old NCG female mice that passed quarantine and met the weight requirements of the SPF (specific pathogen-free) level were selected and divided into 3 groups. Each NCG mouse was injected with 20 million (20M) hPBMC cells in a volume of 200 µL via the tail vein. About 30 min later, after the venous wound healed, the TLNP-1 and TLNP-2 prepared in Table 20 were also injected via the tail vein. Each NCG mouse was administered a dosage of 20 µg-mRNA-LNP in a volume of 200 µL. After 24 h of expression, peripheral blood was collected, and the spleen was harvested, and they were subjected to red blood cell lysis. Then, flow cytometry was performed to detect the expression ratio of eGFP-mRNA in various cell subsets.

**Table 23: In Vivo Experimental Grouping for TLNPs with Different Ratios of Targeting Molecule**

| **Experimental Group No.** | **Name of Administered LNP** |
|---|---|
| **G1** | PBS |
| **G2** | TLNP-1 (0.0001%) |
| **G3** | TLNP-2 (0.01%) |

The experimental results show that TLNP-2 with a ratio of 0.01% of the targeting molecule exhibited better CD8+ T cell targeted delivery efficacy in NCG mice compared to the TLNP-1 group with a ratio of 0.0001%.

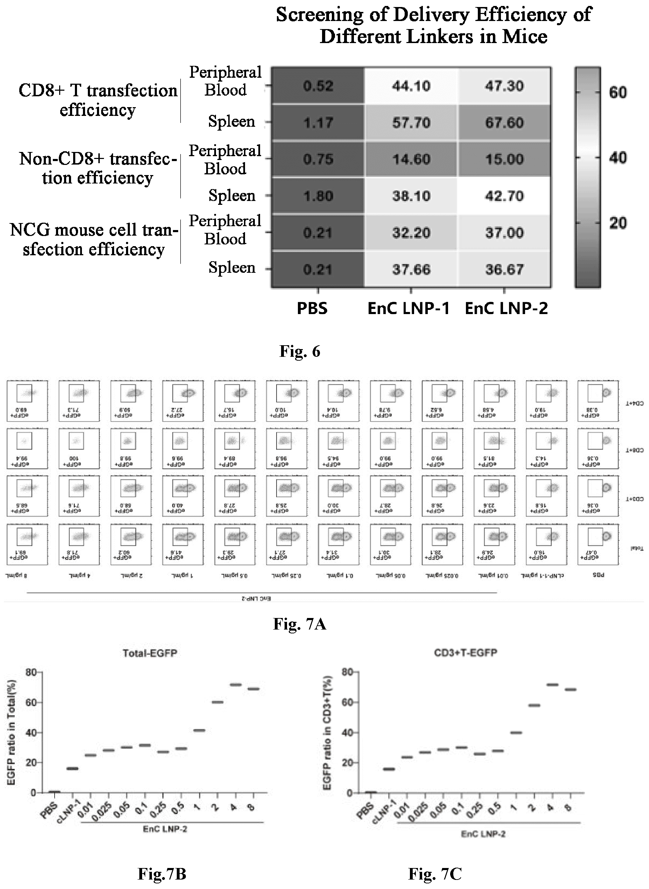

The statistical charts of the in vivo transfection status of TLNPs with different ratios of targeting molecule are shown in Figure 6.

### Example 22:

This Example is for study on the ratio (proportion) of DMG-PEG2000 lipid in nanoparticles targeting human CD8+ T cells.

### (I) Preparation of Nanoparticles Targeting Human CD8+ T Cells

Table 24: Preparation Table of CD8-TLNPs with different ratios of PEG lipids.

| **LNP No.** | **Ratios of Five Components of LNP (Ionizable amino lipid : DSPC : CHO : DMG-PEG2000 : DSPE-PEG2000-Mal )** |
|---|---|
| **TLNP-1** | 47.5 : 10 : 41 : 0.2 : 0.01 |
| **TLNP-2** | 47.5 : 10 : 41 : 0.5 : 0.01 |
| **TLNP-3** | 47.5 : 10 : 41 : 1.0 : 0.01 |
| **TLNP-4** | 47.5 : 10 : 41 : 1.5 : 0.01 |
| **TLNP-5** | 47.5 : 10 : 41 : 2.0 : 0.01 |
| **TLNP-6** | 47.5 : 10 : 41:5.0 :0.01 |

The lipid nanoparticles utilized in this Example were prepared following the method described in Example 20.

### (II) Evaluation of the In Vitro Delivery Efficiency of Nanoparticles Targeting Human CD8+ T Cells

Anti-CD3 Coating: One day in advance, 200 µL of anti-Human-CD3 antibody at a concentration of 1 µg/mL was added to each well of a 24-well plate. The plate was then stored at 4 °C overnight for coating.

Preparation before Cell Plating: The liquid in the 24-well plate was aspirated out. Subsequently, the wells were washed twice with 1640 complete medium (containing 10% New Zealand serum and 1% PS (penicillin-streptomycin) dual antibiotic).

Cell Plating: Human PBMCs were diluted to a concentration of 1 million/mL using 1640 complete medium (containing 10% New Zealand serum and 1% PS dual antibiotic) supplemented with anti-CD28 antibody at a concentration of 1 µg/mL and IL-2 at a working concentration of 400 U/mL. One million cells were added to each well of the 24-well plate. After cell plating, the cells were stabilized for 4 h under conditions of 37 °C and 5% CO₂ before LNPs were added.

Drug Administration: Each well was added with one of the LNP groups in Table 24, ensuring a working concentration of 1.0 µg/mL of LNPs in the well plate. In addition, a negative control group was established by adding phosphate-buffered saline (PBS).

Culture: After adding LNPs, PBMCs were transfected for 24 h at 37 °C and 5% CO₂.

Detection of eGFP-mRNA Expression: Cells from each well were collected, stained with a commercially available antibody for flow cytometry, and then the expression ratios of eGFP-mRNA in all cells of PBMCs, as well as in CD4+ T and CD8+ T cell subsets of PBMCs were detected by flow cytometry.

**Table 25: In Vitro Transfection Results of TLNPs Containing Different ratios of PEG Lipids**

| LNP No. | Positive Rate of All Cells in PBMCs (%) | Positive Rate of CD4+ T Cells (%) | Positive Rate of CD8+ T cells (%) |
|---|---|---|---|
| PBS | 0.16 | 0.30 | 0.33 |
| TLNP-1 | 12.6 | 15.2 | 33.6 |
| TLNP-2 | 27.2 | 36.4 | 76.3 |
| TLNP-3 | 36.4 | 46.5 | 89.2 |
| TLNP-4 | 47.2 | 53.7 | 93.4 |
| TLNP-5 | 32.8 | 27.3 | 87.3 |
| TLNP-6 | 14.2 | 15.4 | 39.7 |

The experimental results show that TLNPs containing 1.0% and 1.5% DMG-PEG2000 exhibited superior CD8+ T cell-targeted delivery efficacy compared to groups with other PEG ratios.

### Example 23:

This Example is for a study on the delivery efficiency of nanoparticles targeting human CD8+ T cells, which is composed of ionizable amino lipids with different structures.

### (I) Preparation of Nanoparticles Targeting Human CD8+ T Cells

**Table 26: Preparation Table of TLNPs composed of different ionizable amino lipids.**

| **LNP No.** | **Name of Ionizable Amino Lipid** |
|---|---|
| **TLNP-1** | E12LA6B6O3 |
| **TLNP-2** | E10LA8B6O3 |
| **TLNP-3** | SM-102 |
| **TLNP-4** | Dlin-MC3-DMA |

The lipid nanoparticles used in this Eexample were prepared according to the ratio of 47.5 : 10 : 41 : 1.5 : 0.01(ionizable amino lipid : DSPC : CHO : DMG-PEG2000 : DSPE-PEG2000-Mal) with reference to the method described in Example 20.

### (II) Evaluation of the In Vitro Delivery Efficiency of Nanoparticles Targeting Human CD8+ T Cells

Anti-CD3 Coating: One day in advance, 200 µL of anti-Human-CD3 antibody at a concentration of 1 µg/mL was added to each well of a 24-well plate. The plate was then stored at 4 °C overnight for coating.

Preparation before Cell Plating: The liquid in the 24-well plate was aspirated out. Subsequently, the wells were washed twice with 1640 complete medium (containing 10% New Zealand serum and 1% PS (penicillin-streptomycin) dual antibiotic).

Cell Plating: Human PBMCs were diluted to a concentration of 1 million/mL using 1640 complete medium (containing 10% New Zealand serum and 1% PS dual antibiotic) supplemented with anti-CD28 antibody at a concentration of 1 µg/mL and IL-2 at a working concentration of 400 U/mL. One million cells were added to each well of the 24-well plate. After cell plating, the cells were stabilized for 4 h under conditions of 37 °C and 5% CO₂ before LNPs were added.

Drug Administration: Each well was added with one of the LNP groups in Table 26, ensuring a working concentration of 1.0 µg/mL of LNPs in the well plate. In addition, a negative control group was established by adding phosphate-buffered saline (PBS).

Culture: After adding LNPs, PBMCs were transfected for 24 h at 37 °C and 5% CO₂.

Detection of eGFP-mRNA Expression: Cells from each well were collected, stained with a commercially available antibody for flow cytometry, and then the expression ratios of eGFP-mRNA in all cells of PBMCs, as well as in CD4+ T and CD8+ T cell subsets of PBMCs were detected by flow cytometry.

**Table 27: In Vitro Transfection Results of CD8-TLNPs composed of different ionizable amino lipids.**

| LNP No. | Positive Rate of All Cells in PBMCs (%) | Positive Rate of CD4+ T Cells (%) | Positive Rate of CD8+ T cells (%) |
|---|---|---|---|
| PBS | 0.2 | 0.21 | 0.13 |
| TLNP-1 | 34.9 | 17.3 | 97.3 |
| TLNP-2 | 26.1 | 10.2 | 89.7 |
| TLNP-3 | 73.2 | 80.4 | 91.5 |
| TLNP-4 | 22.7 | 8.1 | 86.0 |

The experimental results show that CD8-TLNPs composed of E12LA6B6O3, E10LA8B6O3, and the commercially available lipid SM-102 exhibited comparable or even slightly superior transfection efficiency in the CD8+ T cell subset. However, SM-102 showed a higher off-target rate in other non-CD8+ T cell subsets. Based on a comprehensive analysis, E12LA6B6O3 and E10LA8B6O3 exhibited superior targeted delivery effects for the CD8+ T cell subset.

### Example 24:

This Example is for an in vitro and in vivo dosage-dependent study of nanoparticles targeting human CD8+ T cells.

### (I) Preparation of Nanoparticles Targeting Human CD8+ T Cells

The two lipid nanoparticles used in this Example were cLNP-1 and TLNP-2 (with a targeting molecule ratio of 0.01%), which were prepared as described in Example 21.In Vitro and In Vivo dosage-Dependent Study of Nanoparticles Targeting Human CD8+ T Cells

### (II) Investigation of dosage-Dependent In Vitro Delivery Efficiency of Nanoparticles Targeting Human CD8+ T Cells

Anti-CD3 Coating: One day in advance, 200 µL of anti-Human-CD3 antibody at a concentration of 1 µg/mL was added to each well of a 24-well plate. The plate was then stored at 4 °C overnight for coating.

Preparation before Cell Plating: The liquid in the 24-well plate was aspirated out. Subsequently, the wells were washed twice with 1640 complete medium (containing 10% New Zealand serum and 1% PS (penicillin-streptomycin) dual antibiotic).

Cell Plating: Human PBMCs were diluted to a concentration of 1 million/mL using 1640 complete medium (containing 10% New Zealand serum and 1% PS dual antibiotic) supplemented with anti-CD28 antibody at a concentration of 1 µg/mL and IL-2 at a working concentration of 400 U/mL. One million cells were added to each well of the 24-well plate. After cell plating, the cells were stabilized for 4 h under conditions of 37 °C and 5% CO₂ before LNPs were added.

Drug Administration: Each well was added with the corresponding targeting molecule-modified TLNP-2 to achieve the LNP working concentrations specified in the table below. PBS was added as the negative control, while LNP-1 was added as the positive control.

**Table 28: Information on LNP Addition to Plate**

| Experimental Group | Well No. | LNP working concentration (µg/ml) |
|---|---|---|
| Negative Control | 1 | 0 |
| 0.01 | 2 | 0.01 |
| 0.025 | 3 | 0.025 |
| 0.05 | 4 | 0.05 |
| 0.1 | 5 | 0.1 |
| 0.25 | 6 | 0.25 |
| 0.5 | 7 | 0.5 |
| Conventional dosage | 8 | 1 |
| 2 | 9 | 2 |
| 4 | 10 | 4 |
| 8 | 11 | 8 |
| cLNP-1 Conventional dosage | 12 | 1 |

Culture: After adding LNPs, PBMCs were transfected for 24 h at 37 °C and 5% CO₂.

Detection of eGFP-mRNA Expression: Cells from each well were collected, stained with a commercially available antibody for flow cytometry, and then the expression ratios of eGFP-mRNA in all cells of PBMCs, as well as in CD4+ T and CD8+ T cell subsets of PBMCs were detected by flow cytometry.

**Table 29: Summary of In Vitro Targeting Efficiency Data for Nanobody-Modified LNPs in T Cells**

| LNP Working Concentration (µg/mL) | EGFP Positive Rate in All Cells (%) | EGFP Positive Rate in CD3 T Cells (%) | EGFP Positive Rate in CD4 T Cells (%) | EGFP Positive Rate in CD8 T Cells (%) |
|---|---|---|---|---|
| Negative Control | 0.47 | 0.36 | 0.38 | 0.36 |
| 0.01 | 24.9 | 23.6 | 4.58 | 81.5 |
| 0.025 | 28.1 | 26.8 | 6.52 | 99.0 |
| 0.05 | 30.1 | 28.7 | 9.78 | 99.0 |
| 0.1 | 31.4 | 30.0 | 10.4 | 94.5 |
| 0.25 | 27.1 | 25.8 | 10.0 | 96.8 |
| 0.5 | 29.3 | 27.8 | 15.7 | 89.4 |
| Conventional | 41.6 | 40.0 | 27.2 | 99.6 |
| dosage | | | | |
| 2 | 60.2 | 58.0 | 50.9 | 99.8 |
| 4 | 71.8 | 71.6 | 71.3 | 100 |
| 8 | 69.1 | 68.5 | 69.0 | 99.4 |
| cLNP-1 Conventional dosage | 16.0 | 15.8 | 19.0 | 14.3 |

The experimental results show that in the in vitro transfection experiment of hPBMCs, when the working concentration of TLNP-2 is as low as 0.025 µg/mL, a effect of targeting a high proportion of CD8 T cells was still maintained, indicating that the TLNPs developed in the present application have the ability to achieve high cell targeting at a low dosage.

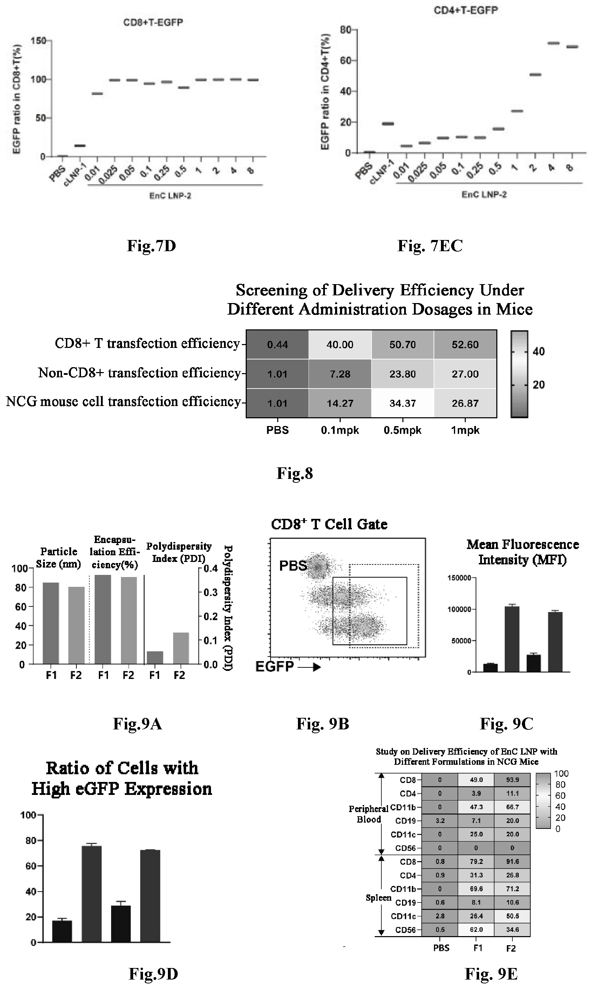

The flow cytometry analysis diagrams of the EGFP-mRNA expression efficiency of cLNP-1 and TLNP-2 in hPBMCs are shown in Figures 7A, 7B, 7C, 7D and 7E.

### (III) Evaluation of dosage-Dependent In Vivo Delivery Efficiency of Nanoparticles Targeting Human CD8+ T Cells

Twelve 6-8-week-old NCG female mice that passed quarantine and met the weight requirements of the SPF (specific pathogen-free) level were selected and divided into 4 groups. Each NCG mouse was injected with 20 million (20M) hPBMC cells in a volume of 200 µL via the tail vein. About 30 min later, after the venous wound healed, different does of TLNP-2 were also injected via the tail vein in a volume of 200 µL. After 24 h of expression, peripheral blood was collected, and the spleen was harvested, and they were subjected to red blood cell lysis. Then, flow cytometry was performed to detect the expression ratio of eGFP-mRNA in various cell subsets.

**Table 30: In Vivo Experimental Grouping for TLNP-2**

| **Experimental Group No.** | **Administration dosage** |
|---|---|
| **G1** | PBS |
| **G2** | 0.1 mpk (2 µg) |
| **G3** | 0.5 mpk (10 µg) |
| **G4** | 1 mpk (20 µg) |

The experimental results show that TLNP-2 with a targeting molecule ratio of 0.01% had a very good CD8+ T cell targeted delivery effect at a administration dosage of 0.5 mpk in NCG mice.

The statistical graphs of in vivo transfection of TLNP-2 at different administration dosages are shown in Figure 8.

### Example 25:

This Example is for a study on the formulations of nanoparticles targeting human CD8+ T cells

### (I) Prepare Nanoparticles Targeting Human CD8 T Cells

**Table 31: Preparation Table of TLNPs with Different Formulations**

| **LNP No.** | **Ratio of Five Components of LNP (Ionizable amino lipid : DSPC : CHO : DMG-PEG2000: DSPE-PEG2000-Mal )** |
|---|---|
| **TLNP-2-F1** | 47.5 : 10 : 41 : 1.5 : 0.01 |
| **TLNP-2-F2** | 47.5 : 15 : 36 : 1.0 : 0.01 |

The two lipid nanoparticles, TLNP-2-F1 and TLNP-2-F2, used in this Example were both prepared according to the method described in Example 20.

### (II) Investigation on the In Vivo Delivery Efficiency of Nanoparticles Targeting Human CD8+ T Cells with Different Formulations

Nine 6-8-week-old NCG female mice that passed quarantine and met the weight requirements of the SPF (specific pathogen-free) level were selected and divided into 3 groups. Each NCG mouse was injected with 20 million (20M) hPBMC cells in a volume of 200 µL via the tail vein. About 30 min later, after the venous wound healed, the prepared TLNP-2-F1 and TLNP-2-F2 were also injected via the tail vein. Each NCG mouse was administered a dosage of 10 µg-mRNA-LNP in a volume of 200 µL. After 24 h of expression, peripheral blood was collected, and the spleen was harvested, and they were subjected to red blood cell lysis. Then, flow cytometry was performed to detect the expression ratio of eGFP-mRNA in various cell subsets.

**Table 32: In Vivo Experimental Grouping for TLNPs with Different Formulations**

| **Experimental Group No.** | **Name of Administered LNP** |
|---|---|
| **G1** | PBS |
| **G2** | TLNP-2-F1 |
| **G3** | TLNP-2-F2 |

The experimental results show that at an administration dosage of 0.5 mpk, TLNP-2-F2 prepared with formulation F2 and with a targeting molecule ratio of 0.01% exhibited better CD8+ T cell targeted delivery effect in NCG mice than group F1

The statistical graphs of in vivo transfection of TLNP-2 with different formulations are shown in Figures 9A, 9B, 9C, 9D and 9E.

### Example 26:

This Example is for in vitro freeze-thaw stability evaluation of nanoparticles targeting human CD8+ T cells.

### (I) Preparation of Nanoparticles Targeting Human CD8+T Cells

The two lipid nanoparticles used in this Example were cLNP-1 and TLNP-2 prepared in Example 21, respectively.

### (II) Investigation on the Freeze-Thaw Stability of Nanoparticles Targeting Human CD8+ T Cells

The prepared LNP sample was stored at -80°C for over 24 hours and then rapidly thawed at 4°C; this sample represented 1 freeze-thaw cycle condition.

Subsequently, the thawed sample was refrozen at -80°C for over 24 hours and rapidly thawed again at 4°C; this sample represented 2 freeze-thaw cycles condition.

The thawed sample was then subjected to a third cycle of freezing at -80°C for over 24 hours followed by rapid thawing at 4°C; this sample represented 3 freeze-thaw cycles condition.

After each thawing step, the LNP samples were analyzed for particle size, polydispersity index (PDI), and encapsulation efficiency to assess their freeze-thaw stability.

**Table 33: Characterization Information of LNPs After Freeze-thaw**

| Group | Partcile Size (nm) | | PDI | | Encapsulation Efficiency (%) | |
|---|---|---|---|---|---|---|
| LNP Name | cLNP1 | TLNP-2 | cLNP1 | TLNP-2 | cLNP1 | TLNP-2 |
| Unfrozen and unthawed | 105.7 | 90.98 | 0.11 | 0.10 | 94.79 | 92.23 |
| 1 freeze-thaw cycle | 106.5 | 91.16 | 0.11 | 0.12 | 94.66 | 91.30 |
| 2 freeze-thaw cycle | 110.2 | 94.85 | 0.13 | 0.14 | 93.78 | 90.07 |
| 3 freeze-thaw cycle | 114.6 | 98.20 | 0.17 | 0.15 | 91.43 | 89.86 |

### Example 27:

This Example is for Evaluation of long-term stability of nanoparticles targeting human CD8+ T cells.

### (I) Preparation of Nanoparticles Targeting Human CD8+ T Cells

**Table 34: Formulation Table for Preparation of LNPs with Different ratios of Targeting Molecules**

| **Group** | **Ratio of Five Components of LNP (Ionizable Amino Lipid : DSPC : CHO : DMG-PEG2000: DSPE-PEG2000-Mal )** |
|---|---|
| **cLNP-1** | 47.5 : 10 : 41 : 1.5 : 0.0 |
| **TLNP-2** | 47.5 : 10 : 41 : 1.5 : 0.01 |
| **TLNP-3** | 47.5 : 10 : 41 : 1.5 : 0.05 |

In this Example, the preparation methods for LNPs containing a conjugation site and nanobody-modified LNPs refer to the processes described in Example 21.

### (II) Investigation on the Stability of Nanoparticles Targeting Human CD8+ T Cells

1. Day 0 After Nanoparticle Preparation:
1) Characterization Testing of Freshly Prepared Nanobody-Modified LNPs

**Table 35: Quality Control Information for Nanobody-Modified LNPs (Day 0)**

| LNP No. | Particle Size (nm) | PDI | Encapsulation Efficiency (%) |
|---|---|---|---|
| cLNP-1 | 105.7 | 0.11 | 94.79 |
| TLNP-2 | 90.98 | 0.10 | 92.23 |
| TLNP-3 | 96.55 | 0.11 | 91.16 |

2) In Vitro Delivery Efficiency Testing of Freshly Prepared Nanobody-Modified LNPs
Anti-CD3 Coating: One day in advance, 200 µL of anti-Human-CD3 antibody at a concentration of 1 µg/mL was added to each well of a 24-well plate. The plate was then stored at 4°C and allowed to be coated overnight.
Preparation before Cell Plating: The liquid in the 24-well plate was aspirated out. Subsequently, the wells were washed twice with 1640 complete medium (containing 10% New Zealand serum and 1% PS (penicillin-streptomycin) dual antibiotic).
Cell Plating: PBMCs were diluted to a concentration of 1 million/mL using 1640 complete medium (containing 10% New Zealand serum and 1% PS dual antibiotic). One million cells were added to each well of the 24-well plate. After cell plating, the cells were stabilized for 4 h under conditions of 37 °C and 5% CO₂ before LNPs were added.
Drug Administration: Each well was added with the corresponding targeting molecule-modified LNPs to achieve a working concentration of 1 µg/mL of LNPs in the plate. Additionally, a negative control group was set up with PBS.
Culture: After adding LNPs, PBMCs were transfected for 24 h at 37 °C and 5% CO₂.
Detection of eGFP-mRNA Expression: Cells from each well were collected, stained with a commercially available antibody for flow cytometry, and then the expression ratios of eGFP-mRNA in all cells of PBMCs, as well as in CD4+ T and CD8+ T cell subsets of PBMCs were detected by flow cytometry.

**Table 36: Summary of In Vitro Targeting Efficiency Data for Nanobody-Modified LNPs on T Cells (Day 0)**

| LNP No | Positive Rate in All PBMC Cells (%) | Positive Rate in CD4 T Cells (%) | Positive Rate in CD8 T Cells (%) |
|---|---|---|---|
| PBS Group | 0.11 | 0.36 | 0.28 |
| LNP-1 | 31.33 | 36.82 | 32.37 |
| LNP-2-Nb | 58.63 | 47.99 | 96.78 |
| LNP-3-Nb | 69.29 | 53.71 | 99.10 |

2. Day 3 After Nanoparticle Preparation:
1) Characterization Testing of Prepared Nanobody-Modified LNPs (Day 3)

**Table 37: Quality Control Information for Nanobody-Modified LNPs (Day 3)**

| LNP No. | Particle Size (nm) | PDI | Encapsulation Efficiency (%) |
|---|---|---|---|
| cLNP-1 | 106.87 | 0.10 | 93.10 |
| TLNP-2 | 91.73 | 0.10 | 92.18 |
| TLNP-3 | 96.57 | 0.09 | 90.94 |

3) In Vitro Delivery Efficiency Testing of Prepared Nanobody-Modified LNPs (Day 3) The process was the same as above.

**Table 38: Summary of In Vitro Targeting Effects of Nanobody-Modified LNPs on T Cells (Day 3)**

| LNP No | Positive Rate in All PBMC Cells (%) | Positive Rate in CD4 T Cells (%) | Positive Rate in CD8 T Cells (%) |
|---|---|---|---|
| PBS Group | 0.16 | 0.33 | 0.25 |
| cLNP-1 | 30.11 | 35.32 | 32.09 |
| TLNP-2 | 54.53 | 47.23 | 95.87 |
| TLNP-3 | 63.49 | 51.98 | 99.00 |

3. Day 7 After Nanoparticle Preparation:
1) Characterization Testing of Prepared Nanobody-Modified LNPs (Day 7)

**Table 39: Quality Control Information for Nanobody-Modified LNPs (Day 7)**

| LNP No. | Particle Size (nm) | PDI | Encapsulation Efficiency (%) |
|---|---|---|---|
| cLNP-1 | 108.28 | 0.09 | 91.38 |
| TLNP-2 | 94.50 | 0.09 | 91.45 |
| TLNP-3 | 98.24 | 0.11 | 89.84 |

3) In Vitro Delivery Efficiency Testing of Prepared Nanobody-Modified LNPs (Day 7) The process was the same as above.

**Table 40: Summary of In Vitro Targeting Effects of Nanobody-Modified LNPs on T Cells (Day 7)**

| LNP No | Positive Rate in All PBMC Cells (%) | Positive Rate in CD4 T Cells (%) | Positive Rate in CD8 T Cells (%) |
|---|---|---|---|
| PBS Group | 0.18 | 0.35 | 0.29 |
| cLNP-1 | 28.61 | 31.56 | 29.09 |
| TLNP-2 | 51.80 | 46.84 | 94.36 |
| TLNP-3 | 60.32 | 49.81 | 97.69 |

4. Day 14 After Nanoparticle Preparation:
1) Characterization Testing of Prepared Nanobody-Modified LNPs (Day 14)

**Table 41: Quality Control Information for Nanobody-Modified LNPs (Day 14)**

| LNP No. | Particle Size (nm) | PDI | Encapsulation Efficiency (%) |
|---|---|---|---|
| LNP-1 | 112.70 | 0.11 | 90.47 |
| TLNP-2 | 98.73 | 0.10 | 90.12 |
| TLNP-3 | 101.97 | 0.11 | 87.04 |

3) In Vitro Delivery Efficiency Testing of Prepared Nanobody-Modified LNPs (Day 14) The process was the same as above.

**Table 42: Summary of In Vitro Targeting Effects of Nanobody-Modified LNPs on T Cells (Day 14)**

| LNP No | Positive Rate in All PBMC Cells (%) | Positive Rate in CD4 T Cells (%) | Positive Rate in CD8 T Cells (%) |
|---|---|---|---|
| PBS Group | 0.21 | 0.48 | 0.36 |
| cLNP-1 | 25.75 | 27.77 | 25.52 |
| TLNP-2 | 42.36 | 46.86 | 91.36 |
| TLNP-3 | 50.28 | 48.26 | 93.59 |

### 5. 1 month After Nanoparticle Preparation:

1) Characterization Testing of Prepared Nanobody-Modified LNPs (Month 1)

**Table 43: Quality Control Information for Nanobody-Modified LNPs (Month 1)**

| LNP No. | Particle Size (nm) | PDI | Encapsulation Efficiency (%) |
|---|---|---|---|
| cLNP-1 | 115.13 | 0.10 | 90.42 |
| TLNP-2 | 104.30 | 0.10 | 88.89 |
| TLNP-3 | 105.77 | 0.14 | 83.76 |

2) In Vitro Delivery Efficiency Testing of Prepared Nanobody-Modified LNPs (Month 1)

The process was the same as above.

**Table 44: Summary of In Vitro Targeting Effects of Nanobody-Modified LNPs on T Cells (Month 1)**

| LNP No | Positive Rate in All PBMC Cells (%) | Positive Rate in CD4 T Cells (%) | Positive Rate in CD8 T Cells (%) |
|---|---|---|---|
| PBS 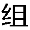 | 0.25 | 0.39 | 0.31 |
| cLNP-1 | 25.88 | 26.48 | 26.33 |
| TLNP-2 | 36.23 | 44.85 | 85.39 |
| TLNP-3 | 45.74 | 46.91 | 88.92 |

The experimental results show that although the trends and magnitudes of changes in the physicochemical properties of nanobody-modified LNPs after being stored at 4°C for one month were similar to those of the traditional four-component cLNPs, they could still maintain good targeting efficacy towards CD8 T cells, indicating that the TLNPs developed in the present application exhibit good stability and can maintain high cell-targeting ability over a long period.

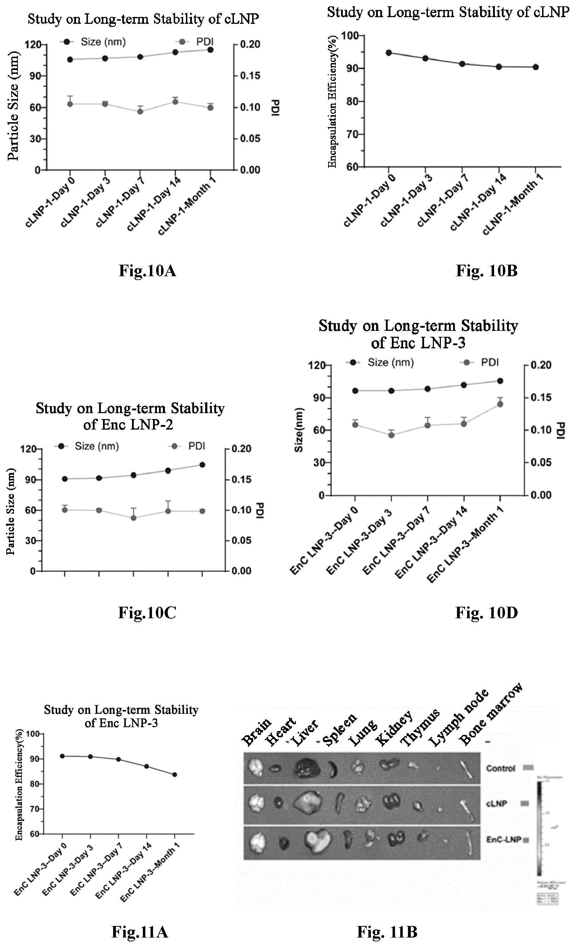

The statistical graphs of the long-term stability study data for traditional LNPs and targeting TLNPs are shown in Figures 10A, 10B, 10C, 10D, 10E, and 10F.

### Example 28:

This Example is for the functional evaluation of nanoparticles targeting human CD8+ T cells after drug delivery.

### (I) Preparation of Nanoparticles Targeting Human CD8+ T Cells

The two lipid nanoparticles used in this Example were cLNP-1 and TLNP-2, which were prepared in Example 21.

### (II) Functional Evaluation of Nanoparticles Targeting Human CD8+ T Cells after Drug Delivery

Anti-CD3 Coating: One day in advance, 200 µL of anti-Human-CD3 antibody at a concentration of 1 µg/mL was added to each well of a 24-well plate. The plate was then stored at 4°C and allowed to be coated overnight.

Preparation before Cell Plating: The liquid in the 24-well plate was aspirated out. Subsequently, the wells were washed twice with 1640 complete medium (containing 10% New Zealand serum and 1% PS (penicillin-streptomycin) dual antibiotic).

Cell Plating: PBMCs were diluted to a concentration of 1 million/mL using 1640 complete medium (containing 10% New Zealand serum and 1% PS dual antibiotic). One million cells were added to each well of the 24-well plate. After cell plating, the cells were stabilized for 4 h before LNPs were added.

Drug Administration: Each well was added with the corresponding targeting molecule-modified LNPs to achieve a working concentration of 1 µg/mL of LNPs in the plate. Additionally, a negative control group was set up with PBS.

Culture: After adding LNPs, PBMCs were transfected for 24 h at 37 °C and 5% CO₂.

Cytotoxicity Assay: Cells from each well plate were collected and counted, then diluted to a concentration of 1 million/mL. The CD19+ B-lymphoma cell line was prepared, counted, and also diluted to a concentration of 1 million/mL,
human PBMCs were thawed to serve as a control for cells transfected with CD19 CAR-LNPs. Different effector-to-target (ET) ratios were established. The corresponding systems were added according to the following table, with each numbered group performed in triplicate.

The samples were incubated at 37°C for 4-8 hours.

The dead cell dye 7-AAD, with a storage concentration of 2 mg/mL, was diluted with water to a concentration of 0.1 µg/10 µL.

2.5 µL of 7-AAD was added to a flow cytometry tube, and the samples were immediately analyzed by flow cytometry. Here, 7-AAD was detected in the PerCP-Cy5.5 channel, and Violet was detected in the BV421 channel.

The cytotoxicity rate was calculated as CFSE+7-AAD+/(Total CFSE+).

**Table 45: Summary of Data from the Functional Evaluation of the Specific Cytotoxic Effects After CAR Delivery by Nanoparticles Targeting Human CD8+ T Cells (Mean of Triplicate Wells)**

| cLNP-1 | Cytotoxicity Rate (%) | TLNP-2 | Cytotoxicity Rate (%) |
|---|---|---|---|
| 1 | 17.0 % | 1 | 31.6 % |
| 2 | 15.2 % | 2 | 34.4 % |
| 3 | 18.8 % | 3 | 26.5 % |

### Example 29:

This example is the study of the in vivo distribution of nanoparticles targeting human CD8+ T cells.

### (I) Preparation of Nanoparticles Targeting Human CD8 T Cells

The two lipid nanoparticles used in this Example were cLNP-1 and TLNP-2, which encapsulated either Luciferase mRNA or 25% Cy5 dye-labeled Luciferase mRNA.and were prepared using the preparation method described in Example 21.

### (II) Cy5-mRNA Biodistribution of Nanoparticles Targeting Human CD8 T Cells in Balb/c Mice

Nine 6-8-week-old Balb/c female mice that passed quarantine and met the weight requirements of the SPF (specific pathogen-free) level were selected (3 mice per group). Each Balb/c mouse in Group G1 was injected with 200 µL of PBS via the tail vein. Each Balb/c mouse in Groups G2 and G3 was injected with 5 µg of 25%-Cy5-Luc-φ-mRNA LNP via the tail vein, with an injection volume of 200 µL.

Two hours after administration, the mice were anesthetized with isoflurane and then dissected. The brain, heart, liver, spleen, lung, kidney, thymus, lymph node, and bone marrow were removed for ex vivo organ imaging. The imaging instrument used was the Living Imaging instrument from PerkinElmer (Shenzhen Bay Laboratory), and the default Cy5 imaging mode of the system was adopted.

**Table 46: Experimental Grouping for Studying the In Vivo Distribution of LNPs Using Cy5 Fluorescence Labeling**

| **Experimental Group No.** | **Name of LNP Administered** |
|---|---|
| **G1** | PBS |
| **G2** | cLNP (25%Cy5-Luc, 100% content) |
| **G3** | TLNP-2(25%Cy5-Luc, 100% content) |

The experimental results show that the biodistribution of traditional LNPs and cell-targeting TLNPs in ordinary Balb/c mice was basically similar. The highest accumulation of Cy5-mRNA was observed in the liver, followed by the spleen, kidney, and lung.

### (III) Luc-mRNA Biodistribution of Nanoparticles Targeting Human CD8 T Cells in Balb/c Mice

Nine 6-8-week-old Balb/c female mice that passed quarantine and met the weight requirements of the SPF (specific pathogen-free) level were selected (3 mice per group). Each Balb/c mouse in Group G1 was injected with 200 µL of PBS via the tail vein. Each Balb/c mouse in Groups G2 and G3 was injected with 5 µg of Luc-φ-mRNA LNP via the tail vein, with an injection volume of 200 µL.

6 hours after administration, the mice were anesthetized with isoflurane, and then injected intraperitoneally with 250 µL of luciferase substrate. The mice were dissected 10 minutes later. The brain, heart, liver, spleen, lung, kidney, thymus, lymph node, and bone marrow were removed for ex vivo organ imaging. The imaging instrument used was the Living Imaging instrument from PerkinElmer (Shenzhen Bay Laboratory), with the parameters set to F8 mode and an exposure time of 2 seconds. After imaging, mRNA was extracted from the harvested organs for qPCR experiments to quantify the enrichment level of mRNA in each organ.

**Table 47: Experimental Grouping for Studying the In Vivo Distribution of LNPs Using Luc Imaging**

| **Experimental Group No.** | **Name of LNP Administered** |
|---|---|
| **G1** | PBS |
| **G2** | cLNP (Luc, 100% content) |
| **G3** | TLNP-2(Luc, 100% content) |

The experimental results show that the biodistribution patterns of traditional LNPs and cell-targeting TLNPs in conventional Balb/c mice are generally similar. The highest expression levels of Luc protein and enrichment of Luc-mRNA were observed in the spleen, with the TLNP-2 group showing slightly higher levels compared to the cLNP group.

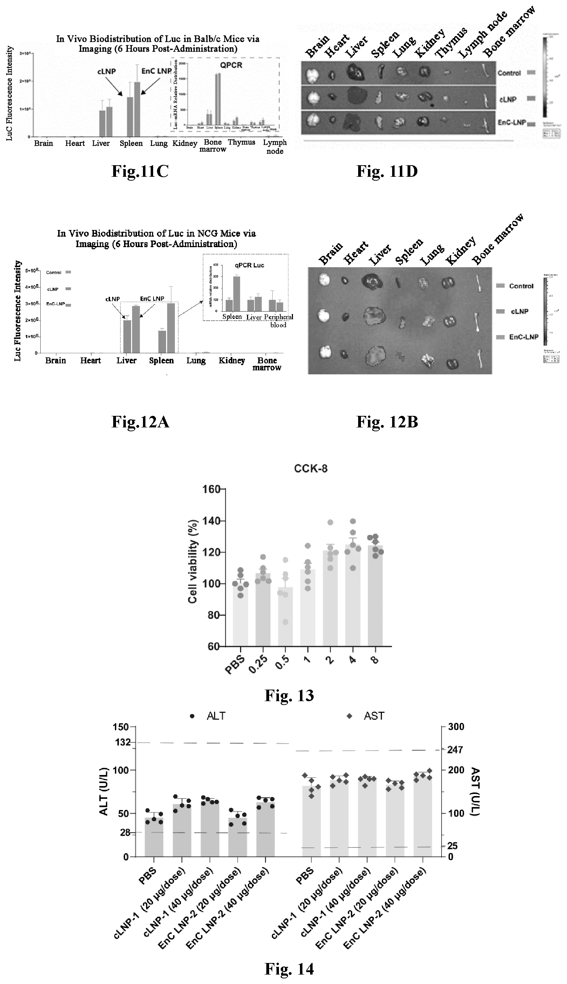

The statistical graphs of the experimental results of the in vivo biodistribution in Balb/c mice using Cy5 fluorescence, Luc imaging, and qPCR methods are shown in Figures 11A, 11B, 11C, and 11D.

### (I) Luc-mRNA Biodistribution of Nanoparticles Targeting Human CD8 T Cells in NCG Mice

Nine 6-8-week-old NCG female mice that passed quarantine and met the weight requirements of the SPF (specific pathogen-free) level were selected and divided into 3 groups. Each NCG mouse was injected with 20 million (20 M) hPBMC cells in a volume of 200 µL via the tail vein. About 30 min later, after the venous wound healed, the prepared cLNP and TLNP-2 were also injected via the tail vein. Each NCG mouse was administered a dosage of 5 µg-Luc-LNP in a injection volume of 200 µL
6 hours after administration, the mice were anesthetized with isoflurane, and then injected intraperitoneally with 250 µL of luciferase substrate. The mice were dissected 10 minutes later. The brain, heart, liver, spleen, lung, kidney and bone marrow were removed for ex vivo organ imaging. The imaging instrument used was the Living Imaging instrument from PerkinElmer (Shenzhen Bay Laboratory), with the parameters set to F8 mode and an exposure time of 2 seconds. After imaging, mRNA was extracted from the harvested spleen, liver and peripheral blood for qPCR experiments to quantify the enrichment level of mRNA in each organ.

**Table 48: Experimental Grouping for Studying the In Vivo Distribution of LNPs Using Luc Imaging**

| **Experimental Group No.** | **Name of LNP Administered** |
|---|---|
| **G1** | PBS |
| **G2** | cLNP (Luc, 100%content) |
| **G3** | TLNP-2(Luc, 100% content) |

The experimental results show that in the NCG mouse model, compared to traditional LNPs, the CD8+ T cell-targeting TLNP-2 demonstrated more selective delivery of Luc-mRNA to organs rich in hPBMCs, exhibiting a higher spleen-to-liver fluorescence intensity ratio.

The statistical graphs of the experimental results of the in vivo biodistribution in NGC mice using Luc imaging, and qPCR methods are shown in Figures 12A and 12B.

### Example 30:

This Example is for evaluation of in vitro dosage toxicity of nanoparticles targeting human CD8+ T cells

### (I) Preparation of Nanoparticles Targeting Human CD8+ T Cells

The two lipid nanoparticles used in this Example were respectively cLNP-1 and TLNP-2 prepared in Example 21.

### (II) Investigation on dosage Toxicity of Delivery by Nanoparticles Targeting Human CD8+ T Cells

Anti-CD3 Coating: One day in advance, 200 µL of anti-Human-CD3 antibody at a concentration of 1 µg/mL was added to each well of a 24-well plate. The plate was then stored at 4°C and allowed to be coated overnight.

Preparation before Cell Plating: The liquid in the 24-well plate was aspirated out. Subsequently, the wells were washed twice with 1640 complete medium (containing 10% New Zealand serum and 1% PS (penicillin-streptomycin) dual antibiotic). Cells were seeded at a density of 0.05 million cells per well in a 96-well plate, with six replicates per condition. After cell plating, the cells were stabilized for 4 h before LNPs were added.

Drug Administration: Each well was added with the corresponding targeting molecule-modified LNPs to achieve a LNP working concentration shown in the table below. A negative control group was set up with PBS.

**Table 49: Information on LNP Addition to Plate**

| Group | Well No. | LNP working concentration (µg/ml) |
|---|---|---|
| Negative Control | 1 | 0 |
| 0.25 | 2 | 0.25 |
| 0.5 | 3 | 0.5 |
| Conventional dosage | 4 | 1 |
| 2 | 5 | 2 |
| 4 | 6 | 4 |
| 8 | 7 | 8 |

Culture: After adding LNPs, PBMCs were transfected for 24 h at 37 °C and 5% CO₂.

Detection of Cell Cytotoxicity: Each well of the 96-well plate was added with 20 µL of CCK-8 reagent, followed by incubation in a 37°C incubator under light-protected conditions. Optical density (OD) values were measured, and cell viability was calculated using the formula: Cell viability = [(As-Ab) / (Ac-Ab)] × 100%,
wherein, As is absorbance of the experimental wells (containing cells, culture medium, CCK-8 solution, and drug solution);
Ac is absorbance of control wells (containing cells, culture medium, and CCK-8 solution, without drugs);
Ab is absorbance of blank wells (containing culture medium and CCK-8 solution, without cells or drugs).

The experimental data were analyzed to assess the cytotoxicity of LNPs.

The experimental results show that TLNP-2 at all tested working concentrations demonstrated no detectable cytotoxicity, indicating that the nanobody-modified TLNP is safe. Cells exhibited exceptionally high dosage tolerance to said LNP.

Data on the dosage-safety of TLNP-2 under different wording concentrations in hPBMCs were shown in Figure 13.

### Example 31

This Example is for evaluation of the in vivo safety of nanoparticles targeting human CD8+T cells in mice

### (I) Preparation of Nanoparticles Targeting Human CD8+T Cells

The two lipid nanoparticles used in this Example were cLNP-1 and TLNP-2, which were prepared in Example 21, respectively.

### (II) Investigation on the In Vivo Safety of Nanoparticles Targeting Human CD8+T Cells

The prepared LNPs were injected into mice via the tail vein at a dosage of 80 µg per mouse (acute toxicity), or 20 µg per mouse administered weekly for 6 consecutive dosages (long-term toxicity).

After 24 hours, blood samples were collected from the mice in the acute toxicity group for serum separation. Liver and kidney functions and cytokine levels were detected. The main tissues and organs were obtained through dissection for histopathological examination.

In the long-term toxicity group, blood samples were collected from mice one week after the last injection for serum separation. Liver and kidney functions and cytokine levels were detected. The main tissues and organs were obtained through dissection for histopathological examination.

The test data were analyzed to confirm the toxicity of LNPs.

The experimental results show that the liver and kidney functions, cytokine levels, and tissue sections of mice from both the acute toxicity group and the long-term toxicity group were basically the same as those of the group without administration, indicating that TLNPs do not exhibit obvious toxicity and are relatively safe.

The detection results of liver toxicity indicators of traditional cLNPs and targeting TLNPs in acute toxicity experiments in mice at different dosages are shown in Figure 14.

### Example 32

This Example is for evaluation of the delivery efficiency of nanoparticles targeting human CD8+T cells in NHP

### (I) Preparation of Nanoparticles Targeting Human CD8+T Cells

The lipid nanoparticles used in this embodiment were TLNP-2 prepared in Example 21.

### (II) Evaluation of Delivery Efficiency of Nanoparticles Targeting Human CD8+T Cells

Two Cynomolgus monkeys (NHP) that passed quarantine and reached the standard body weight were selected and divided into two groups. The prepared TLNP-EGFP was intravenously injected at a drug concentration of 100 µg/mL. One group was administered a dosage of 0.2 mpk as the high dosage group, and the other group was administered a dosage of 0.1 mpk as the low dosage group. Peripheral blood was collected before administration and on Days 1-7, Day 10, and Day 16 after administration for flow cytometry detection of the expression rate (expression ratio) of eGFP-mRNA in each cell subset.

The experimental results show that, compared with the control group, after TLNP administration, NHP exhibited a significant targeted delivery effect in CD8 cells, and the high dosage group showed a higher targeted delivery effect than the low dosage group. Both the high dosage group and the low dosage group demonstrated higher targeting effects and lower off-target. The results of dynamic changes in eGFP expression in vivo after TLNP administration indicates that the expression of eGFP-mRNA could maintain a high level for one week in NHP.

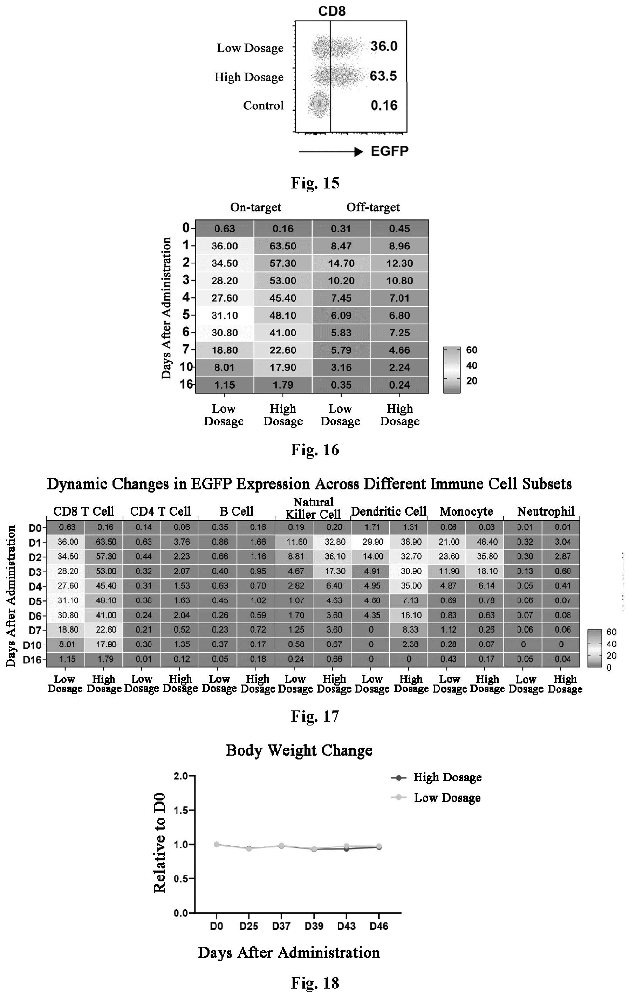

The expression of EGFP by TLNP-2 in NHP is shown in Figure 15.

The EGFP targeting and off-targeting by TLNP-2 in NHP are shown in Figure 16.

The dynamic changes of in vivo EGFP expression in TLNP-2 NHP are shown in Figure 17.

### Example 33:

This Example is for in vivo safety evaluation of nanoparticles targeting human CD8+ T Cells in NHP
(I) Preparation of Nanoparticles Targeting Human CD8+ T Cells The lipid nanoparticles used in this Example were TLNP-2 prepared in Example 21.
(II) Evaluation of the Safety of Nanoparticles Targeting Human CD8+ T Cells

The safety evaluation of nanoparticles targeting human CD8+ T cells in NHP was conducted using the same administration dosages as in Example 32. Weight measurements of NHP were performed before administration and on Days 25, 37, 39, 43, and 46 after administration. Body temperature measurements of NHP were carried out before administration and on Days 1-7, 14, 21, and 25 after administration. Complete blood count (CBC) was conducted before administration and Day 1 after administration. Serum samples were collected before administration and on Days 1-6 after administration for solid-phase chip cytokine detection. Plasma samples were collected for blood biochemical analysis before administration and on Days 1-7, 10, and 16 after administration.

The experimental results indicates that continuous monitoring of NHP weight and body temperature showed that both indicators remained at pre-administration levels after TLNP administration. The complete blood count (CBC) test revealed a slight increase in granulocytes after administration, while other CBC indicators showed little change. Continuous cytokine testing demonstrates slight fluctuations in cytokines after administration. Blood biochemical analysis reveals that only transient changes in CK and AST were observed after administration, and they quickly recovered to pre-administration levels. All these findings indicates that nanoparticles targeting human CD8+ T cells exhibited high safety in NHP.

The weight changes of NHP after TLNP-2 administration are shown in Figure 18.

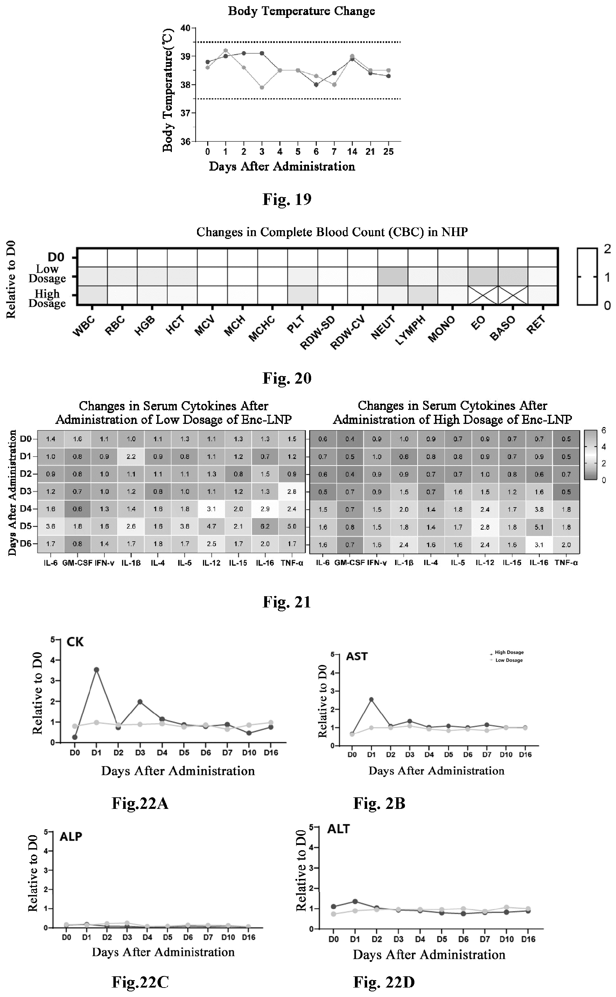

The temperature changes of NHP after TLNP-2 administration are shown in Figure 19.

The changes in NHP complete blood count (CBC) after TLNP-2 administration are shown in Figure 20.

The changes in NHP serum cytokines after TLNP-2 administration are shown in Figure 21.

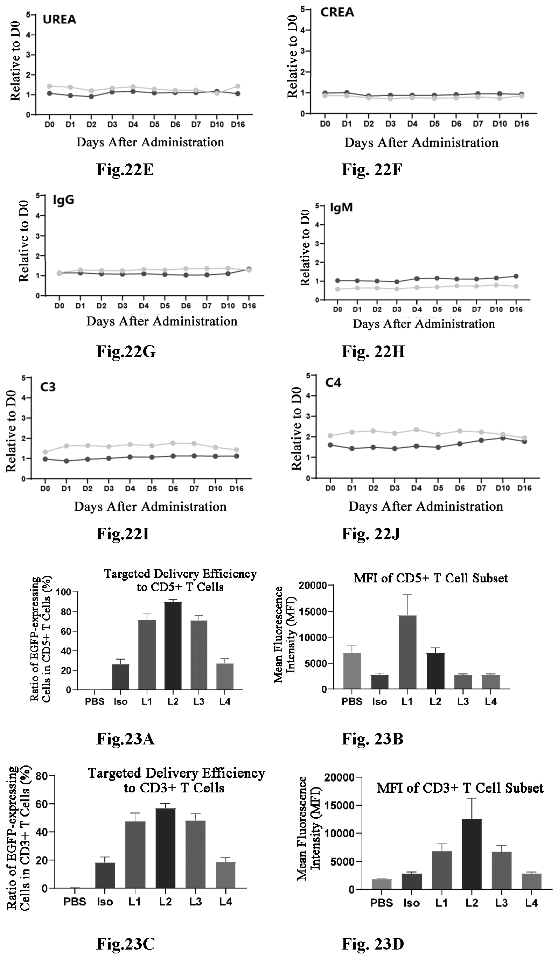

The blood biochemistry of NHP after TLNP-2 administration is shown in Figures 22A, 22B, 22C, 22D, 22E, 22F, 22G, 22H, 22I, and 22J.

### Example 34:

This Example is for Screening of Targeting Molecules in nanoparticles targeting human CD5+ T Cells

### (I) Preparation of Nanoparticles Targeting Human CD5+ T Cells

**Table 50: Preparation Table for LNPs with Different Targeting Molecules (Lx)**

| **LNP No.** | **Targeting Molecule Name and Corresponding Sequence** |
|---|---|
| **Iso-TLNP-1** | Isotype antibody |
| **L1-TLNP-1** | L1 (SEQ ID NO:31) |
| **L2-TLNP-2** | L2 (SEQ ID NO:67) |
| **L3-TLNP-3** | L3 (SEQ ID NO:70) |
| **L4-TLNP-4** | L4 (SEQ ID NO:74) |

The five groups of TLNPs needed in this Example were prepared with reference to the method described in Example 20. Specifically, the five lipid components-ionizable amino lipid, DSPC, CHO, DMG-PEG2000, DSPE-PEG2000-Mal-were dissolved in ethanol at a molar ratio of 47.5 : 10 : 41 : 1.49 : 0.01. Meanwhile, eGFP-φ-mRNA was dissolved in an acidic buffer solution containing 75 mM citric acid at pH 4, ensuring an mRNA concentration of 0.33 mg/mL in the aqueous phase. The molar ratio of the ionizable amino lipid to mRNA (N/P) was set at 6.5.

**Table 51: Quality Control Information for Nanobody-Modified Lx-TLNP Containing Different Targeting Molecules.**

| LNP No. | Particle Size (nm) | PDI | Encapsulation Efficiency (%) |
|---|---|---|---|
| Iso LNP-1 | 100.87 | 0.13 | 90.66% |
| L1-TLNP-1 | 98.57 | 0.10 | 92.54% |
| L2-TLNP-2 | 97.93 | 0.09 | 91.31% |
| L3-TLNP-3 | 95.79 | 0.09 | 92.30% |
| L4-TLNP-4 | 96.83 | 0.09 | 91.50% |

### (II) Evaluation of the In Vitro Delivery Efficiency of Nanoparticles Targeting Human CD5+ T Cells

Anti-CD3 Coating: One day in advance, 200 µL of anti-Human-CD3 antibody at a concentration of 1 µg/mL was added to each well of a 24-well plate. The plate was then stored at 4 °C overnight for coating.

Preparation before Cell Plating: The liquid in the 24-well plate was aspirated out. Subsequently, the wells were washed twice with 1640 complete medium (containing 10% New Zealand serum and 1% PS (penicillin-streptomycin) dual antibiotic).

Cell Plating: Human PBMCs were diluted to a concentration of 1 million/mL using 1640 complete medium (containing 10% New Zealand serum and 1% PS dual antibiotic) supplemented with anti-CD28 antibody at a concentration of 1 µg/mL and IL-2 at a working concentration of 400 U/mL. One million cells were added to each well of the 24-well plate. After cell plating, the cells were stabilized for 4 h under conditions of 37 °C and 5% CO₂ before LNPs were added.

Drug Administration: Each well was added with one of the five LNP groups prepared in Table 50, ensuring a working concentration of 0.5 µg/mL of LNPs in the well plate. In addition, a negative control group was established by adding phosphate-buffered saline (PBS).

Culture: After adding LNPs, PBMCs were transfected for 24 h at 37 °C and 5% CO₂.

Detection of eGFP-mRNA Expression: Cells from each well were collected, stained with a commercially available antibody for flow cytometry, and then the expression ratios of eGFP-mRNA in CD5+ T and CD3+ T cell subsets of PBMCs and mean fluorescence intensity MFI were detected by flow cytometry.

**Table 52: In Vitro Transfection Results of TLNPs with Different Targeting Molecules**

| LNP No. | Positive Rate (%) and MFI of CD5+ T cells | | Positive Rate (%) and MFI of CD3+ T cells | |
|---|---|---|---|---|
| PBS | 0.4 | 7043 | 0.5 | 1840 |
| Iso LNP-1 | 26.3 | 2805 | 18.5 | 2880 |
| L1-TLNP-1 | 71.9 | 14279 | 47.8 | 6878 |
| L2-TLNP-2 | 90.2 | 6932 | 56.9 | 12589 |
| L3-TLNP-3 | 71.4 | 2782 | 48.1 | 6763 |
| L4-TLNP-4 | 27.4 | 2750 | 19.0 | 2899 |

The experimental results show that TLNPs prepared using L1, L2, and L3 exhibits significant targeting effects on CD5+ T cells at lower dosages, along with favorable transfection efficiency.

In vitro transfection results of CD5-TLNP containing different targeting molecules are shown in Figures 23A, 23B, 23C, and 23D.

### Example 35:

This Example is for screening of targeting molecule ratios in nanoparticles targeting human CD5+ T cells.

### (I) Preparation of Nanoparticles Targeting Human CD5+ T Cells

**Table 53: Preparation Table for TLNPs with Different Targeting Molecule ratios**

| **LNP No.** | **Targeting Molecule Name** | **Ratio (Proportion) of DSPE-PEG2000-Mal Lipid** |
|---|---|---|
| **R1-L1-TLNP-1** | L1 | R1 = 0.01% |
| **R1-L2-TLNP-2** | L2 | R1 = 0.01% |
| **R1-L3-TLNP-3** | L3 | R1 = 0.01% |
| **R2-L1-TLNP-4** | L1 | R2 = 0.05% |
| **R2-L2-TLNP-5** | L2 | R2 = 0.05% |
| **R2-L3-TLNP-6** | L3 | R2 = 0.05% |
| **R3-L1-TLNP-7** | L1 | R3 = 0.10% |
| **R3-L2-TLNP-8** | L2 | R3 = 0.10% |
| **R3-L3-TLNP-9** | L3 | R3 = 0.10% |
| **R4-L1-TLNP-10** | L1 | R4 = 0.25% |
| **R4-L2-TLNP-11** | L2 | R4 = 0.25% |
| **R4-L3-TLNP-12** | L3 | R4 = 0.25% |

The twelve groups of TLNPs needed in this Example were prepared with reference to the method described in Example 20. Specifically, the five lipid components-ionizable amino lipid, DSPC, CHO, DMG-PEG2000, DSPE-PEG2000-Mal-were dissolved in ethanol at a molar ratio of 47.5 : 10 : 41 : X : Y (where the sum of X and Y is 1.5). Meanwhile, eGFP-φ-mRNA was dissolved in an acidic buffer solution containing 75 mM citric acid at pH 4, ensuring an mRNA concentration of 0.33 mg/mL in the aqueous phase. The molar ratio of the ionizable amino lipid to mRNA (N/P) was set at 6.5.

**Table 54: Quality Control Information for TLNPs with Different Targeting Molecule Ratios.**

| LNP No. | Particle Size (nm) | PDI | Encapsulation Efficiency (%) |
|---|---|---|---|
| R1-L1-TLNP-1 | 94.80 | 0.11 | 89.97% |
| R1-L2-TLNP-2 | 94.51 | 0.09 | 91.81% |
| R1-L3-TLNP-3 | 94.07 | 0.08 | 91.46% |
| R2-L1-TLNP-4 | 95.58 | 0.11 | 89.33% |
| R2-L2-TLNP-5 | 95.86 | 0.11 | 92.06% |
| R2-L3-TLNP-6 | 95.35 | 0.11 | 91.14% |
| R3-L1-TLNP-7 | 96.72 | 0.10 | 89.94% |
| R3-L2-TLNP-8 | 98.10 | 0.11 | 85.91% |
| R3-L3-TLNP-9 | 99.69 | 0.15 | 90.52% |
| R4-L1-TLNP-10 | 97.00 | 0.10 | 89.24% |
| R4-L2-TLNP-11 | 98.88 | 0.10 | 89.02% |
| R4-L3-TLNP-12 | 98.76 | 0.11 | 90.65% |

### (II) Evaluation of the In Vitro Delivery Efficiency of Nanoparticles Targeting Human CD5+ T Cells

Anti-CD3 Coating: One day in advance, 200 µL of anti-Human-CD3 antibody at a concentration of 1 µg/mL was added to each well of a 24-well plate. The plate was then stored at 4 °C overnight for coating.

Preparation before Cell Plating: The liquid in the 24-well plate was aspirated out. Subsequently, the wells were washed twice with 1640 complete medium (containing 10% New Zealand serum and 1% PS (penicillin-streptomycin) dual antibiotic).

Cell Plating: Human PBMCs were diluted to a concentration of 1 million/mL using 1640 complete medium (containing 10% New Zealand serum and 1% PS dual antibiotic) supplemented with anti-CD28 antibody at a concentration of 1 µg/mL and IL-2 at a working concentration of 400 U/mL. One million cells were added to each well of the 24-well plate. After cell plating, the cells were stabilized for 4 h under conditions of 37 °C and 5% CO₂ before LNPs were added.

Drug Administration: Each well was added with one of the twelve LNP groups prepared in Table 53, ensuring a working concentration of 0.5 µg/mL of LNPs in the well plate. In addition, a negative control group was established by adding phosphate-buffered saline (PBS).

Culture: After adding LNPs, PBMCs were transfected for 24 h at 37 °C and 5% CO₂.

Detection of eGFP-mRNA Expression: Cells from each well were collected, stained with a commercially available antibody for flow cytometry, and then the expression ratios of eGFP-mRNA in CD5+ T cell subsets of PBMCs and mean fluorescence intensity MFI were detected by flow cytometry.

**Table 55: In Vitro Transfection Results of TLNPs with Different Targeting Molecule Ratios**

| LNP No. | Positive Rate(%) | MFI |
|---|---|---|
| PBS | 0.4 | 1580 |
| R1-L1-TLNP-1 | 66.8 | 6228 |
| R1-L2-TLNP-2 | 84.7 | 10078 |
| R1-L3-TLNP-3 | 64.0 | 6001 |
| R2-L1-TLNP-4 | 72.8 | 7094 |
| R2-L2-TLNP-5 | 91.5 | 12959 |
| R2-L3-TLNP-6 | 67.7 | 6449 |
| R3-L1-TLNP-7 | 76.3 | 7576 |
| R3-L2-TLNP-8 | 92.7 | 13841 |
| R3-L3-TLNP-9 | 68.0 | 6542 |
| R4-L1-TLNP-10 | 67.4 | 5967 |
| R4-L2-TLNP-11 | 89.0 | 11936 |
| R4-L3-TLNP-12 | 61.7 | 5725 |

The experimental results show that L2-TLNP, prepared using L2, exhibited the best targeting effect and delivery efficiency for CD5+ T cells at lower dosages. Furthermore, when the ratio (proportion) of targeting molecules exceeds 0.05%, no significant differences in delivery efficiency are observed among TLNP groups with different ratios. Considering factors such as the physicochemical properties of LNPs and material costs, a targeting molecule ratio of 0.05% was selected for subsequent studies.

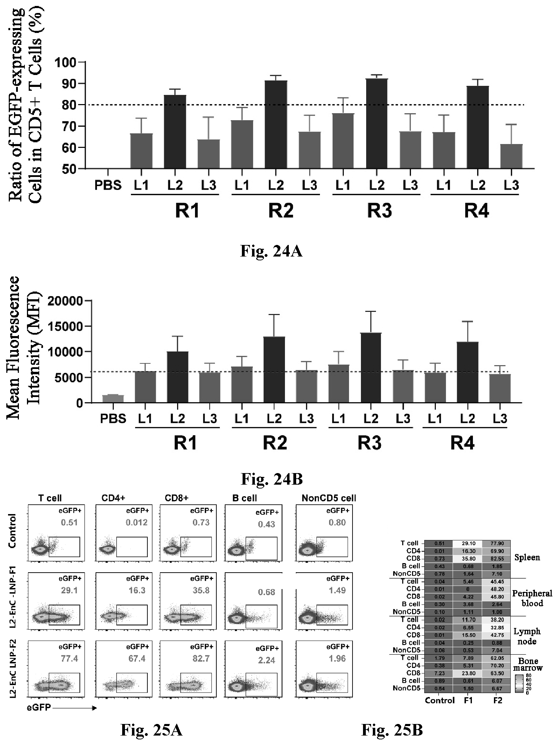

In vitro transfection results of CD5-TLNP with different targeting molecule ratios are shown in Figures 24A and 24B.

### Example 36:

This Example is for screening of component formulations for nanoparticles targeting human CD5+ T cells.

### (I) Preparation of Nanoparticles Targeting Human CD5+ T Cells

**Table 56: Preparation Table for CD5-TLNP with Different Component Ratios.**

| **LNP No.** | **Ratio of Five Components of LNPs (Ionized amino lipid: DSPC : CHO : DMG-PEG2000: DSPE-PEG2000-Mal )** |
|---|---|
| **L2-TLNP-F1** | 47.5: 10: 41: 1.45: 0.05 |
| **L2-TLNP-F2** | 47.5: 15: 36: 1.0: 0.05 |

The two lipid nanoparticles used in this Example, L2-TLNP-F1 and L2-TLNP-F2, were prepared with reference to the method described in Example 21.

### (II) Investigation on In Vivo Delivery Efficiency of Nanoparticles Targeting Human CD5+ T Cells with Different Formulations

Nine 6-8-week-old female hCD5 humanized mice that passed quarantine and met the weight requirements of the SPF (specific pathogen-free) level were selected and divided into 3 groups. The prepared L2-TLNP-F1 and L2-TLNP-F2 were administered via tail vein injection to the mice. Each mouse received a dosage of 20 µg-mRNA-LNP with an injection volume of 200 µL. After 24 hours of expression, peripheral blood, spleen, lymph node, and bone marrow from tibial bone were collected for flow cytometry analysis to determine the eGFP-mRNA expression rates (ratios) in cellular subsets of each organ.

**Table 57: In Vivo Experimental Grouping of L2-TLNPs with Different Formulations**

| **Experimental Group No.** | **Name of Administrated LNP** |
|---|---|
| **G1** | PBS |
| **G2** | L2-TLNP-F1 |
| **G3** | L2-TLNP-F2 |

The experimental results show that L2-TLNP-F2, prepared with formulation F2 and having a 0.05% targeting molecule ratio, exhibited superior CD5+ T cell targeting and delivery efficiency in various organs of hCD5 humanized mice at a dosage of 1.0 mpk compared to the F1 group.

Statistical graphs of in vivo transfection with L2-TLNPs with different formulations are shown in Figures 25A and 25B.

### Example 37:

This Example is for the study of the in vivo biodistribution of nanoparticles targeting human CD5+ T cells.

### (I) Preparation of Nanoparticles Targeting Human CD5 T Cells

The two lipid nanoparticles used in this Example were cLNP-1 and L2-TLNP-F2 encapsulating Luciferase mRNA, respectively, which were prepared using the preparation method described in Example 21.

### (II) Biodistribution of Luc-mRNA in hCD5 Humanized Mice for Nanoparticles Targeting Human CD5 T Cells

Nine 6-8-week-old female hCD5 humanized mice that passed quarantine and met the weight requirements of the SPF (specific pathogen-free) level were selected (three mice per group). Mice in group G1 received a tail vein injection of 200 µL of PBS per mouse. Mice in groups G2 and G3 received a tail vein injection of 2 µg of Luc-φ-mRNA LNP per mouse with an injection volume of 200 µL.

Six hours after administration, the mice were anesthetized with isoflurane, and then injected intraperitoneally with 250 µL of luciferase substrate. The mice were dissected 10 minutes later. The brain, heart, liver, spleen, lung, kidney, thymus, lymph node, and bone marrow were removed for ex vivo organ imaging. The imaging instrument used was the Living Imaging instrument from PerkinElmer (Shenzhen Bay Laboratory), with the parameters set to F8 mode and an exposure time of 2 seconds. After imaging, mRNA was extracted from the harvested organs for qPCR experiments to quantify the enrichment level of mRNA in each organ.

**Table 58: Experimental Grouping for Biodistribution of LNPs Using Luc Imaging**

| **Experimental Group No.** | **Name of Administrated LNP** |
|---|---|
| **G1** | PBS |
| **G2** | cLNP |
| **G3** | L2-TLNP-F2 |

The experimental result show that, in the hCD5 humanized mouse model, compared to conventional LNPs, the CD5+ T cell-targeting L2-TLNP-F2 can more selectively deliver Luc-mRNA to T cell-rich organs, demonstrating a higher spleen-to-liver fluorescence intensity ratio.

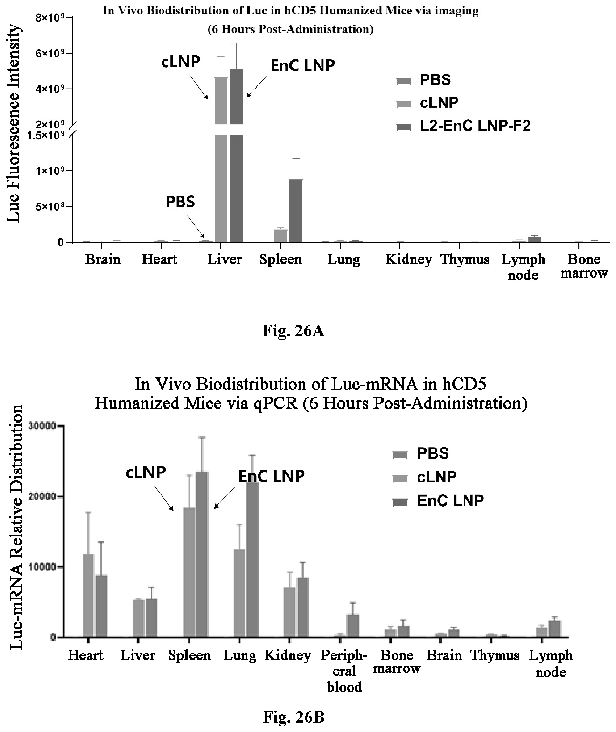

Statistical graphs of the biodistribution results in hCD5 humanized mice as determined by Luc imaging and qPCR are shown in Figures 26A and 26B.

The aforementioned Examples merely represent several embodiments of the present invention, providing a specific and detailed understanding of the technical solutions herein. However, they should not be construed as limiting the scope of protection of the invention. It should be noted that for those skilled in the art, various modifications and improvements can be made without departing from the inventive concept, all of which fall within the scope of protection of the present invention.

### Industrial Applicability

The present invention provides a targeting molecule capable of specifically delivering drugs to particular cells, facilitating precise in vivo programming of specific cells to exert therapeutic effects. This approach significantly reduces drug dosage and side effects, achieving precise cellular therapy within the body, and thus holds considerable economic value and promising application prospects.

## Claims

1. A molecule having targeting function, comprising:
(I) one or more hydrophobic chains;
(II) one or more linkers;
(III) one or more nanobodies.

2. The molecule having targeting function of claim 1, wherein the number of the nanobody is 20 or less.

3. The molecule having targeting function of claim 1 or claim 2, wherein the number of the nanobody is 1 to 7.

4. The molecule having targeting function of any one of claims 1-3, wherein one amino acid containing a reactive site is interposed between the nanobody and the linker, and the amino acid comprises, but not limited to, cysteine and lysine.

5. The molecule having targeting function of claim 4, wherein the nanobody is connected via a connecting sequence, which is located between the nanobody and the amino acid, and the connecting sequence comprises (GS)n and/or (GGGS)m, where n and m are the same or different values, and the values of n or m are selected from 0, 1, 2, 3, or 4.

6. The molecule having targeting function of any one of claims 1-5, wherein the hydrophobic chain comprises one or more substituted or unsubstituted C12-C18 alkyl.

7. The molecule having targeting function of any one of claims 1-6, wherein the linker comprises one or more structural units selected from -C(=O)O-, -O-C(=O)O-, -C(=O)NH-, -C(=O)S-, -S-, -S-S-, triazolyl, hydrazone bond, SMCC, Sulfo-SMCC, SATA, polypeptides as structural units, PEG, or PEG derivatives;
wherein the PEG derivatives can be selected from maleimide-functionalized polyethylene glycol, including but not limited to, PEG-MAL.

8. The molecule having targeting function of claim 7, wherein the relative molecular mass of PEG or PEG derivatives is 500-5,000.

9. The molecule having targeting function of claim 8, wherein the relative molecular mass of PEG or PEG derivatives is 1,000, 2,000, 3,000, 4,000, or 5,000.

10. The molecule having targeting function of any one of claims 1-9, wherein the nanobody comprises an antigen-binding domain specific to an antigen, and the antigen is a membrane protein molecule.

11. The molecule having targeting function of claim 10, wherein the membrane protein molecule is an immune cell-related cell membrane protein molecule or an immune-related cell membrane protein molecule, including but not limited to, one or more of CD3, CD4, CD5, CD7, CD8, CD25, CD38, CD61, CD42a, CD105, CD90, CD15, CD127, CD56, CD68, CD19, CD11c, CD138, F4/80, CD62P, CD49f, CD31, RANK, ALPL, PDPN, CD34, FcεR1α, CD203c, CD63, CD193, CD66b, CD41, CD117, and ASGPR.

12. The molecule having targeting function of claim 10, wherein the membrane protein molecule is a central nervous system cell membrane protein molecule, including but not limited to, one or more of GD2, GD3, MOG, and TMEM119.

13. The molecule having targeting function of claim 10, wherein the membrane protein molecule is a tumor-associated antigen, including but not limited to, one or more of CD133, PSMA, CLDN18.2, DLL3, TROP2, EGFRVIII, CA125, MUC1, MUC16, MSLN, CA9, HER2, HER3, TGM4, PSCA, CLDN6, STEAP2, GPC3, IL-13Ra2, EGFR, STEAP1, BCMA, FRa, VEGFR2, PDGFR-β, CEA, NCAM, FAP, SLC2A2, SEZ6L2 and LRP11.

14. The molecule having targeting function of claim 10, wherein the membrane protein molecule is an immune regulatory molecule, including but not limited to, one or more of B7-H1, B7-H3, and B7-H4.

15. The molecule having targeting function of claim 10, wherein the membrane protein molecule comprises, but is not limited to, one or more of the following targets: CD3, CD4, CD5, CD7, CD8, CD25, CD38, CD61, CD42a, CD105, CD90, CD15, CD127, CD56, CD68, CD19, CD11c, CD138, F4/80, CD62P, CD49f, CD31, RANK, ALPL, PDPN, CD34, FcεR1α, CD203c, CD63, CD193, CD66b, CD41, CD117, ASGPR, GD2, GD3, MOG, TMEM119, CD133, PSMA, CLDN18.2, DLL3, TROP2, EGFRVIII, CA125, MUC1, MUC16, MSLN, CA9, HER2, HER3, TGM4, PSCA, CLDN6, STEAP2, GPC3, IL-13Ra2, EGFR, STEAP1, BCMA, FRa, VEGFR2, PDGFR-β, CEA, NCAM, FAP, SLC2A2, SEZ6L2, LRP11, B7-H1, B7-H3 and B7-H4.

16. The molecule having targeting function of any one of claims 1-15, wherein the nanobody is a nanobody targeting CD8, with the amino acid sequence of the nanobody represented by any one of SEQ ID NOs:1-3; and/or
,the nanobody is a nanobody targeting CD19, with the amino acid sequence of the nanobody represented by any one of SEQ ID NOs:79-81; and/or
,the nanobody is a nanobody targeting CD56; and/or
,the nanobody is a nanobody targeting CD5, with the amino acid sequence of the nanobody represented by any one of SEQ ID NOs:4-78; and/or
, the nanobody is a nanobody targeting PSMA, with the amino acid sequence of the nanobody represented by any one of SEQ ID NOs:82-83; and/or
, the nanobody is a nanobody targeting CD133, with the amino acid sequence of the nanobody represented by any one of SEQ ID NOs:84-85.

17. A nanobody having targeting function, wherein the amino acid sequence of the nanobody is represented by any one of SEQ ID NOs:4-78; or the amino acid sequence of the nanobody has a homology of 80% or greater with the amino acid sequence represented by any one of SEQ ID NOs:4-78.

18. A lipid nanoparticle, wherein its components comprise the molecule having targeting function of any one of claims 1-16, and the content of the molecule having targeting function is 0.0001-1.0 mol% of the total lipids of the lipid nanoparticle.

19. The lipid nanoparticle of claim 18, wherein the content of the molecule having targeting function is 0.01-0.5 mol% of the total lipids of the lipid nanoparticle.

20. The lipid nanoparticle of claim 18 or 19, wherein the components of the lipid nanoparticle comprise a polymer-conjugated lipid and an ionizable amino lipid; the polymer-conjugated lipid is selected from lipids modified with PEG or PEG derivatives; and the relative molecular mass of the lipids modified with PEG or PEG derivatives is 2,000-5,000.

21. The lipid nanoparticle of claim 20, wherein the relative molecular mass of the lipids modified with PEG or PEG derivatives is 2,000, 3,000, 4,000, or 5,000.

22. The lipid nanoparticle of claim 20 or 21, wherein the content of the polymer-conjugated lipid is 0.2-5 mol% of the total lipids of the lipid nanoparticle.

23. The lipid nanoparticle of claim 22, wherein the content of the polymer-conjugated lipid is 0.5-2 mol% of the total lipids of the lipid nanoparticle.

24. The lipid nanoparticle of any one of claims 20-23, wherein the PEG derivatives comprise maleimide-functionalized polyethylene glycol and non-maleimide-functionalized polyethylene glycol; wherein the maleimide-functionalized polyethylene glycol can be selected from PEG-MAL; and the content of the maleimide-functionalized polyethylene glycol is 0.0001-1.0 mol% of the total lipids of the lipid nanoparticle.

25. The lipid nanoparticle of claim 24, wherein the content of the maleimide-functionalized polyethylene glycol is 0.01-0.5 mol% of the total lipids of the lipid nanoparticle.

26. The lipid nanoparticle of any one of claims 20-25, wherein the content of the ionizable amino lipid is 30-70 mol% of the total lipids of the lipid nanoparticle.

27. The lipid nanoparticle of claim 26, wherein the content of the ionizable amino lipid is 40-60 mol% of the total lipids of the lipid nanoparticle.

28. The lipid nanoparticle of any one of claims 20-27, wherein the ionizable amino lipid has the structure represented by general formula (I), or is an isomer, a pharmaceutically acceptable salt, a prodrug, or a solvate of the structure represented by general formula (I); 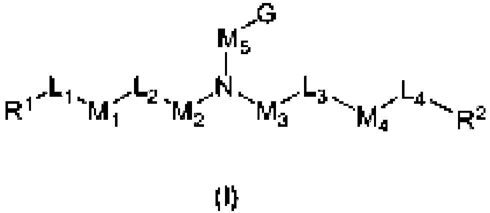
wherein, G is selected from H, OR, CN, -C(=O)OR', -OC(=O)R', -C(=O)NR'R'', -NR'C(=O)R'', NR'R'', or a cycloalkyl structure containing at least one heteroatom; the carbon atom or heteroatom that is substitutable in the cycloalkyl structure is unsubstituted or substituted with one or more hydroxyl, C₁-C₄ alkyl, C₂-C₄ alkenyl, C₃-C₈ cycloalkyl , or C3-C8 cycloalkenyl;
M₁, M₂, M₃, and M₄ are the same or different from each other, and each is independently selected from C₁-C₂₄ alkylene, C₃-C₂₄ cycloalkylene, C₂-C₂₄ alkenylene, or C₃-C₂₄ cycloalkenylene;
R¹ and R² are the same or different, and each is independently selected from H, C₁-C₂₄ alkyl, C₃-C₂₄ cycloalkyl, C₂-C₂₄ alkenyl, or C₃-C₂₄ cycloalkenyl;
L₁, L₂, L₃, and L₄ are the same or different from each other, and each is independently selected from -C(=O)O-, -OC(=O)-, -C(=O)S-, -SC(=O)-, -C(=O)NR-, -NRC(=O)-, -S(=O)-, -OS(=O)₂-, -S(=O)₂O-, -O-, -S-, or -S-S-;
R, R', and R'' are the same or different from each other, and each is independently selected from H, C₁-C₁₀ alkyl, C₃-C₁₀ cycloalkyl, C₃-C₁₀ alkenyl, or C₃-C₁₀ cycloalkenyl, C₁-C₁₀ alkyl with a terminal tertiary amino, C₃-C₁₀ cycloalkyl with a terminal tertiary amino, C₃-C₁₀ alkenyl with a terminal tertiary amino, or a cycloalkyl structure containing at least one heteroatom; the cycloalkyl structure is unsubstituted or substituted with one or more C₁-C₄ alkyl, C₂-C₄ alkenyl, C₃-C₈ cycloalkyl, or C₃-C₈ cycloalkenyl;
M₅ is selected from a single bond, C₁-C₁₆ alkylene, C₂-C₁₆ alkenylene, C₃-C₈ cycloalkylene, or C₃-C₈ cycloalkenylene.

29. The lipid nanoparticle of any one of claims 20-28, wherein the components of the lipid nanoparticle further comprise at least one of a steroid and a neutral lipid.

30. The lipid nanoparticle of claim 29, wherein the steroid is one or more of cholesterol and derivatives thereof, cholesterol ester, steroid hormone, steroid vitamin, and phytosterol; and/or the neutral lipid is a phospholipid; and/or the polymer-conjugated lipid is a PEGylated lipid.

31. The lipid nanoparticle of claim 30, wherein the steroid is cholesterol.

32. The lipid nanoparticle of claim 30, wherein the PEGylated lipid is DMG-PEG2000.

33. The lipid nanoparticle of any one of claims 29-32, wherein the molar ratio of the ionizable amino lipid, steroid, neutral lipid, and polymer-conjugated lipid is (30-70) : (0-65) : (0-30) : (0.2-5).

34. The lipid nanoparticle of claim 33, wherein the molar ratio of the ionizable amino lipid, steroid, neutral lipid, and polymer-conjugated lipid is (40-60) : (35-60) : (0-20) : (0.5-2).

35. The lipid nanoparticle of claim 34, wherein the molar ratio of the ionizable amino lipid, steroid, neutral lipid, and polymer-conjugated lipid is (40-55) : (30-50) : (0-20) : (0.5-2).

36. The lipid nanoparticle of claim 35, wherein the molar ratio of the ionizable amino lipid, steroid, neutral lipid, and polymer-conjugated lipid is (40-50) :(30-45) :(0-15) : (0.5-2).

37. The lipid nanoparticle of claim 36, wherein the molar ratio of the ionizable amino lipid, steroid, neutral lipid, and polymer-conjugated lipid is (47-50) : (34-36) : (13-16) : (1.0-1.1).

38. The lipid nanoparticle of claim 33, wherein the molar ratio of the ionizable amino lipid, steroid, neutral lipid, and polymer-conjugated lipid is 47.5 : 36 : 15 : 1.0.

39. A method for preparing the lipid nanoparticle of any one of claims 18-38, wherein the method comprises the following steps: mixing a polymer-conjugated lipid, an ionizable amino lipid, a steroid, and an auxiliary phospholipid to obtain a lipid nanoparticle without targeting function, and then connecting the molecule having targeting function of any one of claims 1-16 to the lipid nanoparticle without targeting function through membrane fusion or chemical bond to obtain the lipid nanoparticle having targeting function.

40. A method for preparing the lipid nanoparticle of any one of claims 18-38, wherein the method comprises the following steps: mixing a polymer-conjugated lipid, an ionizable amino lipid, a steroid, and an auxiliary phospholipid to obtain a lipid nanoparticle without targeting function, and then connecting a nanobody to the lipid nanoparticle without targeting function through a chemical bond ,linkage to obtain the lipid nanoparticle having targeting function.

41. The method of claim 40, wherein the polymer-conjugated lipid comprises maleimide-functionalized polyethylene glycol, and the nanobody is connected to the maleimide-functionalized polyethylene glycol.

42. A method for preparing the lipid nanoparticle of any one of claims 18-38, wherein the method comprises the following steps: mixing a polymer-conjugated lipid, an ionizable amino lipid, a steroid, an auxiliary phospholipid, and the molecule having targeting function of any one of claims 1-16 to obtain the lipid nanoparticle having targeting function.

43. A method for targeted delivery of pharmacologically active molecules to specific cells of a subject, the method comprises contacting the specific cells with the lipid nanoparticle of any one of claims 18-38.

44. The method of claim 43, wherein the specific cells comprise, but are not limited to, immune cells, immune-related cells, central nervous cells, tumor cells, somatic cells, and cells infected by viruses, bacteria, or fungi.

45. The method of claim 43 or 44, wherein the target cells are B cells, T cells, or NK cells.

46. A method for expressing a target protein or polypeptide in target cells of a subject, the method comprises contacting the target cells with the lipid nanoparticle of any one of claims 18-38.

47. The method of claim 46, wherein the target cells comprise, but are not limited to, immune cells, immune-related cells, central nervous cells, tumor cells, somatic cells, and cells infected by viruses, bacteria, or fungi.

48. The method of claim 46 or 47, wherein the target cells are B cells, T cells, or NK cells.

49. Use of the lipid nanoparticle of any one of claims 18-38 as a drug delivery carrier.

50. A pharmaceutical composition, wherein it comprises the lipid nanoparticle of any one of claims 18-38, and the lipid nanoparticle comprises a pharmacologically active molecule.

51. The pharmaceutical composition of claim 50, wherein the pharmacologically active molecule comprises one or more of mRNA, DNA, siRNA, saRNA, shRNA, miRNA, circle RNA, lnc RNA, gRNA, polypeptide, or protein.

52. The pharmaceutical composition of claim 50 or 51, wherein the pharmacologically active molecule comprises mRNA, and the mRNA is used to encode a CAR molecule, a TCR molecule, an immune regulatory molecule, a functional protein molecule, or a gene editing tool such as a base editor or Cas9 protein.

53. The pharmaceutical composition of any one of claims 50-52, wherein the CAR molecule comprises at least one sequence of the following sequences: a sequence that can specifically recognize a tumor-specific antigen, a transmembrane connecting sequence, and/or a sequence that assists the immune function of T cells.

54. The pharmaceutical composition of any one of claims 50-53, wherein a sequence encoding a TCR molecule comprises a sequence capable of recognizing at least one molecule of NY-ESO-1, AFP, HBsAg, MAGEA1, Mesothelin, MART-1, CD19, CD28, PD-1, CD8, CT83, E6, E7, E8, GPC3, H3.3-K27M, HIV Gag polyprotein, HLA-A/AFP, KRASG12(V/D), KRASG12D, KRASG12V, LMP1, LMP2, EBNA1, MAGEA10, MAGEA3, MAGEA4, MC2R, mHag HA-1, MYO1G, PRAME, WT1, gp100, CEA, p53, HLA-A2, EGFR, DR5, RAS, LAGE-1, CMV, HCV, MCPyv, and HA-1H.

55. The pharmaceutical composition of any one of claims 50-54, wherein a sequence encoding an immune regulatory molecule comprises a sequence of at least one molecule of CD28, PD-1, CTLA-4, RGMB, ICOS, CD28H, NKp30, HVEM, OX40, Fas, 4-1BB, CD27, CD30, APO-2, APO-3, BCMA, BAFFR, GITR, IL-2, IL-12A, IL-12B, IL-15, IL-23, IL-27, FLT3L, IL-36A, IL-36B, IL-36C, GM-CSF, CCL20, CXCL9, CXCL10, CXCL11, CXCL12, CCR7, CXCR4, CCR5, CCL4, CCL5, CCL19, CXCR3, CCR6, B7-H1, B7-DC, B7-H3, B7-H4, PD-1H, IL-10, TGF-β, HGF, B7-1, B7-2, B7-H2, B7-H3, CD40, FasL, CD70, CD30L, 4-1BBL, OX40L, TRAIL, RANKL, TWEAK, APRIL, BAFF, LIGHT, GITRL, CD73, STAT1, IRF4, CCAR2, BCL6, iNOS and CD103.

56. The pharmaceutical composition of any one of claims 50-55, wherein a nucleic acid encoding a CAR molecule comprises a sequence capable of recognizing at least one molecule of CD19, BMCA, CD22, CD20, CD123, GD2, CD30, GPC3, CLDN18.2, Mesothelin, CD33, CD38, EGFRvlll, CD138, CEA, HER2, PSMA, CLL1, CD56, EGFR, MUC-1, EpCAM, CD7, NKG2D, PD-L1, LewisY, FAP, c-MET, ROR1, IL13Rα2, AFP, CD133, CD4, BTK, ROBO1, CD5, CD70, LILRB4, FLT3, Sigle-6, CD229 and SLAMF7.

57. A pharmaceutical formulation, comprising the pharmaceutical composition of claim 50 and a pharmaceutically acceptable carrier.

58. The pharmaceutical formulation of claim 57, wherein the pharmaceutical formulation is an aqueous injection, a freeze-dried powder, or a spray.
